(19) Europäisches Patentamt
European Patent Office
Office européen des brevets

(11) EP 1 736 474 A1

(12) **EUROPEAN PATENT APPLICATION**
published in accordance with Art. 158(3) EPC

(43) Date of publication:
**27.12.2006 Bulletin 2006/52**

(21) Application number: **05728764.1**

(22) Date of filing: **07.04.2005**

(51) Int Cl.:
***C07D 409/14*** *(2006.01)* ***A61K 31/454*** *(2006.01)*
***A61K 31/4709*** *(2006.01)* ***A61K 31/538*** *(2006.01)*
***A61K 31/5415*** *(2006.01)* ***A61P 9/10*** *(2006.01)*
***A61P 21/02*** *(2006.01)* ***A61P 25/04*** *(2006.01)*
***A61P 25/06*** *(2006.01)* ***A61P 25/08*** *(2006.01)*
***A61P 25/14*** *(2006.01)* ***A61P 25/16*** *(2006.01)*
***A61P 25/22*** *(2006.01)* ***A61P 25/28*** *(2006.01)*
***A61P 43/00*** *(2006.01)* ***C07D 413/14*** *(2006.01)*
***C07D 417/14*** *(2006.01)*

(86) International application number:
**PCT/JP2005/006859**

(87) International publication number:
**WO 2005/097782 (20.10.2005 Gazette 2005/42)**

(84) Designated Contracting States:
**AT BE BG CH CY CZ DE DK EE ES FI FR GB GR HU IE IS IT LI LT LU MC NL PL PT RO SE SI SK TR**

(30) Priority: **07.04.2004 JP 2004112645**

(71) Applicant: **KYOWA HAKKO KOGYO CO., LTD.**
**Chiyoda-ku,**
**Tokyo 100-8185 (JP)**

(72) Inventors:
- **TAKAI, Haruki**
  **c/oBioFrontier Laboratories,**
  **Machida-shi, Tokyo 194-8533 (JP)**
- **KUNORI, Shunji**
  **c/o Pharmaceutical Research Center**
  **Sunto-gun, Shizuoka, 411-8731 (JP)**
- **SHIRAKURA, Shiro**
  **Parmaceutical Research Center**
  **Sunto-gun, Shizuoka, 411-8731 (JP)**
- **SHINODA, Katsumi**
  **Pharmaceutical Research Center**
  **Sunto-gun, Shizuoka, 411-8731 (JP)**
- **MIZUTANI, Atsuko**
  **Pharmaceutical Research Center**
  **Sunto-gun, Shizuoka, 411-8731 (JP)**
- **YAMADA, Koji**
  **Pharmaceutical Research Center**
  **Sunto-gun, Shizuoka, 411-8731 (JP)**
- **TOKI, Shinichiro**
  **Pharmaceutical Research Center**
  **Sunto-gun, Shizuoka, 411-8731 (JP)**
- **NISHIKAWA, Tomoyuki**
  **BioFrontier Laboratories**
  **Machida-shi, Tokyo 194-8533 (JP)**

(74) Representative: **Casalonga, Axel et al**
**Bureau Casalonga & Josse**
**Bayerstrasse 71/73**
**80335 München (DE)**

# (54) PIPERIDINE DERIVATIVE

(57) For example, a piperidine derivative represented by following formula (I) (wherein, -C(=O)-Z- represents -C(=O)-$CH_2$-, -C(=O)-C$(CH_3)_2$-, -C(=O)-NH-, -C(=O)-O-, -C(=O)-S-, - C(=O)-$CH_2CH_2$-, -C(=O)-CH=CH-, -C(=O)-$CH_2$O-, -C(=O)-$CH_2$S-, - C(=O)-$CH_2CH_2CH_2$- or -C(=O)-$NR^8CH_2$-; $R^1$ represents a hydrogen atom, substituted or unsubstituted lower alkyl, or the like; $R^2$ represents a hydrogen atom or hydroxy; $R^3$ represents a hydrogen atom, substituted or unsubstituted lower alkyl, or the like; $R^5$ represents a hydrogen atom, hydroxy, or the like; $R^6$ represents a hydrogen atom, hydroxy, substituted or unsubstituted lower alkyl, or the like, etc.; $R^7$ represents a hydrogen atom, halogen, substituted or unsubstituted lower alkyl, or the like; n and k each independently represents an integer of 0 to 2; and $\overset{----}{\text{——}}$ represents a single bond, or a double bond together with $R^4$, where in case $\overset{----}{\text{——}}$ is a single bond, $R^4$ represents a hydrogen atom, hydroxy, or the like) or a pharmaceutically acceptable salt thereof and the like having an antagonistic activity for a glutamic acid receptor (NR2B/NMDA receptor) of an N-methyl-D-aspartic acid (NMDA) type containing an NR2B subunit are provided.

EP 1 736 474 A1

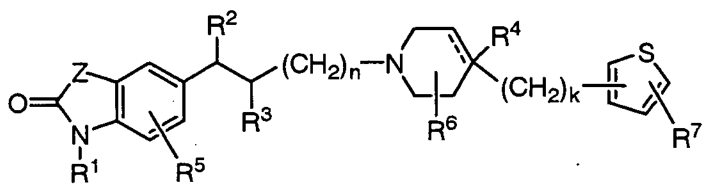

(I)

## Description

Technical Field

The present invention relates to piperidine derivatives having an antagonistic activity for glutamic acid receptor of an N-methyl-D-aspartic acid (NMDA) type (an NMDA receptor), or a pharmaceutically acceptable salts thereof.

Background Art

Glutamate is a principal excitatory neurotransmitter in the central nervous system, and it also has a function of endogenous excitatory toxin which induces degeneration and injury of neurons. The glutamate signal was transmitted into the cells via a glutamate receptor such as an NMDA receptor. However, it has been known that the excessive stimulation of NMDA receptors resulting from inappropriate release of glutamate in the brain leads to a variety of neurological disorders due to the neuronal degeneration or the neuronal injury (epilepsy, stroke, anxiety, cerebral ischemia, muscle spasm, Alzheimer's disease, dementia, Huntington's disease, Parkinson's disease, cerebral apoplexy, cerebral infarction and the like). The NMDA receptor antagonist is believed to be useful for the treatment of these diseases (Drug Dev. Res., 1989, vol. 17, page 299; Clinical Sci., 1985, vol. 68, page 113).
In addition, it has been known that glutamate plays an important role in pain (chronic pain, neuropathic pain, headache, migraine and the like) via a glutamate receptor and that the NMDA receptor is essentially implicated in development and maintenance of neuropathic pain. For example, non-selective NMDA receptor antagonists such as ketamine, dextromethorphan and CPP (3-(2-carboxypiperazin-4-yl)propyl-1-phosphoric acid) alleviates a variety of symptoms of neuropathic pain including chronic pain, post herpetic pain, central pain after spinal cord injury and phantom limb pain [Japanese Patent No. 308,135; Pain, 1992, vol. 51, pages 249 to 253; Pain, 1995, vol. 61, pages 221 to 228; Intensive Care, 1995, vol. 23, pages 620 to 622; Clinical Neuropharmacology, 1995, vol. 18, pages 360 to 368; etc.]. However, those compounds cause side effects at their analgesic dose such as dizziness, headache, hallucination, discomfort and disorder in recognition function and motor function, and accordingly, they have not been clinically used so much. Furthermore, ifenprodil which has been available in the market as an ameliorant of metabolism in cerebral circulation selectively inhibits NMDA receptors containing the NR2B subunit (selective NR2B/NMDA receptor antagonist) whereby it has been known as a drug for ameliorating the side effect by the non-selective NMDA receptor antagonists. However, ifenprodil reduced blood pressure, because it also has an antagonistic activity for $\alpha_1$-adrenoceptors (refer to Non-Patent Document 1 and Non-Patent Document 2). Thus, there has been a demand for the development of a selective antagonist of NR2B/NMDA receptor (selective NR2B/NMDA receptor antagonist) having little side effect.
It has been also known that ifenprodil and compounds similar thereto have an analgesic activity (refer to Non-Patent Document 3 and Non-Patent Document 4).
On the other hand, with regard to piperidine derivatives having an aromatic ring group at 4-position, arylpiperidine derivatives and aralkylpiperidine derivatives having an antagonistic activity at NMDA receptor or a selective antagonistic activity at NR2B/NMDA receptor have been known (refer to Patent Document 1, Patent Document 2, Patent Document 3, Patent Document 4, Patent Document 5, Patent Document 6, Patent Document 7 and Patent Document 8).

Patent Document 1: WO 91/17156
Patent Document 2: WO 94/10166
Patent Document 3: WO 03/91241
Patent Document 4: U. S. Patent No. 5,710,168
Patent Document 5: JP-A-2002-322092
Patent Document 6: WO 97/07098
Patent Document 7: WO 90/14087
Patent Document 8: WO 90/14088
Non-Patent Document 1: Journal of Pharmaceutical and Experimental Therapeutics, 1988, vol. 247, pages 1211 to 1221
Non-Patent Document 2: Molecular Pharmacology, 1993, vol. 44, pages 851 to 859
Non-Patent Document 3: British Journal of Pharmacology, 1997, vol. 122, pages 809 to 812
Non-Patent Document 4: European Journal of Pharmacology, 1996, vol. 298, pages 51 to 55.

Disclosure of the Invention

Problems that the Invention is to Solve

An object of the invention is to provide a piperidine derivative having, for example, an antagonistic activity at

NR2B/NMDA receptor, etc. or a pharmaceutically acceptable salt thereof.

Means for Solving the Problems

The present invention relates to the following (1) to (37).

(1) A piperidine derivative represented by the formula (I)

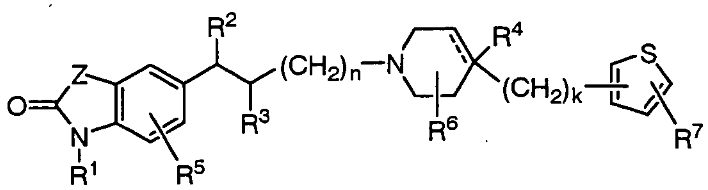

( I )

{Wherein,
-C(=O)-Z- represents -C(=O)-$CH_2$-, -C(=O)-$C(CH_3)_2$-, -C(=O)-NH-, -C(=O)-O-, -C(=O)-S-, -C(=O)-$CH_2CH_2$-, -C(=O)-CH=CH-, -C(=O)-$CH_2$O-, -C(=O)-$CH_2$S-, -C(=O)-$CH_2CH_2CH_2$-, or -C(=O)-$NR^8CH_2$- (wherein, $R^8$ represents a hydrogen atom, or substituted or unsubstituted lower alkyl);
$R^1$ represents a hydrogen atom, substituted or unsubstituted lower alkyl, substituted or unsubstituted lower alkenyl, or substituted or unsubstituted lower alkynyl;
$R^2$ represents a hydrogen atom or hydroxy;
$R^3$ represents a hydrogen atom, substituted or unsubstituted lower alkyl, substituted or unsubstituted lower alkenyl, or substituted or unsubstituted lower alkynyl;
$R^5$ represents a hydrogen atom, hydroxy, substituted or unsubstituted lower alkyl, substituted or unsubstituted lower alkenyl, substituted or unsubstituted lower alkynyl, or substituted or unsubstituted lower alkoxy;
$R^6$ represents a hydrogen atom, hydroxy, substituted or unsubstituted lower alkyl, substituted or unsubstituted lower alkenyl, or substituted or unsubstituted lower alkynyl;
$R^7$ represents a hydrogen atom, halogen, substituted or unsubstituted lower alkyl, substituted or unsubstituted lower alkenyl, substituted or unsubstituted lower alkynyl, substituted or unsubstituted lower alkoxy, or -$(CH_2)_m$Y [wherein, m represents an integer of 1 to 4; Y represents - $NR^9R^{10}$ (wherein, $R^9$ and $R^{10}$ each represents independently a hydrogen atom, substituted or unsubstituted lower alkyl, or substituted or unsubstituted aralkyl, or $R^9$ and $R^{10}$ form a heterocyclic group together with adjacent nitrogen atom), or substituted or unsubstituted heterocyclic alkyl];
n and k each represents independently an integer of 0 to 2; and

$\overset{----}{—}$ represents a single bond or, a double bond together with $R^4$, and when $\overset{----}{—}$ is a single bond, $R^4$ represents a hydrogen atom, hydroxy, substituted or unsubstituted lower alkoxy, or halogen), or a pharmaceutically acceptable salt thereof.
(2) A piperidine derivative represented by the formula (IA)

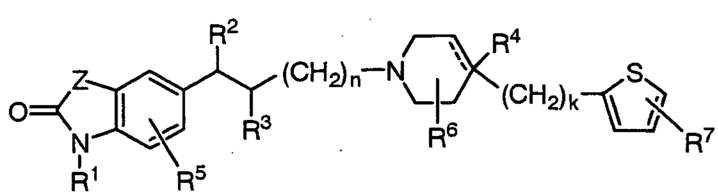

( IA )

(Wherein, $\overset{----}{—}$, -C(=O)-Z-, $R^1$, $R^2$, $R^3$, $R^4$, $R^5$, $R^6$, $R^7$, n and k have the same meaning as defined above, respectively), or a pharmaceutically acceptable salt thereof.
(3) The piperidine derivative or a pharmaceutically acceptable salt thereof according to (1) or (2), wherein - C(=O)-Z- is -C(=O)-S-.
(4) The piperidine derivative or a pharmaceutically acceptable salt thereof according to (1) or (2), wherein - C(=O)-Z-

is $-C(=O)-CH_2-$.

(5) The piperidine derivative or a pharmaceutically acceptable salt thereof according to (1) or (2), wherein - C(=O)-Z- is $-C(=O)-CH_2CH_2-$.

(6) The piperidine derivative or a pharmaceutically acceptable salt thereof according to (1) or (2), wherein - C(=O)-Z- is $-C(=O)-CH_2CH_2CH_2-$.

(7) The piperidine derivative or a pharmaceutically acceptable salt thereof according to any one of (1) to (6), wherein 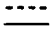 is a single bond.

(8) The piperidine derivative or a pharmaceutically acceptable salt thereof according to any one of (1) to (6), wherein $R^4$ is hydroxy.

(9) The piperidine derivative or a pharmaceutically acceptable salt thereof according to any one of (1) to (6), wherein $R^4$ is a hydrogen atom or hydroxy.

(10) The piperidine derivative or a pharmaceutically acceptable salt thereof according to any one of (1) to (9), wherein $R^6$ is a hydrogen atom.

(11) The piperidine derivative or a pharmaceutically acceptable salt thereof according to any one of (1) to (9), wherein a binding position of $R^6$ is 3-position on the morpholine ring, and $R^6$ is hydroxy.

(12) The piperidine derivative or a pharmaceutically acceptable salt thereof according to any one of (1) to (11), wherein $R^2$ is a hydrogen atom or hydroxy.

(13) The piperidine derivative or a pharmaceutically acceptable salt thereof according to any one of (1) to (11), wherein $R^2$ is hydroxy.

(14) The piperidine derivative or a pharmaceutically acceptable salt thereof according to any one of (1) to (13), wherein $R^3$ is a hydrogen atom or lower alkyl.

(15) The piperidine derivative or a pharmaceutically acceptable salt thereof according to any one of (1) to (13), wherein $R^3$ is a hydrogen atom.

(16) The piperidine derivative or a pharmaceutically acceptable salt thereof according to any one of (1) to (15), wherein n is 0.

(17) The piperidine derivative or a pharmaceutically acceptable salt thereof according to any one of (1) to (16), wherein k is 0.

(18) The piperidine derivative or a pharmaceutically acceptable salt thereof according to any one of (1) to (17), wherein $R^5$ is a hydrogen atom.

(19) The piperidine derivative or a pharmaceutically acceptable salt thereof according to any one of (1) to (18), wherein $R^7$ is a hydrogen atom.

(20) The piperidine derivative or a pharmaceutically acceptable salt thereof according to any one of (1) to (19), wherein the piperidine derivative is a racemate.

(21) The piperidine derivative or a pharmaceutically acceptable salt thereof according to any one of (1) to (19), wherein the piperidine derivative is an optically active compound.

(22) A pharmaceutical composition comprising the piperidine derivative or a pharmaceutically acceptable salt thereof according to any one of (1) to (21) as an active ingredient.

(23) An N-methyl-D-aspartic acid (NMDA) type glutamic acid receptor containing NR2B subunit (NR2B/NMDA receptor) antagonist, which comprises the piperidine derivative or a pharmaceutically acceptable salt thereof according to any one of (1) to (21) as an active ingredient.

(24) A selective antagonist of NR2B/NMDA receptor (a selective NR2B/NMDA receptor antagonist) which comprises the piperidine derivative or a pharmaceutically acceptable salt thereof according to any one of (1) to (21) as an active ingredient.

(25) A therapeutic agent for a diseases in which an NR2B subunit of an NMDA receptor is participated, which comprises the piperidine derivative or a pharmaceutically acceptable salt thereof according to any one of (1) to (21) as an active ingredient.

(26) A therapeutic agent for pain which comprises the piperidine derivative or a pharmaceutically acceptable salt thereof according to any one of (1) to (21) as an active ingredient.

(27) A therapeutic agent for neurogenic pain which comprises the piperidine derivative or a pharmaceutically acceptable salt thereof according to any one of (1) to (21) as an active ingredient.

(28) A method for antagonizing at an NR2B/NMDA receptor, which comprises administering an effective amount of the piperidine derivative or a pharmaceutically acceptable salt thereof according to any one of (1) to (21).

(29) A method for selective antagonizing at an NR2B/NMDA, receptor, which comprises administering an effective amount of the piperidine derivative or a pharmaceutically acceptable salt thereof according to any one of (1) to (21).

(30) A method for treating a disease in which an NR2B subunit of an NMDA receptor is participated, which comprises administering an effective amount of the piperidine derivative or a pharmaceutically acceptable salt thereof according to any one of (1) to (21).

(31) A method for treating pain, which comprises administering an effective amount of the piperidine derivative or a pharmaceutically acceptable salt thereof according to any one of (1) to (21).

(32) A method for treating neurogenic pain, which comprises administering an effective amount of the piperidine derivative or a pharmaceutically acceptable salt thereof according to any one of (1) to (21).

(33) Use of the piperidine derivative or a pharmaceutically acceptable salt thereof according to any one of (1) to (21) for the manufacture of an NR2B/NMDA receptor antagonist.

(34) Use of the piperidine derivative or a pharmaceutically acceptable salt thereof according to any one of (1) to (21) for the manufacture of a selective NR2B/NMDA receptor antagonist.

(35) Use of the piperidine derivative or a pharmaceutically acceptable salt thereof according to any one of (1) to (21) for the manufacture of a therapeutic agent for a diseases in which an NR2B subunit of an NMDA receptor is participated.

(36) Use of the piperidine derivative or a pharmaceutically acceptable salt thereof according to any one of (1) to (21) for the manufacture of a therapeutic agent for pain.

(37) Use of the piperidine derivative or a pharmaceutically acceptable salt thereof according to any one of the above (1) to (21) for the manufacture of a therapeutic agent for neurogenic pain.

Advantages of the Invention

In accordance with the present invention, there are provided, for example, a piperidine derivative or a pharmaceutically acceptable salt thereof having an antagonistic activity at an NR2B/NMDA receptor, etc.; and an agent for the treatment of a disease in which an NR2B subunit of an NMDA receptor is participated (for example, a disease due to degeneration and injury of nerve cells such as epilepsy, stroke, anxiety, cerebral ischemia, muscle spasm, Alzheimer's disease, dementia, Huntington's disease, Parkinson's disease, cerebral apoplexy, cerebral infarction and the like, and pain such as chronic pain, neurogenic pain, headache, migraine and the like) comprising the piperidine derivative or a pharmaceutically acceptable salt thereof as an active ingredient.

Brief Description of the Drawings

[Fig. 1] Fig. 1 shows an improvement effect of allodynia by Compound 6 in strangulated nerve-injured rats. An ordinate shows a pain threshold (g) and an abscissa shows time course (hour(s)) after administration of Compound 6 or a solvent.

[Fig. 2] Fig. 2 shows an improvement effect of allodynia by Compound 10 in strangulated nerve-injured rats. An ordinate shows a pain threshold (g) and an abscissa shows time course (hour(s)) after administration of Compound 10 or a solvent.

Explanation of Marks

Fig. 1

-•- Compound 6 treated group (3 mg/kg)
-▲- Compound 6 treated group (10 mg/kg)
-♦- Compound 6 treated group (30 mg/kg)
-o- Solvent treated group
-□- a normal rat group

Fig. 2

-•- Compound 10 treated group (0.3 mg/kg)
-▲- Compound 10 treated group (1 mg/kg)
-♦- Compound 10 treated group (3 mg/kg)
-o- Solvent treated group
-□- a normal rat group

Best Mode for Carrying Out the Invention

In the definition of each group in the formula (I) and (IA):

Examples of the lower alkyl and the lower alkyl moiety of the lower alkoxy include, for example, straight or branched alkyl having 1 to 6 carbon atoms, such as methyl, ethyl, propyl, isopropyl, butyl, isobutyl, sec-butyl, tert-butyl, pentyl, neopentyl, hexyl and the like.

Examples of the lower alkenyl include, for example, straight or branched alkenyl having 2 to 6 carbon atoms, such as vinyl, allyl, 1-propenyl, isopropenyl, 2-butenyl, 3-butenyl, 2-methylallyl, 2-pentenyl, 4-pentenyl, 2-hexenyl, 5-hexenyl and the like.

Examples of the lower alkynyl include, for example, straight or branched alkynyl having 2 to 6 carbon atoms, such as ethynyl, propynyl, 2-butynyl, 3-butynyl, 2-pentynyl, 4-pentynyl, 2-hexynyl, 5-hexynyl, 4-methyl-2-pentynyl and the like.

The halogen means each of atom of fluorine, chlorine, bromine and iodine.

Examples of the aralkyl include, for example, aralkyl having 7 to 15 carbon atoms, such as benzyl, phenethyl, phenylpropyl, phenylbutyl, benzhydryl, naphthylmethyl, naphthylethyl and the like.

The alkylene moiety of the heterocyclic alkyl is the same meaning as a group where one hydrogen atom is elminated from the aforementioned lower alkyl.

Examples of a heterocyclic group moiety of the heterocyclic alkyl include aromatic heterocyclic group and alicyclic heterocyclic group.

Examples of the aromatic heterocyclic group include, for example, a 5- or 6-membered monocyclic aromatic heterocyclic group containing at least one atom selected from a nitrogen atom, an oxygen atom, and a sulfur atom; and a bicyclic or tricyclic condensed aromatic heterocyclic group in which 3- to 8-membered rings are condensed and containing at least one atom selected from a nitrogen atom, an oxygen atom, and a sulfur atom. More specific examples include furyl, thienyl, pyrrolyl, imidazolyl, pyrazolyl, oxazolyl, isoxazolyl, oxadiazolyl, oxo-oxadiazolyl, thiazolyl, isothiazolyl, thiadiazolyl, triazolyl, tetrazolyl, pyridyl, pyridazinyl, oxopyridazinyl, pyrimidinyl, pyrazinyl, triazinyl, benzofuranyl, benzothienyl, benzothiazolyl, benzoxazolyl, indolyl, isoindolyl, indazolyl, benzimidazolyl, benztriazolyl, phthalazinyl, quinazolinyl, quinoxalinyl, naphthylidinyl, cinnolinyl, quinolyl, isoquinolyl, purinyl, dibenzofuranyl and the like.

Examples of the alicyclic heterocyclic group include, for example, a 5- to 9-membered monocyclic alicyclic heterocyclic group containing at least one atom selected from a nitrogen atom, an oxygen atom, and a sulfur atom; and a bicyclic or tricyclic condensed alicyclic heterocyclic group in which 3- to 8-membered rings are condensed and containing at least one atom selected from a nitrogen atom, an oxygen atom, and a sulfur atom. More specific examples include pyranyl, thiopyranyl, pyrrolidinyl, oxopyrrolidinyl, piperidino, piperidinyl, perhydroazepinyl, oxiranyl, tetrahydrofuranyl, tetrahydropyranyl, imidazolidinyl, pyrazolidinyl, pieperazinyl, oxopiperazinyl, homopiperazinyl, oxazolidinyl, oxo-oxazolidinyl, morpholino, morpholinyl, thiazolidinyl, thiomorpholino, thiomorpholinyl, tetrahydropyridinyl, indolinyl, isoindolinyl, dihydrobenzofuranyl, dihydrobenzimidazolyl, dihyrobenzoxazolyl, dihydrobenzothiazolyl, dihydroquinolyl, tetrahydroquinolyl, dihydroisoquinolyl, tetrahydroisoquinolyl, oxazolinyl, oxadiazolinyl, dioxoranyl, tetrahydroquinozolinyl, tetrahydroquinoxalinyl, chromanyl, isochromanyl, benzodioxolyl, dihydrobenzodioxanyl, benzodioxepinyl, benzopyranyl, perhydrodiazepinyl, perhydrodiazocinyl, perhydrodiazoninyl and the like.

Examples of the heterocyclic group formed together with the adjacent nitrogen atom are five-, six- or seven-membered monocyclic heterocyclic group containing at least one nitrogen atom, and a fused bicyclic heterocyclic group comprising six members and six members. More specific examples include pyrrolyl, imidazolyl, imidazolidinyl, pyrrolidinyl, piperidino, perhydroazepinyl, piperazinyl, morpholino, thiomorpholino, homopiperazinyl, tetrahydropyridyl, tetrahydropyrazinyl, tetrahydroquinolyl, tetrahydroisoquinolyl, indolyl, isoindolyl, dihydroindolyl and the like.

Examples of the substituents (A) in the substituted lower alkyl, the substituted lower alkenyl, the substituted lower alkynyl and the substituted lower alkoxy may be the same or different, and include, for example, one to three substituent(s) such as hydroxy, cyano, nitro, carboxy, carbamoyl, halogen, lower alkoxy, cycloalkyl, lower alkanoyl, lower alkoxycarbonyl and the like.

The halogen and the lower alkoxy exemplified for the substituents (A) are the same meanings mentioned above, respectively; the lower alkyl moiety of the lower alkanoyl and the lower alkoxycarbonyl is the same meaning as the lower alkyl mentioned above; and examples of the cycloalkyl include cycloalkyl having 3 to 8 carbon atoms such as cyclopropyl, cyclobutyl, cyclopentyl, cyclohexyl, cycloheptyl, cyclooctyl and the like.

Examples of the substituents (B) in the substituted aralkyl, the substituted heterocyclic alkyl and the substituted heterocyclic group formed together with the adjacent nitrogen atom include lower alkyl, substituted lower alkyl, lower alkenyl, lower alkynyl and the like in addition to the groups exemplified for the above-mentioned substituents (A).

The lower alkyl, the lower alkenyl and the lower alkynyl exemplified for the substituents (B) are the same meanings as mentioned above, respectively; examples of the substituents (a) in the substituted lower alkyl for the substituents (B) may be same or defferent, and include, for example, one to three substituent(s) such as halogen and the like; and the halogen exemplified for the substituents (a) is the same meaning as defined above.

Hereinafter, the compounds represented by the formula (I) and (IA) are referred to as Compounds (I) and (IA), respectively. The other formula numbers are referred to in the same manner.

Among Compounds (I) and (IA), stereoisomers such as geometric isomer, optical isomer, tautomer and the like may exist, however, the present invention includes all possible isomers and a mixture thereof including the above-mentioned ones.

Examples of the optical isomers include optically active compound such as enantiomers, diastereomers and the like, a mixture thereof, a racemate and the like. Specific examples thereof include Compound (I) or (IA) where the configuration of -$CHR^2$-$CHR^3$- (wherein, $R^2$ and $R^3$ are the same meanings as defined above, respectively) is threo or erythro; that where the relative configuration of -$CHR^2$-$CHR^3$- (wherein, $R^2$ and $R^3$ are the same meanings as defined above, respectively) is (S*, S*) or (S*, R*); that where the absolute configuration of -$CHR^2$-$CHR^3$- (wherein, $R^2$ and $R^3$ are the same meanings as defined above, respectively) is (S, S), (R, R), (S, R) or (R, S); that where the configuration of - $CHR^2$-$CHR^3$- (wherein, $R^2$ and $R^3$ are the same meanings as defined above, respectively) is the mixture thereof; the Compound (I) or (IA) in which $R^3$ is hydrogen, where the steric configuration of -$CHR^2$- (wherein, $R^2$ is the same meaning as defined above) is (R) or (S); and that where the configuration of -$CHR^2$- (wherein, $R^2$ is the same meaning as defined above) is the mixture thereof.

Examples of the pharmaceutically acceptable salt of the compounds (I) and (IA) include, for example, pharmaceutically acceptable acid addition salts, metal salts, ammonium salts, organic amine addition salts, amino acid addition salts and the like. Examples of the acid addition salts inclide, for example, inorganic acid salts such as hydrochloride, sulfate, phosphate and the like; and organic acid salts such as acetate, maleate, fumarate, tartrate, citrate, methanesulfonate and the like. Examples of the pharmaceutically acceptable metal salts include alkaline metal salts such as sodium salts, potassium salts and the like; alkaline earth metal salts such as magnesium salts, calcium salts and the like; aluminum salts; zinc salts and the like. Examples of the pharmaceutically acceptable ammonium salt include salts of ammonium, tetramethylammonium or the like. Examples of the pharmaceutically acceptable organic amine addition salts include addition salts of morpholine, piperidine or the like. Examples of the pharmaceutically acceptable amino acid addition salts include addition salts of lysine, glycine, phenylalanine or the like.

Compound (I) or (IA) or a pharmaceutically acceptable salt thereof may be used for the treatment of diseases in which, for example, an NR2B subunit of an NMDA receptor is participated. As the diseases in which an NR2B subunit of an NMDA receptor is participated, for example, any disease caused by hyperfunction of an NMDA receptor may be included, and specific examples thereof include a disease due to degeneration and injury of nerve cells such as epilepsy, stroke, anxiety, cerebral ischemia, muscle spasm, Alzheimer's disease, dementia, Huntington's disease, Parkinson's disease, cerebral apoplexy, cerebral infarction and the like, and pain such as chronic pain, neurogenic pain, headache, migraine and the like.

Next, Production Methods of Compound (I) will be described below.

In the Production Methods described below, when a defined group changes under the reaction conditions or is not suitable for carrying out the method, it is possible to obtain the desired compound easily using a method commonly used in synthetic organic chemistry such as protection/deprotection of functional group [for example, Protective Groups in Organic Synthesis, third edition, T. W. Greene, John Wiley & Sons Inc. (1999)]. If necessary, the order of reaction processes such as introduction of substituents can be changed.

Compound (I) may be prepared, for example, according to the Production Method mentioned as follows.

Production Method 1:

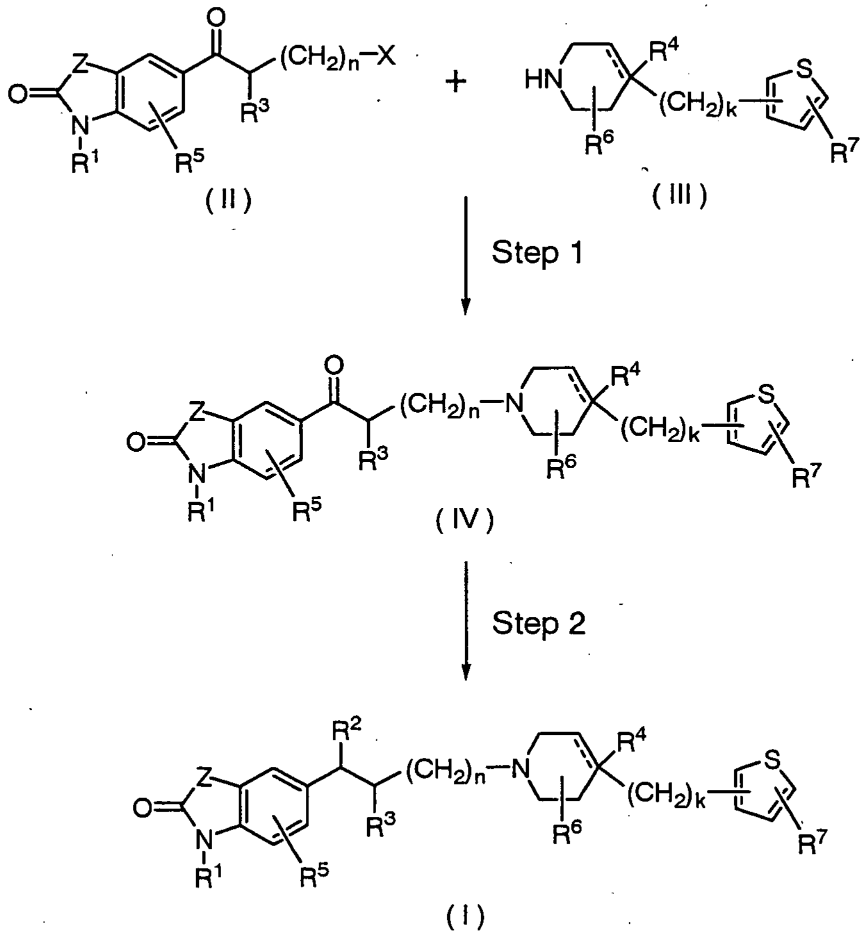


(II) (III) (IV) (I)

[Whereine, $\overline{\underline{\;\;\;\;}}$, -C(=O)-Z-, $R^1$, $R^2$, $R^3$, $R^4$, $R^5$, $R^6$, $R^7$, n and k have the same meanings as defined above, respectively, and X represents halogen (said halogen has the same meaning as mentioned above)].

[Step 1]

Compound (IV) can be prepared by reacting Compound (II) with Compound (III) in a solvent and in the presence of a base in an amount of 1 to 10 equivalent(s), preferably 1 to 5 equivalent(s) to Compound (II). Also, Compound (IV) can be prepared by reacting Compound (II) with an excessive amount of Compound (III) in a solvent. If necessary, the above reactions may be carried out in the presence of water or in the presence of sodium iodide.

The reaction is usually carried out at a temperature between 0°C and the boiling point of the solvent used, preferably at room temperature, for 1 to 96 hour(s), preferably for 3 to 50 hours.

As the base, for example, an organic base such as triethylamine, pyridine, dimethylaminopyridine, N-methylmorpholine, N,N-diisopropylamine and the like, or an inorganic base such as sodium carbonate, potassium carbonate and the like can be used. Triethylamine is particularly preferred.

Examples of the solvent include, for example, chloroform, methylene chloride, pyridine, tetrahydrofuran (THF), 1,2-dimethoxyethane, toluene, ethyl acetate, methanol, ethanol, acetonitrile, dimethylformamide (DMF), hexamethyl phosphoramide (HMPA) and the like, and each of them may be used solely or in combination thereof. DMF and HMPA are particularly preferred.

Compound (II) can be prepared by a method mentioned in, for example, JP-A-52-118465, JP-T-6-504293, JP-A-51-118770; JP-A-56-156263 and JP-A-2-72173, or by analogous methods thereto. Compound (III) can be prepared by a method mentioned in, for example, JP-A-48-56687, or by analogous methods thereto.

[Step 2]

Among Compound (I), Compound (Ia) in which $R^2$ is hydroxy can be prepared by reducing Compound (IV) in a solvent using a metal hydride in an amount of 1 to 10 equivalent(s) to Compound (IV).

The reaction is usually carried out at a temperature between 0˚C and the boiling point of the solvent used, preferably at room temperature, for 5 minutes to 48 hours, preferably for 5 to 10 hours.

As the solvent, for example, an alcohol such as methanol, ethanol and the like can be used.

As the metal hydride, for example, sodium borohydride, potassium borohydride or the like can be used.

Compound (Ia) can also be prepared by reducing Compound (IV) in a solvent using, for example, a catalytic amount of a catalytic hydrogenation catalyst such as a Pd catalyst under a hydrogen atmosphere.

The reaction is usually carried out at a temperature between 0˚C and the boiling point of the solvent used, preferably at a temperature between 30˚C and 50˚C, for one hour to one week, preferably for 13 to 20 hours. If necessary, this reaction may be carried out in the presence of an acid such as hydrochloric acid, sulfuric acid and the like.

As the solvent, for example, an alcohol such as methanol, ethanol and the like, acetic acid or the like can be used.

Among Compound (I), Compound (Ib) in which $R^2$ is a hydrogen atom can be prepared by reducing Compound (Ia) or Compound (IV) in a solvent under a hydrogen atmosphere using, for example, catalytic hydrogenation catalyst such as a Pd catalyst. If necessary, this reaction may be carried out in the presence of an acid such as hydrochloric acid, sulfuric acid and the like.

Also, Compound (Ib) can be prepared by reducing Compound (Ia) in an acidic solvent such as trifluoroacetic acid using, for example, triethylsilane or the like.

The reaction from Compound (Ia) is usually carried out at a temperature between 0˚C and the boiling point of the solvent used, or preferably at a temperature between 30˚C and 50˚C, for one hour to one week, preferably for 13 to 20 hours. The reaction from Compound (IV) is usually carried out at a temperature between 0˚C and the boiling point of the solvent used, preferably at a temperature between 30˚C and 50˚C, for one hour to two weeks, preferably for 13 to 72 hours.

As the solvent in the reduction using a catalytic hydrogenation catalyst, an alcohol such as methanol, ethanol and the like may be used.

Among Compound (I), Compound (Ic) in which n is 1 can also be prepared by, for example, the Production Method as mentioned below.

Production Method 2:

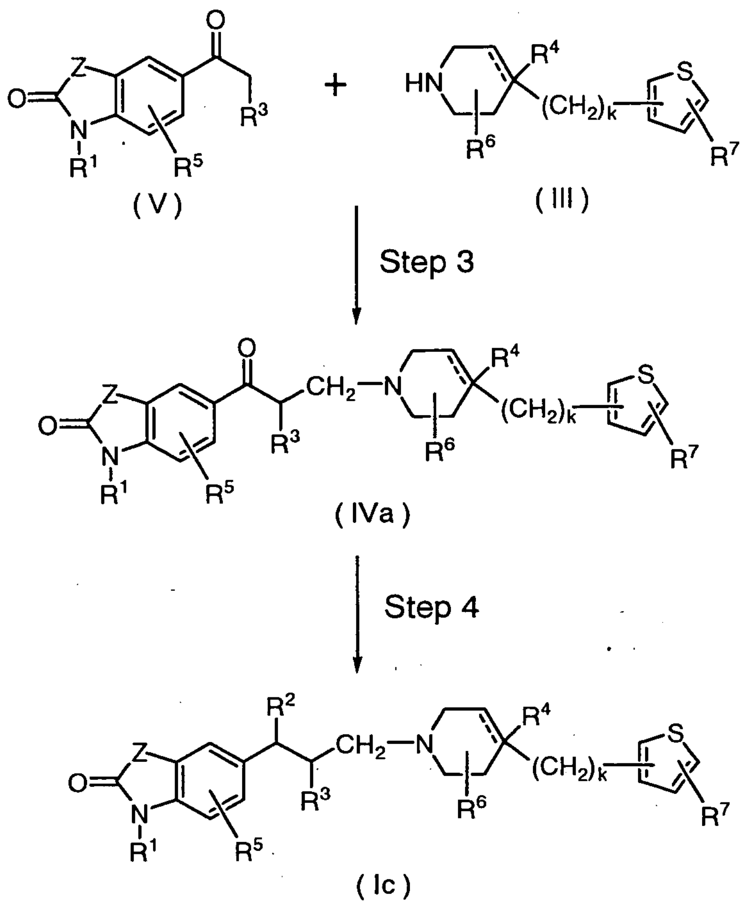


(V) (III) (IVa) (Ic)

(Whereine, ----, -C(=O)-Z-, $R^1$, $R^2$, $R^3$, $R^4$, $R^5$, $R^6$, $R^7$ and k have the same meanings as defined above, respectively).

[Step 3]

Compound (IVa) can be prepared reacting Compound (V) with Compound (III) in an amount of 1 to 2 equivalent (s) to Compound (V) and a precursor of formaldehyde such as paraformaldehyde or aqueous formalin in an amount of 1 to 5 equivalent(s) in a solvent and in the presence of an acid in an amount of a catalytic amount to 5 equivalents to Compound (II). If necessary, this reaction may be carried out in the presence of water.

The reaction is usually carried out at a temperature between 0˚C and the boiling point of the solvent used, preferably at room temperaturem, for 1 to 48 hour(s), preferably for 1 to 24 hours(s).

As the acid, an inorganic acid such as hydrochloric acid, sulfuric acid and the like can be used. Hydrochloric acid is particularly preferred.

Examples of the solvent include, for example, methanol, ethanol, DMF and the like, and each of them may be used solely or in combination thereof. DMF is particularly preferred.

Compound (V) can be prepared by a method mentioned in, for example, JP-A-51-115480, or by analogous methods thereto.

[Step 4]

Cmpound (Ic) can be prepared from the compound (IVa) by an analogous method to that mentioned in the Step 2 of the Production Method 1.

An optically active isomer of Compound (I) can be prepared by subjecting a racemate or a mixture of enantiomer of Compound (I) prepared in the above-mentioned Production Method 1 or 2 to a common separating procedure (optical resolution; "Separation of Optical Isomer" in Kikan Kagaku Sosetsu 6 (Quarterly Review of Chemistry), edited by the Chemical Society of Japan, 1989). Examples of optical resolution include, for example, separation by a high-performance liquid chromatography using an optically active stationary phase, separation by crystallization using a preferential crystallization, a diastereomer method and the like, and an optical resolution using an enzyme, and the like. If necessary, they may be conducted in combination thereof.

An optically active isomer of Compound (I) may to be prepared by, for example, enantio-selective or diastereoselective asymmetric synthesis such as enzymatic asymmetric synthesis, asymmetric reduction and asymmetric hydrolysis (for example, "Separation of Optical Isomer" in Kikan Kagaku Sosetsu 6 (Quarterly Review of Chemistry), edited by the Chemical Society of Japan, 1989; WO 97/20789; etc.).

For example, Among Compound (Ia), optical active isomers, an (R) isomer and an (S) isomer of Compound (Id) in which $R^3$ is a hydrogen atom may be prepared by the following Production Method as well.

Production Method 3:

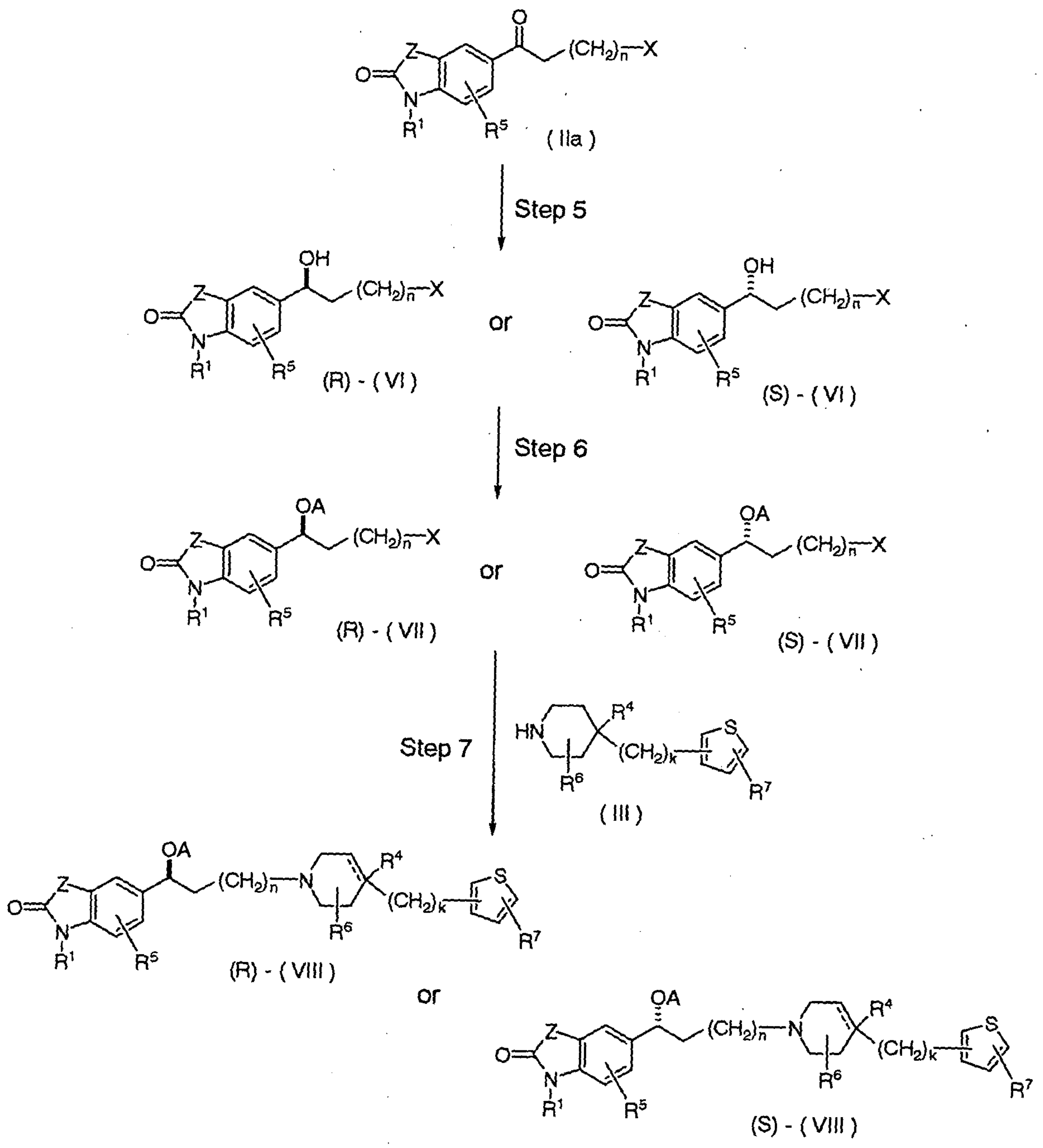


( IIa )

(R) - ( VI ) or (S) - ( VI )

(R) - ( VII ) or (S) - ( VII )

( III )

(R) - ( VIII ) or (S) - ( VIII )

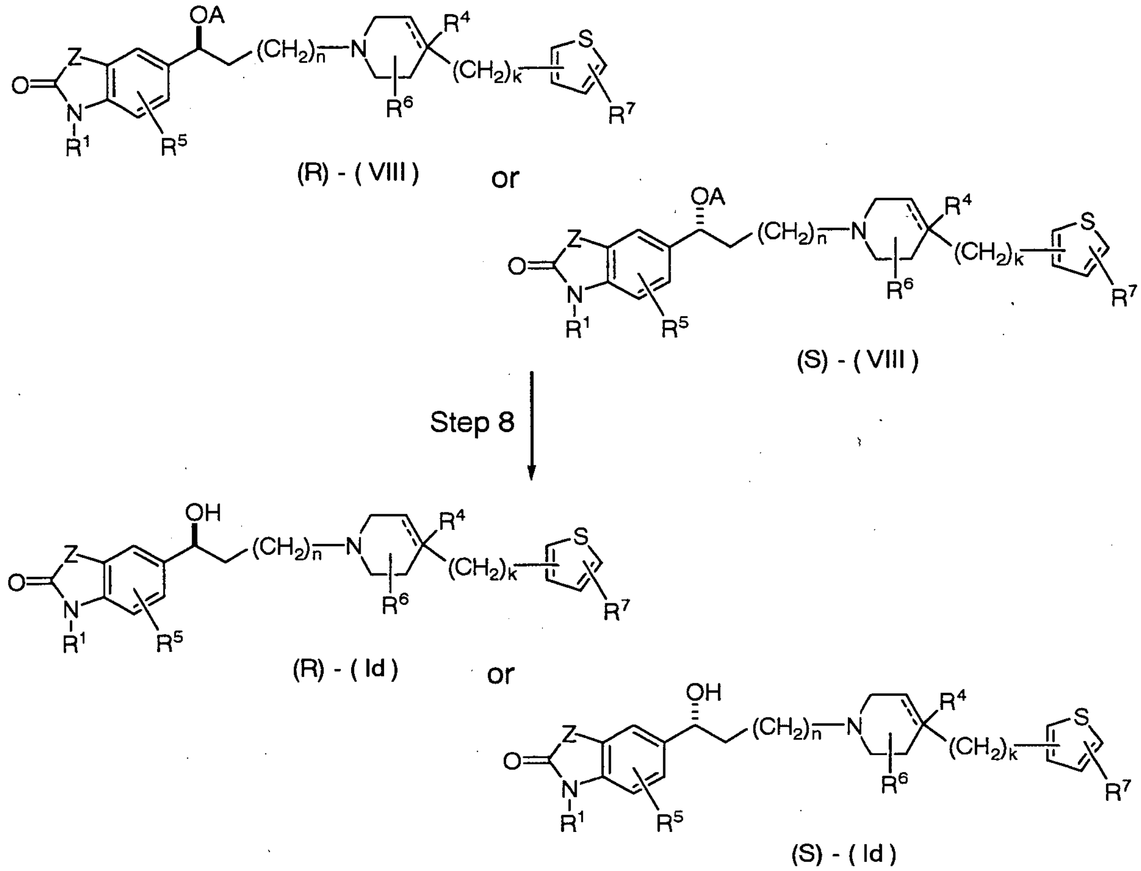


(R) - ( VIII ) or (S) - ( VIII )

(R) - ( Id ) or (S) - ( Id )

(Whereine, ----, -C(=O)-Z-, $R^1$, $R^4$, $R^5$, $R^6$, $R^7$, k and n have the same meanings as defined above, respectively; and A represents a protective group for hydroxyl group such as trimethylsilyl, triethylsilyl, tert-butyldimethylsilyl and the like).

[Step 5]

Among Compound (II), Compound (IIa) in which $R^3$ is a hydrogen atom is reduced in an inert solvent or without solvent, in the presence of the corresponding asymmetric transition metal complex and a hydrogen donor, and also in the presence or absence of a base to be able to provide each of the desired (R) and (S) isomers of Compound (VI), selectively.

The reaction is usually carried out at a temperature between -50°C and the boiling point of the solvent used, preferably at a temperature between 10°C and 50°C, for 5 minutes to 100 hours.

Examples of the asymmetric transition metal complex include, for example,
chloro[(1S,2S)-N-(p-toluenesulfonyl)-1,2-diphenylethylenediamine](mesitylene) ruthenium;
chloro[(1R,2R)-N-(p-toluenesulfonyl)-1,2-diphenylethylenediamine](mesitylene) ruthenium;
chloro[(1S,2S)-N-(p-toluenesulfonyl)-1,2-diphenylethylenediamine](p-cymene) ruthenium;
chloro[(1R,2R)-N-(p-toluenesulfonyl)-1,2-diphenylethylenediamine](p-cymene) ruthenium;
chloro[(1S,2S)-N-(p-toluenesulfonyl)-1,2-diphenylethylenediamine](pentamethylcyclopentadienyl) ruthenium;
chloro[(1R,2R)-N-(p-toluenesulfonyl)-1,2-diphenylethylenediamine](pentamethylcyclopentadienyl) ruthenium;
chloro[(1S,2S)-N-methanesulfonyl-1,2-diphenylethylenediamine](pentamethylcyclopentadienyl) ruthenium;
chloro[(1R,2R)-N-methanesulfonyl-1,2-diphenylethylenediamine](pentamethylcyclopentadienyl) ruthenium;
hydride[(1S,2S)-N-(p-toluenesulfonyl)-1,2-diphenylethylenediamine](pentamethylcyclopentadienyl) ruthenium;
hydride[(1R,2R)-N-(p-toluenesulfonyl)-1,2-diphenylethylenediamine](pentamethylcyclopentadienyl) ruthenium;
chloro[(1S,2S)-N-(p-toluenesulfonyl)-1,2-diphenylethylenediamine](mesitylene) rhodium;
chloro[(1R,2R)-N-(p-toluenesulfonyl)-1,2-diphenylethylenediamine](mesitylene) rhodium;
chloro[(1S,2S)-N-(p-toluenesulfonyl)-1,2-diphenylethylenediamine](p-cymene) rhodium;

chloro[(1R,2R)-N-(p-toluenesulfonyl)-1,2-diphenylethylenediamine](p-cymene) rhodium;
chloro[(1S,2S)-N-(p-toluenesulfonyl)-1,2-diphenylethylenediamine](pentamethylcyclopentadienyl) rhodium;
chloro[(1R,2R)-N-(p-toluenesulfonyl)-1,2-diphenylethylenediamine](pentamethylcyclopentadienyl) rhodium;
chloro[(1S,2S)-N-methanesulfonyl-1,2-diphenylethylenediamine](pentamethylcyclopentadienyl) rhodium;
chloro[(1R,2R)-N-methanesulfonyl-1,2-diphenylethylenediamine](pentamethylcyclopentadienyl) rhodium;
hydride[(1S,2S)-N-(p-toluenesulfonyl)-1,2-diphenylethylenediamine](pentamethylcyclopentadienyl) rhodium;
hydride[(1R,2R)-N-(p-toluenesulfonyl)-1,2-diphenylethylenediamine](pentamethylcyclopentadienyl) rhodium;
chloro[(1S,2S)-N-(p-toluenesulfonyl)-1,2-diphenylethylenediamine](mesitylene) irridium;
chloro[(1R,2R)-N-(p-toluenesulfonyl)-1,2-diphenylethylenediamine](mesitylene) irridium;
chloro[(1S,2S)-N-(p-toluenesulfonyl)-1,2-diphenylethylenediamine](p-cymene) irridium;
chloro[(1R,2R)-N-(p-toluenesulfonyl)-1,2-diphenylethylenediamine](p-cymene) irridium;
chloro[(1S,2S)-N-(p-toluenesulfonyl)-1,2-diphenylethylenediamine](pentamethylcyclopentadienyl) irridium;
chloro[(1R,2R)-N-(p-toluenesulfonyl)-1,2-diphenylethylenediamine](pentamethylcyclopentadienyl) irridium;
chloro[(1S,2S)-N-methanesulfonyl-1,2-diphenylethylenediamine](pentamethylcyclopentadienyl) irridium;
chloro[(1R,2R)-N-methanesulfonyl-1,2-diphenylethylenediamine](pentamethylcyclopentadienyl) irridium;
hydride[(1S,2S)-N-(p-toluenesulfonyl)-1,2-diphenylethylenediamine](pentamethylcyclopentadienyl) irridium;
hydride[(1R,2R)-N-(p-toluenesulfonyl)-1,2-diphenylethylenediamine](pentamethylcyclopentadienyl) iridium; and the like.

When an asymmetric transition metal complex is available in the market, the commercial available one may be used as it is or after purification. When it is not available in the market, it may be prepared by a method mentioned in, for example, JP-A-11-335385, WO 97/20789, or by analogus methods thereto. The asymmetric transition metal complex is usually used, but not limited to, in an amount of 0.00001 to 1.0 equivalent, preferably 0.001 to 0.2 equivalent to Compound (IIa).

Examples of the hydrogen donor include, for example, an alcohol having a hydrogen atom at $\alpha$-position such as methanol, ethanol, 1-propanol, 2-propanol, 1-butanol, 2-butanol, 2-methyl-1-propanol, amyl alcohol, benzyl alcohol and the like; formic acid; a formate such as sodium formate, potassium formate, ammonium formate and the like; an unsaturated hydrocarbon having a partially saturated carbon bond such as tetrahydronaphthalene and the like; hydroquinone; and the like. The hydrogen donor is usually used, but not limited to, in an amount of 0.5 to 100 equivalent(s), preferably 1.0 to 10 equivalent(s) to Compound (IIa).

Examples of the base include, for example, an organic base such as triethylamine, diisopropylethylamine, 1,8-diazabicyclo[5.4.0]undec-7-ene, 1,5-diazabicyclo[4.3.0]nona-5-ene, diethylaniline, pyridine, lutidine, N-methylmorpholine and the like; a metal alkoxide such as sodium methoxide, sodium ethoxide, potassium tert-butoxide and the like; a carbonate such as sodium carbonate, potassium carbonate, calcium carbonate, cesium carbonate and the like; a metal hydroxide such as lithium hydroxide, sodium hydroxide, potassium hydroxide, calcium hydroxide and the like; and the like. The base is usually used, but not limited to, in an amount of 0.5 to 10,000 equivalent(s), preferably 1.0 to 500 equivalent(s), more preferably 1.0 to 50 equivalent(s) to the transition metal atom.

Examples of the inert solvent include, but not limited to so far as it is a solvent which is inert to the reaction, for example, an aliphatic hydrocarbon such as pentane, hexane, heptane, cyclohexane and the like; an aromatic hydrocarbon such as toluene, xylene and the like; a halogenated hydrocarbon such as methylene chloride, chloroform, dichloroethane and the like; a non-aromatic organic solvent such as acetonitrile, dimethyl sulfoxide, DMF, N,N-dimethylacetamide and the like; an ether such as dioxane, THF, diethyl ether, cyclopentyl methyl ether, dimethoxyethane, ethylene glycol dimethyl ether and the like; an ester such as methyl acetate, ethyl acetate, isopropyl acetate and the like; and water; and the like. Each of them may be used solely or in combination thereof.

[Step 6]

Each of the (R) and (S) isomers of Compound (VII) can be prepared by reacting the corresponding (R) isomer or (S) isomer of the compound (VI) with 1.0 to 10 equivalent(s), preferably 1.0 to 3.0 equivalent(s) of A-C1 (wherein, A has the same meaning as defined above) in an inert solvent and in the presence or absence of a base.

The reaction is usually carried out at a temperature between 0˚C and the boiling point of the solvent used, preferably at room temperature, for 1 to 48 hour(s), or preferably for 3 to 10 hours.

Examples of the base include, for example, an organic base such as triethylamine, diisopropylethylamine, 1,8-diazabicyclo[5.4.0]undec-7-ene, 1,5-diazabicyclo[4.3.0]nona-5-ene, diethylaniline, pyridine, lutidine, N-methylmorpholine, imidazole and the like; a metal alkoxide such as sodium methoxide, sodium ethoxide, potassium tert-butoxide and the like; a carbonate such as sodium carbonate, potassium carbonate, calcium carbonate, cesium carbonate and the like; a metal hydroxide such as lithium hydroxide, sodium hydroxide, potassium hydroxide, calcium hydroxide and the like; and the like. The base is usually used, but not limited to, in an amount of 1.0 to 50 equivalent(s), preferably 1.0 to 10 equivalent(s) to the (R) or (S) isomer of Compound (VI).

Example of the inert solvent include, but not limited to so far as it is a solvent which is inert to the reaction, for example, an aliphatic hydrocarbon such as pentane, hexane, heptane, cyclohexane and the like; an aromatic hydrocarbon such as toluene, xylene and the like; a halogenated hydrocarbon such as methylene chloride, chloroform, dichloroethane and the like; a non-aromatic organic solvent such as acetonitrile, dimethyl sulfoxide, DMF, N,N-dimethylacetamide and the like; an ether such as dioxane, THF, diethyl ether, cyclopentyl methyl ether, dimethoxyethane, ethylene glycol dimethyl ether and the like; an ester such as methyl acetate, ethyl acetate, isopropyl acetate and the like; water; and the like. Each of them may be used solely or in combination thereof.

[Step 7]

Each of the (R) and (S) isomers of Compound (VIII) can be prepared from corresponding (R) isomer or (S) isomer of Compound (VII) by an analogus method to that mentioned in the Step 1 of the Production Method 1.

[Step 8]

Each of the (R) and (S) isomers of Compound (Id) can be prepared by treating corresponding (R) isomer or (S) isomer of Compound (VIII) with an acid or tetrabutylammonium fluoride (TBAF) in an inert solvent. If necessary, this reaction may be carried out in the presence of a molecular sieve.
The reaction is usually carried out at a temperature between -10˚C and the boiling point of the solvent used, preferably at a temperature between 10˚C and 50˚C, for 5 minutes to 100 hours, preferably for 15 minutes to 24 hours.
Examples of the insert solvent include, but not limited to so far as it is a solvent inactive to the reaction, for example, dioxane, THF, diethyl ether, cyclopentyl methyl ether, dimethoxyethane, ethylene glycol dimethyl ether, methanol, ethanol, water and the like. Each of them may be used solely or in combination thereof.
Examples of the acid inclide, for example, an acidic aqueous solution of hydrochloric acid, sulfuric acid, methanesulfonic acid or the like. The acid or TBAF is usually used, but not limited to, in an amount of 1.0 to 50 equivalent(s), preferably 1.0 to 10 equivalent(s) to the (R) or (S) isomer of Compound (VIII).
Each of the (R) and (S) isomers of Compound (Id) can lso be prepared by the Production Method as mentioned below.

Production Method 4:

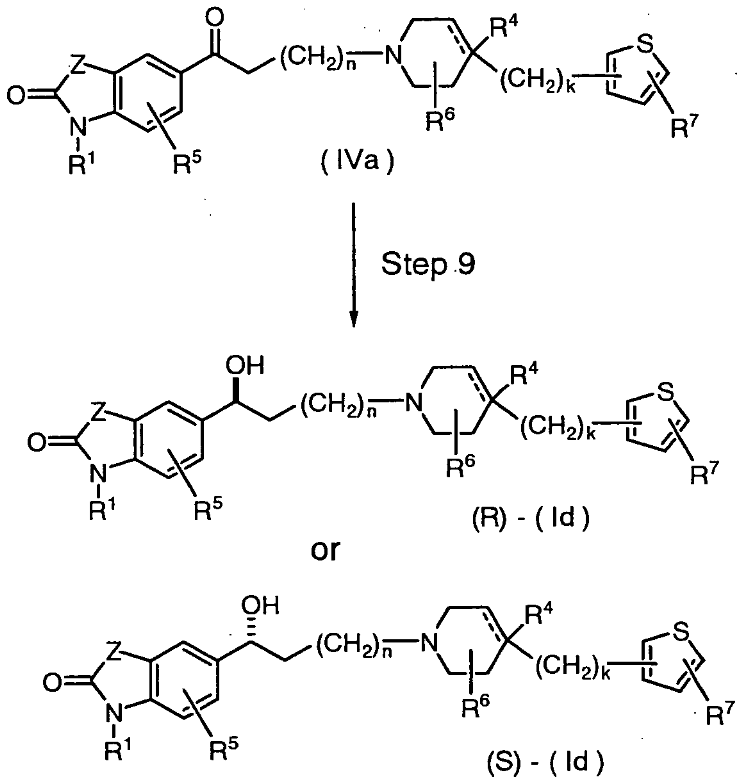


(IVa)

(R) - (Id)

or

(S) - (Id)

(Whereine, ----, -C(=O)-Z-, $R^1$, $R^4$, $R^5$, $R^6$, $R^7$, n and k have the same meanings as mentioned above.)

[Step 9]

The (R) isomer and (S) isomer of Compound (Id) can be prepared by the analogus method to that mentioned in the Step 5 of the Production Method 3 from Compound (IVa) prepared in the step 1 of the production process 1 which is the compound (IV) where $R^3$ is a hydrogen atom.

Intermediates and desired products in each of the above-mentioned Production Methods may be isolated and purified by separation and purification methods which have been commonly used in organic synthetic chemistry such as filtration, extraction, washing, drying, concentration, recrystallization and various kinds of chromatographic techniques. It is also possible that an intermediate is not particularly purified but is just subjected to the next reaction.

When a salt of Compound (I) or Compound (IA) is to be prepared, the product may be purified as it is in case Compound (I) or (IA) is obtained in a form of a salt, while in case it is obtained in a free form, Compound (I) or (IA) may be dissolved or suspended in an appropriate solvent and then an acid or a base is added thereto followed by subjecting to isolation and purification.

Compounds (I) and (IA) and pharmaceutically acceptable salts thereof may be also present in a form of adducts with water or various kinds of solvents, and those adducts are also included in the present invention.

Specific examples of the compound (I) obtaied by the present invention are shown in Tables 1 to 5. Numerals before the group show a substituted position.

[Table 1]

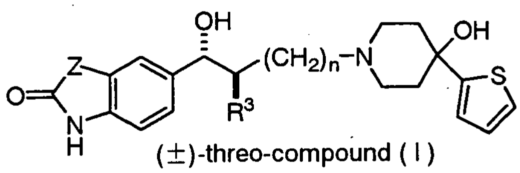

(±)-threo-compound (I)

| Compound No. | Z | $R^3$ | n |
|---|---|---|---|
| 1 | -S- | $CH_3$ | 0 |
| 3 | -S- | $C_2H_5$ | 0 |
| 6 | $-CH_2CH_2-$ | $CH_3$ | 0 |
| 8 | $-CH_2CH_2-$ | $C_2H_5$ | 0 |
| 12 | $-CH_2CH_2-$ | $CH_3$ | 1 |
| 14 | $-CH_2-$ | $CH_3$ | 0 |
| 17 | $-CH_2CH_2CH_2-$ | $CH_3$ | 0 |
| 19 | -NH- | $CH_3$ | 0 |
| 20 | -O- | $CH_3$ | 0 |
| 22 | $-CH_2S-$ | $CH_3$ | 0 |
| 24 | $-CH_2O-$ | $CH_3$ | 0 |

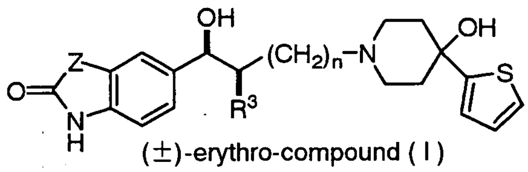

(±)-erythro-compound (I)

| Compound No. | Z | $R^3$ | n |
|---|---|---|---|
| 2 | -S- | $CH_3$ | 0 |
| 4 | -S- | $C_2H_5$ | 0 |
| 7 | $-CH_2CH_2-$ | $CH_3$ | 0 |
| 9 | $-CH_2CH_2-$ | $C_2H_5$ | 0 |
| 13 | $-CH_2CH_2-$ | $CH_3$ | 1 |
| 15 | $-CH_2-$ | $CH_3$ | 0 |
| 18 | $-CH_2CH_2CH_2-$ | $CH_3$ | 0 |
| | | | |
| 21 | -O- | $CH_3$ | 0 |
| 23 | $-CH_2S-$ | $CH_3$ | 0 |
| 25 | $-CH_2O-$ | $CH_3$ | 0 |

[Table 2]

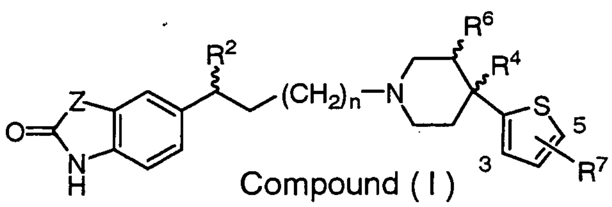

Compound (I)

| Compound No. | Z | $R^2$ | n | $R^4$ | $R^5$ | $R^7$ |
|---|---|---|---|---|---|---|
| 5 | -S- | OH | 0 | OH | H | H |
| 10 | $-CH_2CH_2-$ | OH | 0 | OH | H | H |
| 11 | $-CH_2CH_2$ | OH | 1 | OH | H | H |
| 16 | $-CH_2-$ | OH | 0 | OH | H | H |
| 34 | $-CH_2CH_2-$ | OH | 0 | OH | H | 5-Cl |
| 35 | $-CH_2CH_2-$ | OH | 0 | OH | H | $3-CH_3$ |
| 36 | $-CH_2CH_2-$ | OH | 0 | OH | H | $5-CH_3$ |
| 37 | $-CH_2CH_2-$ | H | 0 | OH | H | 5-Cl |
| 38 | $-CH_2CH_2-$ | H | 0 | OH | H | H |

(continued)

| Compound No. | Z | $R^2$ | n | $R^4$ | $R^5$ | $R^7$ |
|---|---|---|---|---|---|---|
| 39 | $-CH_2CH_2-$ | H | 0 | H | H | H |
| 40 | $-CH_2CH_2-$ | H | 0 | H | OH | H |
| 41 | $-CH_2CH_2-$ | H | 0 | OH | OH | H |
| 42 | $-CH_2CH_2-$ | OH | 0 | H | H | H |

[Table 3]

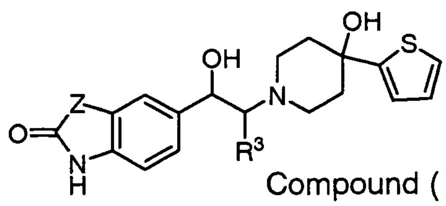

Compound ( I )

| Compound No. | Z | $R^3$ | Compound No. | Z | $R^3$ |
|---|---|---|---|---|---|
| 26 | $-CH_2-$ | H | 27 | $-CH_2-$ | H |
| 28 | $-CH_2CH_2-$ | H | 29 | $-CH_2CH_2-$ | H |
| 30 | $-CH_2CH_2-$ | $CH_3$ | 31 | $-CH_2CH_2-$ | $CH_3$ |
| 32 | $-CH_2CH_2CH_2-$ | H | 33 | $-CH_2CH_2CH_2-$ | H |

Steric configuration of $-CHOH-CHCH_3-$ of Compound 30 and Compound 31 in Table 3 is threo, and Compound 26 and Compound 27, Compound 28 and Compound 29, Compound 30 and Compound 31, and Compound 32 and Compound 33 each are in a relationships of enantiomers each other.

[Table 4]

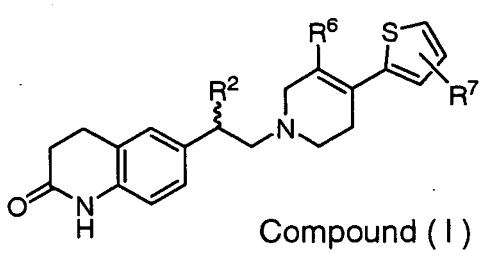

Compound ( I )

| Compound No. | $R^2$ | $R^6$ | $R^7$ |
|---|---|---|---|
| 43 | H | H | H |
| 44 | OH | H | H |

[Table 5]

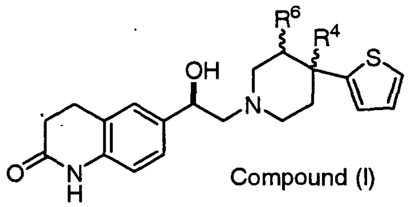

Compound (I)

| Compound No. | $R^4$ | $R^6$ | Steric Configuration on Piperidine Ring |
|---|---|---|---|
| 46 | H | OH | trans |
| 49 | OH | OH | cis |
| 50 | OH | OH | trans |

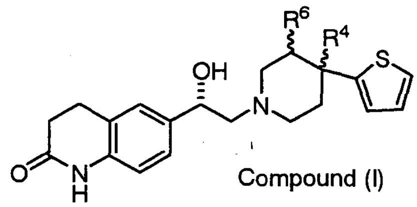

Compound (I)

| Compound No. | $R^4$ | $R^6$ | Steric Configuration on Piperidine Ring |
|---|---|---|---|
| 45 | H | OH | trans |
| 47 | OH | OH | cis |
| 48 | OH | OH | trans |

Next, the pharmacological effect of the representative Compounds (I) will be specifically illustrated by way of following Test Examples.

Test Example 1: Antagonistic activity at rat NMDA receptor NR2B subunit

The forebrain membrane specimen of male rats of an SD strain (final concentration: 250 μg protein/mL), [$^3$H]-ifenprodil [diluted with an HEPES buffer (20 mmol/L HEPES-KOH and 100 μmol/L trifluoroperazine (pH 7.4)) to give the final concentration of 4 nmol/L] and a test compound (each being diluted with an HEPES buffer to give the final concentration of 0.1 nmol/L to 1 μmol/L) were mixed, and incubated at 25°C for 90 minutes shading from light. The above-incubated membrane was recovered to a G/F-B filter using a cell harvester (Brandel; M-24) to stop the reaction. The resulting membrane was further washed with an HEPES buffer for three times, and radioactivity on the filter was measured (Beckman LS 6500). The antagonistic activity of the test compound for an NMDA receptor NR2B subunit was calculated by the following formula as an inhibiting rate for binding of [$^3$H]ifenprodil to the NR2B subunit of the NMDA receptor. The result is shown in Table 6 in terms of the concentration ($IC_{50}$ value) of the test compound for 50% inhibition of binding of [$^3$H]ifenprodil to the NR2B subunit of the NMDA receptor.

[Formula 1]

$$\text{Inhibiting Rate (\%)} = \frac{1 - \left[\left(\begin{array}{l}\text{Binding amount in the presence}\\ \text{of test compound}\end{array}\right) - \left(\begin{array}{l}\text{Non-specific}\\ \text{binding amount}\end{array}\right)\right]}{(\text{Total binding amount}) - (\text{Non-specific binding amount})} \times 100$$

Non-specific binding amount: Binding amount of [$^3$H]ifenprodil in the presence of 10 μmol/L of ifenprodil Total binding amount: Binding amount of [$^3$H]ifenprodil in the absence of the test compound Binding amount in the present of test compound: Binding amount of [$^3$H]ifenprodil in the presence of the test compound in each concentration

[Table 6]

| Compound No. | $IC_{50}$ (nmol/L) |
|---|---|
| 1 | 2.0 |
| 2 | 4.1 |
| 5 | 2.9 |
| 6 | 4.6 |
| 7 | 5.5 |
| 10 | 5.2 |

As shown in Table 6, Compound (I) inhibited the binding of [$^3$H]ifenprodil to the NR2B subunit of the rat NMDA receptor. Therefore, it was shown that Compound (I) had an antagonistic activity at the NMDA receptor NR2B subunit.
From the above, it is believed that Compound (I) or a pharmaceutically acceptable salt thereof is useful as a therapeutic agent for diseases in which NR2B subunit of NMDA receptor is participated (for example, a disease due to degeneration and injury of nerve cells such as epilepsy, stroke, anxiety, cerebral ischemia, muscle spasm, Alzheimer's disease, dementia, Huntington's disease, Parkinson's disease, cerebral apoplexy, cerebral infarction and the like, and pain such as chronic pain, neurogenic pain, headache, migraine and the like).

Test Example 2: Suppressive effect of the Compound to neurogenic pain in strangulated nerve-damaged rats

The test was carried out in accordance with a method by G. M. Pitcher, et al. [Pain, 1999, vol. 83, pages 37 to 46] which is an improvement of a method by T. Mosconi and K. Kruger [Pain, 1996, vol. 64, pages 37 to 57].
Using male rats of an SD strain (Sprague-Dawley rats), strangulated nerve-damaged rats were prepared by removing sciatic nerve under anesthetizing with pentobarbital, and covering with a PE-60 polyethylene tube (trade name: Intramedic; size: PE-60; manufactured by Becton Dickinson) having a length of 2 mm over the removed area. After 14 to 21 days from the preparation, the following test was carried out. Incidentally, a pain threshold (g) was calculated by the following method, and the rats (strangulated rat-damaged rats) showing less than 4 g of a 50% pain threshold were used for the test in which one group comprised six rats.
The strangulated nerve-damaged rats were placed in a cage made of stainless steel (750 mm width x 210 mm length x 170 mm height), and acclimated at least for 20 minutes, then subjected to measurements of pain threshold (g) before administration of the test compound (called 0 hour) and after each 1, 3 and 5 hour(s) from the administration.
The test compound was suspended in a 0.5% aqueous solution of methyl cellulose (0.5% MC) and orally administered to the strangulated nerve-damaged rat at the dose of 5 mL/kg (the test compound treated group). In the meanwhile, only a 0.5% MC aqueous solution was orally administered to the strangulated nerve-damaged rats at the dose of 5 mL/kg (Solvent treated group).
For the measurement of allodynia which is characteristically noted in neurogenic pain, a von Frey filament (trade name: touch test sensory evaluator; catalog number: model 58011) was used and the result is expressed as a pain threshold (g). The pain threshold (g) was calculated by the up-down method by W. J. Dixon [Annual Review of Pharmacology and Toxicology, 1980, vol. 20, pages 441-462]. The result is shown in Fig. 1 and Fig. 2.
The pain threshold of normal rats is about 12 g, and in the strangulated nerve-damaged rats, a decrease in the pain threshold (g) recognized to be allodynia was confirmed.
As the results of the above, the following fact was found.
In the Solvent treated group to which only the solvent was administered, a large decrease of the pain threshold (g) recognized to be allodynia was confirmed. On the contrary, in the test compound treated group to which the test compound (Compound 6 or Compound 10) was orally administered at the dose of 5 and 10 mg/kg, the pain threshold noted in the strangulated nerve-damaged rats showed greatly increased value as compared with that of the Solvent treated group. Thus, allodynia was significantly improved by administration of Compound 6 or Compound 10.
From the above, it was suggested that Compound (I) or a pharmaceutically acceptable salt thereof is useful for the treatment of neurogenic pain.
Compound (I) or a pharmaceutically acceptable salt thereof may be sufficiently useful as administered by itself. However, usually, Compound (I) or a pharmaceutically acceptable salt thereof is preferably provided as various types of pharmaceutical preparations. Such pharmaceutical preparations are used for animals and humans.
The pharmaceutical preparations according to the present invention may comprise Compound (I) or a pharmaceutically acceptable salt thereof alone as an active ingredient. Alternatively, the pharmaceutical preparations may comprise a mixture of Compound (I) or a pharmaceutically acceptable salt thereof with any effective ingredient used for another treatment. Furthermore, these pharmaceutical preparations are prepared by mixing the active ingredient(s) with one or more pharmaceutically acceptable carrier(s) and then employing any method well-known in the technical field of pharmaceutics.
As for administration routes, it is preferred to select the most effective route of administration. Examples of the administration routes include oral administration and parenteral administration such as intravenous administration.
As for the dosage form, tablets, injections and the like are included.
For example, the tablet suitable for oral administration can be prepared with, for example, excipients such as lactose; disintegrants such as starch; lubricants such as magnesium stearate; binders such as hydroxypropylcellulose; and the like.
For example, the injections suitable for parenteral administration are prepared, using, for example, saline solution, glucose solution, a mixture of saline solution and glucose solution, or the like.
The dose and the frequency of administration of Compound (I) or a pharmaceutically acceptable salt thereof may vary depending on the administration form, the age and body weight of a patient, nature and severity of the condition

to be treated, and the like. In oral administration, the dosage may be 0.01 mg/adult to 1 g/adult, preferably 0.05 to 50 mg/adult, once or a few times a day. In parenteral administration such as intravenous administration, the dosage may be 0.001 to 100 mg/adult, preferably 0.01 to 10 mg/adult, once or a few times a day. However, the dose and the frequency of administration may vary depending upon the above-mentioned various conditions.

The embodiment of the present invention will be illustrated by way of the following Examples and Reference Examples.

Example 1

(±)-threo-6-{1-Hydroxy-2-[4-hydroxy-4-(thiophenee-2-yl)piperidino]propyl}benzothiazol-2(3H)-one (Compound 1) and (±)-erythro-6-{1-hydroxy-2-[4-hydroxy-4-(thiophenee-2-yl)piperidino]propyl}benzothiazol-2(3H)-one (Compound 2)

Step 1:

6-(2-Bromopropionyl)benzothiazol-2(3H)-one (5.00 g, 17.5 mmol) prepared in Reference Example 1, 4-hydroxy-4-(thiophenee-2-yl)piperidine (3.20 g, 17.5 mmol) and triethylamine (2.45 mL, 17.5 mmol) were suspended in DMF (35 mL), and the mixrure was stirred overnight at room temperature. After the reaction solution was concentrated in vacuo, water was added to the resulting residue, and the mixture was extracted with ethyl acetate. The organic layer was washed with water and brine, dried, and concentrated in vacuo. The resulting residue was purified by silica gel column chromatography (eluant: a mixture of chloroform/methanol = 30/1) to give 6-{2-[4-hydroxy-4-(thiophene-2-yl)piperidino]-propionyl}benzothiazol-2(3H)-one (1.43 g) as a yellow oil.

Step 2:

6-{2-[4-Hydroxy-4-(thiophene-2-yl)piperidino]-propionyl}benzothiazol-2(3H)-one (494 mg, 1.27 mmol) prepared in the step 1 was suspended in ethanol (30 mL), and sodium borohydride (430 mg) was added thereto with stirring at room temperature, then the mixture was further stirred overnight at room temperature. The reaction solution was concentrated in vacuo, and water was added to the resulting residue, then the mixture was extracted with ethyl acetate. The organic layer was washed with water and brine, dried, and concentrated in vacuo. The resulting residue was purified by silica gel column chromatography (eluant: a mixture of chloroform/methanol = 10/1) to give Compound 1 (176 mg, 35.5%) and Compound 2 (210 mg, 42.4%).

Compound 1

$^1$H-NMR($CD_3OD$/400 MHz) δ (ppm): 1.09(3H, d, J = 6.8 Hz), 1.98-2.09 (2H, m), 2.12-2.26 (2H, m), 3.02-3.37 (5H, m), 5.13 (1H, d, J=3.4 Hz), 6.96 (1H, dd, J = 3.5, 5.1 Hz), 7.01(1H, dd, J = 1.2, 3.5 Hz), 7.05 (1H, d, J = 8.1 Hz), 7.18-7.21 (1H, m), 7.26-7.28 (1H, m), 7.28(1H, d, j = 1.2Hz).

Compound 2

$^1$H-NMR (DMSO-$d_6$/400 MHz) δ (ppm): 0.94 (3H, d, J = 6.8 Hz), 1.66-1.88 (4H, m), 2.43 -2.56 (1H, m), 2.58 -2.87 (4H, m), 4.69 (1H, d, J = 4.9Hz), 5.17 (1H, brs), 6.89 (1H, dd, J = 1.2, 3.5 Hz), 6.93 (1H, dd, J = 3.5, 5.0 Hz), 7.04 (1H, d, J = 8.2 Hz), 7.21 (1H, dd, J = 1.6, 8.2 Hz), 7.31 (1H, dd, J = 1.2, 5.0 Hz) 7.47 (1H, d, J = 1.6 Hz), 11.70 (1H, brs).

Example 2

(±)-threo-6-{1-Hydroxy-2-[4-hydroxy-4-(thiophene-2-yl)piperidino]butyl}benzothiazol-2(3H)-one (Compound 3) and (±)-erythro-6-{1-hydroxy-2-[4-hydroxy-4-(thiophene-2-yl)piperidino]butyl}benzothiazol-2(3H)-one (Compound 4)

In accordance with the similar manner as mentioned in Example 1, a mixture of crude products of Compound 3 and Compound 4 was obtained from 6-(2-bromobutyryl)benzothiazol-2(3H)-one prepared in Reference Example 2 and 4-hydroxy-4-(thiophene-2-yl)piperidine. The mixture was purified by silica gel column chromatography (eluant: a mixture of chloroform/methanol = 15/1) to give Compound 3 (5%) and Compound 4 (1%), respectively.

Compound 3

$^1$H-NMR (DMSO-$d_6$/400 MHz) δ (ppm): 0.67 (3H, d, J = 7.5 Hz), 1.01-1.14 (1H, m), 1.39-1.54 (1H, m), 1.74-2.03

(4H, m), 2.38-2.47 (1H,m), 2.56-2.73 (2H, m), 2.80-2.91 (1H, m), 3.03-3.14 (1H, m), 4.30 (1H, d, J = 8.8 Hz), 5.26 (1H, s), 6.94-7.00 (2H, m), 7.05 (1H, d, J = 8.1 Hz), 7.24 (1H, dd, J = 1.5, 8.1 Hz), 7.33 (1H, dd, J = 1.5, 4.9 Hz), 7.53 (1H, d, J = 1.5 Hz), 11.78 (1H, brs).

Compound 4

$^1$H-NMR (DMSO-$d_6$/400 MHz) δ (ppm): 0.80 (3H, d, J = 7.3 Hz), 1.31-1.45 (1H, m), 1.49-1.64 (1H, m), 1.65-1.93 (4H, m), 2.34-2.71 (3H, m), 2.77-2.95 (2H, m), 4.81 (1H, brs), 5.10 (1H, brs), 5.18 (1H, brs), 6:87-6.95 (2H, m), 7.04 (1H, d, J = 8.2 Hz), 7.20 (1H, dd, J = 1.2, 8.2 Hz), 7.32 (1H, dd, J = 1.2, 3.9 Hz), 7.48 (1H, brs), 11.77 (1H, brs).

Example 3

6-{1-Hydroxy-2-[4-hydroxy-4-(thiophene-2-yl)-piperidino]ethyl}benzothiazol-2(3H)-one (compound 5)

In accordance with the similar manner as mentioned in Example 1, a crude product of Compound 5 was obtained from 6-(chloroacetyl)benzothiazol-2(3H)-one prepared by the similar manner as mentioned in Reference Example 1 and 4-hydroxy-4-(thiophene-2-yl)piperidine. The crude product was purified by silica gel column chromatography (eluant: a mixture of chloroform/methanol = 10/1), and further crystallized from ethanol to give Compound 5 (9.44%).

Compound 5

$^1$H-NMR (DMSO=$d_6$/400 MHz) δ (ppm): 1.69-1.83 (2H, m), 1.85-2.01 (2H, m), 2.36-2.60 (4H, m), 2.61-2.76 (2H, m), 4.65-4.75 (1H, m), 5.23(1H, brs), 6.90-6.98 (2H, m), 7.05 (1H, d, J = 8.3 Hz), 7.25 (1H, dd, J = 1.7, 8.3 Hz), 7.30-7.35 (1H, m), 7.52 (1H, d, J = 1.5 Hz), 11.78 (1H, brs).

Example 4

(±)-threo-6-{1-Hydroxy-2-[4-hydroxy-4-(thiophene-2-yl)piperidino]propyl}-3,4-dihydroquinolin-2(1H)-one (Compound 6)

Step 1:

6-(2-Bromopropionyl)-3,4-dihydroquinolin-2(1H)-one (4.0 g, 14.2 mmol) prepared by the similar manner as mentioned in JP-T-6-504293 or JP-A-2-72173, 4-hydroxy-4-(thiophene-2-yl)piperidine (2.60 g, 14.2 mmol) and triethylamine (2.00 mL, 14.2 mmol) were suspended in DMF (40 mL), and the mixture was stirred overnight at room temperature. After the reaction solution was concentrated in vacuo, water was added to the resulting residue, and the mixture was extracted with ethyl acetate. The precipitated crystals were filtered, and washed with water to give 6-{2-[4-hydroxy-4-(thiophene-2-yl)piperidino]propionyl-3,4-dihydroquinolin-2(1H)-one (2.35 g). The organic layer was washed with water and brine, dried, and concentrated in vacuo. The resulting residue was purified by silica gel column chromatography (eluant: a mixture of chloroform/methanol = 30/1), and further crystallized from methanol to give 6-{2-[4-hydroxy-4-(thiophene-2-yl)-piperidino]propionyl}-3,4-dihydroquinolin-2(1H)-one (2.01 g).

Step 2:

6-{2-[4-Hydroxy-4-(thiophene-2-yl)piperidino]propionyl}-3,4-dihydroquinolin-2(1H)-one (1.01 g, 2.63 mmol) prepared in the step 1 was suspended in ethanol (26 mL), sodium borohydride (497 mg, 13.1 mmol) was added thereto with stirring at room temperature, and the mixture was further stirred overnight at room temperature. The reaction solution was concentrated in vacuo, and water was added to the resulting residue. The precipitated crystals were filtered, and washed with water to give Compound 6 (492 mg). The filtrate was extracted with ethyl acetate. The organic layer was washed with water and brine, dried, and concentrated in vacuo. The resulting residue was purified by silica gel column chromatography (eluant: a mixture of chloroform/methanol = 25/1) to give Compound 6 (172 mg) and Compound 7 (126 mg) as a by-product.

Compound 6

$^1$H-NMR (DMSO-$d_6$/400 MHz) δ (ppm): 0.70 (3H, d, J = 6.8 Hz), 1.72-2.18 (4H, m), 2.37-2.70 (6H, m), 2.80-2.97 (3H, m), 4.19 (1H, d, J = 9.3 Hz), 4.93 (1H, brs), 5.28 (1H, s), 6.79 (1H, d, J = 8.1 Hz), 6.96 (1H, dd, J = 3.5, 5.1 Hz), 6.99 (1H, dd, J = 1.3, 3.5 Hz), 7.08 (1H, dd, J = 1.7, 8.1 Hz), 7.14 (1H, brs), 7.33 (1H, dd, J = 1.3, 5.1 Hz), 10.01 (1H, s).

Example 5

(±)-erythro-6-{1-Hydroxy-2-[4-hydroxy-4-(thiophene-2-yl)piperidino]propyl}-3,4-dihydroquinolin-2(1H)-one (Compound 7)

6-{2-[4-hydroxy-4-(thiophene-2-yl)piperidino]propionyl}-3,4-dihydroquinolin-2(1H)-one (2.67 g, 6.91 mmol) prepared by the similar manner as mentioned in JP-T-6-504293 or JP-A-2-72173 was dissolved in methanol (250 mL). To the solution was added methanolic HCl (6.91 mmol) under ice-cooling, and the mixture was concentrating in vacuo. The resulting residue was dissolved in ethanol (200 mL), sodium borohydride (1.42 g, 37.5 mmol) was added thereto with stirring at room temperature, and the mixture was further stirred overnight at room temperature. After the reaction solution was concentrated in vacuo, water was added thereto, and the mixture was extracted with ethyl acetate. The organic layer was washed with water and brine, dried, and concentrated in vacuo. The resulting residue was purified by silica gel column chromatography (eluant: a mixture of chloroform/methanol = 25/1 to chloroform/methanol/triethylamine = 4/1/0.1) to give Compound 7 (1.12 g).

Compound 7

$^1$H-NMR ($CD_3OD$/400 MHz) δ (ppm):
1.02 (3H, d, J = 6.8 Hz), 1.86-1.98 (2H, m), 2.00-2.14 (2H, m), 2.51-2.62 (2H, m), 2.68-3.01 (7H, m), 4.89 (1H, d, J = 3.9 Hz), 6.82 (1H, d, J = 7.8 Hz), 6.90-7.00 (2H, m), 7.12-7.20 (2H, m), 7.23 (1H, dd, J = 5.0, 1.3 Hz).

Example 6

**[0116]** [0086] (±)-threo-6-{1-Hydroxy-2-[4-hydroxy-4-(thiophene-2-yl)piperidino]butyl}-3,4-dihydroquinolin-2 (1H)-one (Compound 8) and (±)-erythro-6-{1-hydroxy-2-[4-hydroxy-4-(thiophene-2-yl)piperidino]butyl}-3,4-dihydro-quinolin-2(1H)-one

(Compound 9)

In accordance with the similar manner as mentioned in Example 4, a mixture of crude products of Compound 8 and Compound 9 was obtained from 6-(2-bromobutyryl)-3,4-dihydroquinolin-2(1H)-one prepared by the similar manner as mentioned in JP-T-6-504293 or JP-A-51-118770 and 4-hydroxy-4-(thiophene-2-yl)piperidine. The mixture was purified by silica gel column chromatography (eluant: a mixture of chloroform/methanol = 15/1) to give Compound 8 (46.6%) and Compound 9 (24.1%) each.

Compound 8

$^1$H NMR ($CD_3OD$/400 MHz) δ (ppm): 0.71 (3H, d, J = 7.6 Hz), 1.12-1.17 (1H, m), 1.55-1.70 (1H, m), 1.94-2.25 (4H, m), 2.48-2.61 (3H, m), 2.67-2.83 (2H, m), 2.85-3.02 (3H, m), 3.21-3.32 (1H, m), 4.23 (1H, d, J = 9.3 Hz), 6.83 (1H, d, J = 8.1 Hz), 6.95 (1H, dd, J = 3.6, 5.0 Hz), 7.02 (1H, dd, J = 1.2, 3.6 Hz), 7.18 (1H, dd, J = 1.8, 8.1 Hz), 7.19-7.23 (1H, m), 7.25 (1H, dd, J = 1.2, 5.0 Hz).

Compound 9

$^1$H-NMR (DMSO-$d_6$/400 MHz) δ (ppm): 0.80 (3H, d, J = 7.3 Hz), 1.29-1.42 (1H, m), 1.48-1.63 (1H, m), 1.68-1.93 (4H, m), 2.34-2.47 (4H, m), 2.56-2.68 (1H, m), 2.77-2.98 (4H, m), 4.75 (1H, brs), 4.89 (1H, brs), 5.14 (1H, s), 6.77 (1H, d, J = 8.1 Hz), 6.90-6.95 (2H, m), 7.05 (1H, dd, J = 1.6, 8.1 Hz), 7.09 (1H, d, J = 1.6 Hz), 7.31 (1H, dd, J = 1.3, 4.8 Hz).

Example 7

6-{1-Hydroxy-2-[4-hydroxy-4-(thiophene-2-yl)piperidino]ethyl}-3,4-dihydroquinolin-2(1H)-one (Compound 10)

In accordance with the similar manner as mentioned in Example 4, a crude product of Compound 10 was obtained from 6-(chloroacetyl)-3,4-dihydroquinolin-2(1H)-one prepared by the similar manner as mentioned in JP-T-6-504293 or JP-A-2-72173 and 4-hydroxy-4-(thiophene-2-yl)piperidine. The crude product was purified by silica gel column chromatography (eluant: a mixture of chloroform/methanol = 10/1), and further crystallized from ethyl acetate to give Compound 10 (47.1%).
$^1$H-NMR (DMSO-$d_6$/500 MHz) δ (ppm): 1.76 (2H, brd, J = 12.7 Hz), 1.97 (2H, dd, J = 3.9, 12.7 Hz), 2.32-2.57 (4H, m),

2.43 (2H, t, J = 7.5 Hz), 2.62-2.72 (2H, m), 2.85 (2H, t, J = 7.5 Hz), 4.57-4.63 (1H, m), 4.79 (1H, d, J = 3.4 Hz), 5.23 (1H, s), 6.78 (1H, d, J = 8.1 Hz), 6.93-6.96 (2H, m), 7.09 (1H, dd, J = 1.8, 8.1 Hz), 7.14 (1H, brs), 7.33 (1H, dd, J = 2.4, 3.7 Hz), 9.99 (1H, s).

Example 8

6-{1-Hydroxy-3-[4-hydroxy-4-(thiophene-2-yl)piperidino]propyl}-3,4-dihydroquinolin-2(1H)-one

(Compound 11)

In accordance with the similar manner as mentioned in Example 4, a crude product of Compound 11 was obtained from 6-(3-chloropropionyl)-3,4-dihydroquinolin-2(1H)-one prepared by the similar manner as mentioned in JP-T-6-504293 or JP-A-51-118770 and 4-hydroxy-4-(thiophene-2-yl)piperidine. The crude product was crystallized from methanol to give Compound 11 (72.5%).

<u>Compound 11</u>

$^1$H-NMR (DMSO-$d_6$/500 MHz) δ (ppm): 1.64-1.84 (4H, m), 1.85-1.96 (2H, m), 2.27-2.49 (6H, m), 2.53-2.68 (2H, m), 2.81-2.89 (2H, m), 4.54 (1H, dd, J = 4.8, 7.8 Hz), 5.38 (1H, s), 5.42 (1H, brs), 6.78 (1H, d, J = 8.1 Hz), 6.92-6.98 (2H, m), 7.07 (1H, dd, J = 1.6, 8.1 Hz), 7.12 (1H, d, J = 1.6 Hz), 7.32 (1H, dd, J = 1.9, 4.3 Hz), 9.99 (1H, brs).

Example 9

(±)-threo-6-{1-Hydroxy-3-[4-hydroxy-4-(thiophene-2-yl)piperidino]-2-methylpropyl}-3,4-dihydroquinolin-2(1H)-one (Compound 12) and (±)-erythro-6-{1-hydroxy-3-[4-hydroxy-4-(thiophene-2-yl)piperidino]-2-methylpropyl}-3,4-dihydroquinolin-2(1H)-one (Compound 13)

Step 1:

A mixture of 6-propionyl-3,4-dihydroquinolin-2(1H)-one (609 mg, 3.00 mmol) prepared by the similar manner as mentioned in JP-T-6-504293 or JP-A-2-72173, 4-hydroxy-4-(thiophene-2-yl)piperidine (605 mg, 3.30 mol), paraformaldehyde (99 mg), 12 mol/L hydrochloric acid (60 μL) and ethanol (1.5 mL) was stirred at 70°C for 24 hours. The reaction mixture was concentrated in vacuo, and the resulting residue was purified by silica gel column chromatography (eluant: a mixture of chloroform/methanol = 20/1) to give 6-{3-[4-hydroxy-4-(thiophene-2-yl)piperidino]-2-methylpropionyl}-3,4-dihydroquinolin-2(1H)-one (630 mg, 52.8%) as an amorphous powder.

Step 2:

6-{3-[4-hydroxy-4-(thiophene-2-yl)piperidino]-2-methylpropionyl}-3,4-dihydroquinolin-2(1H)-one (630 mg, 1.58 mmol) prepared in the step 1 was suspended in ethanol (50 mL), sodium borohydride (300 mg, 7.93 mmol) was added thereto with stirring at room temperature, and the mixture was further stirred overnight at room temperature. The reaction solution was concentrated in vacuo, water was added to the resulting residue, and the mixture was extracted with ethyl acetate. The organic layer was washed with water and brine, dried, and concentrated in vacuo. The resulting residue was purified by silica gel column chromatography (eluant: a mixture of chloroform/methanol = 20/1) to give a diastereomer mixture as an oily product. The resulting mixture was crystallized from methanol, and the resulting crystals were filtered to give crude crystals of Compound 12 (126.5 mg; content of Compound 13: 35.9%). The filtrate was concentrated, and methanol was added to the resulting residue to crystallize, then Compound 13 (107 mg; content of Compound 12: 20.6%) was prepared as crude crystals. Further, each of the crude crystals was recrystallized from ethanol to give Compound 12 (18.7 mg) and Compound 13 (27.1 mg), respectively.

<u>Compound 12</u>

$^1$H-NMR (DMSO-$d_6$/400 MHz) δ (ppm): 0.57 (3H, d, J = 6.8 Hz), 1.74-2.03 (5H, m), 2.22-2.56 (6H, m), 2.56-2.67 (1H, m), 2.77-2.91 (3H, m), 4.30 (1H, d, J = 7.6 Hz), 5.30 (1H, s), 6.45 (1H, brs), 6.77 (1H, d, J = 8.1 Hz), 6.94-6.98 (2H, m), 7.05 (1H, dd, J = 1.7, 8.1 Hz), 7.10 (1H, d, J = 1.7 Hz), 7.34 (1H, dd, J = 1.5, 4.9 Hz), 9.99 (1H, brs).

Compound 13

$^1$H-NMR (DMSO-$d_6$/400 MHz) δ (ppm): 0.70 (3H, d, J = 6.1 Hz), 1.72-1.85 (2H, m), 1.85-2.02 (3H, m), 2.04-2.16 (1H, m), 2.25-2.49 (5H, m), 2.52-2.71 (2H, m), 2.80-2.92 (2H, m), 4.51-4.60 (1H, m), 5.18-5.27 (2H, m), 6.87 (1H, d, J = 8.1 Hz), 6.91-6.98 (2H, m), 7.04 (1H, dd, J = 1.5, 8.1 Hz), 7.06 (1H, d, J = 1.5 Hz), 7.32 (1H, dd, J = 1.7, 4.6 Hz), 9.97 (1H, brs).

Example 10

(±)-threo-5-{1-Hydroxy-3-[4-hydroxy-4-(thiophene-2-yl)piperidino]propyl}indol-2(3H)-one (Compound 14) and (±)-erythro-5-{1-Hydroxy-2-[4-hydroxy-4-(thiophene-2-yl)piperidino]propyl}indol-2(3H)-one (Compound 15)

Step 1:

6-(2-Bromopropionyl)indol-2(3H)-one (18.3 g, 68.1 mmol) prepared by the similar manner mentioned in JP-A-52-118465, 4-hydroxy-4-(thiophene-2-yl)-piperidine (12.9g, 70.4 mol) and triethylamine (10.4 mL, 74.3 mmol) were suspended in DMF (100 mL), and the mixture was stirred overnight at room temperature. The reaction solution was concentrated in vacuo, and the resulting residue was purified by silica gel column chromatography (eluant: a mixture of chloroform/methanol = 10/1) to give 6-{2-[4-hydroxy-4-(thiophene-2-yl)piperidino]propionyl}indol-2(3H)-one (17.3 g, 68.6%) as a yellow amorphous powder.

Step 2:

6-{2-[4-Hydroxy-4-(thiophene-2-yl)piperidino]propionyl}indol-2(3H)-one (1.01 g, 2.63 mmol) prepared in the step 1 was suspended in ethanol (26 mL), sodium borohydride (497 mg, 13.1 mmol) was added thereto with stirring at room temperature, and the mixture was further stirred overnight at room temperature. The reaction solution was concentrated in vacuo, and water was added to the resulting residue, then the precipitated crystals were filtered. The resulting crystals were washed with ethyl acetate to give Compound 14 (456 mg). The filtrate was extracted with ethyl acetate. The organic layer was washed with water and brine, dried, and concentrated in vacuo. The resulting residue was purified by silica gel column chromatography (eluant: a mixture of chloroform/methanol = 20/1 to 4/1) to give Compound 14 (106 mg; total yield of Compound 14: 562 mg, 55.3%) and Compound 15 (126 mg, 12.4%), respectively.

Compound 14

$^1$H-NMR (DMSO-$d_6$/400 MHz) δ (ppm) : 0.79 (3H, d, J = 6.8 Hz), 1.94-2.33 (4H, m), 2.51-2.80 (4H, m), 3.00-3.13 (1H, m), 3.26-3.34 (2H, m), 4.29 (1H, d, J = 9.5 Hz), 6.85 (1H, d, J = 8.1 Hz), 6.95 (1H, dd, J = 3.6, 5.1 Hz), 7.02 (1H, dd, J = 1.2, 3.6 Hz), 7.21 (1H, dd, J = 1.7, 8.1 Hz), 7.25 (1H, dd, J = 1.2, 5.1 Hz), 7.29 (1H, d, J = 1.7 Hz).

Compound 15

$^1$H-NMR (DMSO-$d_6$/400 MHz) δ (ppm): 1.03 (3H, d, J = 6.6 Hz), 1.84-1.97 (2H, m), 1.98-2.14 (2H, m), 2.68-3.00 (5H, m), 3.37-3.32 (2H, m), 4.90 (1H, d, J = 4.2 Hz), 6.84 (1H, d, J = 8.1 Hz), 6.93 (1H, dd, J = 3.6, 5.0 Hz), 6.96 (1H, dd, J = 1.3, 3.6 Hz), 7.16-7.22 (1H, m), 7.23 (1H, dd, J = 1.3, 5.0 Hz), 7.25 (1H, d, J = 1.7 Hz).

Example 11

5-{1-Hydroxy-2-[4-hydroxy-4-(thiophene-2-yl)piperidino]ethyl}indol-2(3H)-one (Compound 16)

In accordance with the similar manner as mentioned in Example 10, a crude product of Compound 11 was obtained from 6-(chloroacetyl)indol-2(3H)-one prepared by the similar manner as mentioned in JP-A-2-72173 and 4-hydroxy-4-(thiophene-2-yl)piperidine. The crude product was purified by silica gel column chromatography (eluant: a mixture of chloroform/methanol = 10/1), and recrystallized from ethyl acetate to give Compound 16 (44.3%).
$^1$H-NMR (DMSO-$d_6$/400 MHz) δ (ppm): 1.71-1.83 (2H, m), 1.86-2.01 (2H, m), 2.34-2.59 (4H, m), 2.63-2.74 (2H, m), 3.44 (2H, s), 4.59-4.67 (1H, m), 4.76-4.83 (1H, m), 5.23 (1H, s), 6.74 (1H, d, J = 7.8 Hz), 6.91-6.97 (2H, m), 7.13 (1H, dd, J = 1.2, 7.8 Hz), 7.19 (1H, d, J = 1.2 Hz), 7.31-7.33 (1H, m), 10.28 (1H, br).

Example 12

(±)-threo-7-{1-Hydroxy-2-[4-hydroxy-4-(thiophene-2-yl)piperidino]propyl}-2,3,4,5-tetrahydro-1H-1-benzoazepin-2-one (Compound 17) and (±)-erythro-7-{1-hydroxy-2-[4-hydroxy-4-(thiophene-2-yl)piperidino]propyl}-2,3,4,5-tetrahydro-1H-1-benzoazepin-2-one (Compound 18)

Step 1:

7-(2-Chloropropionyl)-2,3,4,5-tetrahydro-1H-1-benzo-azepin-2-one (755 mg, 3 mmol) prepared by the similar manner mentioned in JP-A-2-72173, 4-hydroxy-4-(thiophene-2-yl)-piperidine (549 mg, 3 mmol), sodium iodide (150 mg, 3 mmol) and triethylamine (0.84 mL, 6 mmol) were suspended in DMF (6 mL), and the mixture was stirred overnight at room temperature. The reaction solution was concentrated in vacuo, and water was added to the resulting residue, then the mixture was extracted with ethyl acetate. The organic layer was washed with water and brine, dried, and concentrated in vacuo. The resulting residue was purified by silica gel column chromatography (eluant: a mixture of chloroform/methanol = 10/1) to give 7-{2-[4-hydroxy-4-(thiophene-2-yl)piperidino]propionyl}-2,3,4,5-tetrahydro-1H-benzo[b]azepin-2-one (1.10 g, 89.0%) as a amorphous powder.

Step 2:

7-{2-[4-Hydroxy-4-(thiophene-2-yl)piperidino]propionyl}-2,3,4,5-tetrahydro-1H-1-benzoazepin-2-one (800 mg, 1.94 mmol) prepared in the step 1 was suspended in ethanol (20 mL), sodium borohydride (400 mg, 10.6 mmol) was added thereto with stirring at room temperature, and the mixture was further stirred overnight at room temperature. The precipitated white crystals (544 mg) were filtered. The filtrate was concentrated in vacuo, and water was added to the resulting residue, then the mixture was extracted with ethyl acetate. The organic layer was washed with water and brine, dried, and concentrated in vacuo. The resulting residue was combined with the above-prepared crystals, and purified by silica gel column chromatography (eluant: a mixture of chloroform/methanol = 5/1) to give Compound 17 (391 mg, 48.7%) and Compound 18 (95 mg, 11.8%), respectively.

Compound 17

$^1$H-NMR (DMSO-$d_6$/400 MHz) δ (ppm): 0.71 (3H, d, J = 6.7 Hz), 1.79-1.88 (2H, m), 1.91-2.20 (6H, m), 2.42-2.75 (6H, m), 2.85-2.94 (1H, m), 4.24 (1H, d, J = 8.2 Hz), 5.29 (1H, s), 6.90 (1H, d, J = 8.0 Hz), 6.96 (1H, dd, J = 3.5, 5.0 Hz), 6.99 (1H, dd, J = 1.2, 3.5 Hz), 7.17 (1H, dd, J = 1.8, 8.0 Hz), 7.22 (1H, d, J = 1.8 Hz), 7.34 (1H, dd, J = 1.2, 5.0 Hz), 8.31 (1H, s).

Compound 18

$^1$H-NMR (DMSO-$d_6$/400 MHz) δ (ppm): 0.93 (3H, d, J = 6.7 Hz), 1.65-1.90 (4H, m), 2.01-2.18 (4H, m), 2.49-2.92 (7H, m), 4.70 (1H, brs), 5.20 (1H, brs), 6.84-6.90 (2H, m), 6.92 (1H, dd, J = 3.5, 5.0 Hz), 7.10-7.19 (2H, m), 7.30 (1H, dd, J = 1.0, 5.0 Hz), 9.39 (1H, s).

Example 13

(±)-threo-5-{1-Hydroxy-2-[4-hydroxy-4-(thiophene-2-yl)piperidino]propyl}-2,3-dihydro-1H-benzimidazol-2-one

(Compound 19)

Step 1:

5-(2-Bromopropionyl)-2,3-dihydro-1H-benzimidazol-2-one (269 mg, 1 mmol) prepared by the similar manner mentioned in JP-T-6-504293 or JP-A-2-72173, 4-hydroxy-4-(thiophene-2-yl)piperidine (183 mg, 1 mmol) and triethylamine (0.14 mL, 1 mmol) were suspended in DMF (2 mL), and the mixture was stirred overnight at room temperature. The reaction solution was concentrated in vacuo, and methanol was added to the resulting residue, then the mixture was concentrated once again in vacuo. Saturated aqueous sodium hydrogen carbonate was added to the resulting residue, and the precipitated crystals were filtered. The resulting crystals were washed with water, and dried to give crude crystals of 5-{2-[4-hydroxy-4-(thiophene-2-yl)piperidino]propionyl}-2,3-dihydro-1H-benzimidazol-2-one (365 mg, 98.4%).

Step 2:

5-{2-[4-Hydroxy-4-(thiophene-2-yl)piperidino]propionyl}-2,3-dihydro-1H-benzimidazol-2-one (300 mg, 0.808 mmol) prepared in the step 1 was suspended in ethanol (10 mL), sodium borohydride (200 mg, 5.3 mmol) was added thereto with stirring at room temperature, and the mixture was further stirred overnight at room temperature. The precipitated crystals were filtered. After the filtrate was concentrated in vacuo, water was added to the resulting residue, and the precipitated crystals were filtered. The crystals obtained from each of the above processes were combined, and recrystallized from methanol to give Compound 19 (94.2 mg, 31.2%).

Compound 19

$^1$H-NMR (DMSO-$d_6$/500 MHz) δ (ppm): 0.68 (3H, d, J = 6.7 Hz), 1.78-1.90 (2H, m), 1.90-2.02 (1H, m), 2.02-2.14 (1H, m), 2.44-2.69 (4H, m), 2.85-2.96 (1H, m), 4.24 (1H, d, J = 9.3 Hz), 4.94 (1H, s), 5.29 (1H, brs), 6.83-6.94 (3H, m), 6.96 (1H, dd, J = 3.5, 5.0 Hz), 6.99 (1H, dd, J = 1.1, 3.5 Hz), 7.33 (1H, dd, J = 1.1, 5.0 Hz), 10.52 (2H, brs).

Example 14

(±)-threo-6-{1-Hydroxy-2-[4-hydroxy-4-(thiophene-2-yl)piperidino]propyl}benzoxazol-2(3H)-one (Compound 20) and (±)-erythro-6-{1-hydroxy-2-[4-hydroxy-4-(thiophene-2-yl)piperidino]propyl}benzoxazol-2(3H)-one (Compound 21)

Step 1:

6-(2-Chloropropionyl)benzoxazol-2(3H)-one (2.46 mg, 10.9 mmol) prepared in Reference Example 3, 4-hydroxy-4-(thiophene-2-yl)piperidine (2.00 g, 10.9 mmol) and triethylamine (1.67 mL, 11.9 mmol) were suspended in DMF (20 mL), and the mixture was stirred overnight at room temperature. Water was added to the reaction solution, and the mixture was extracted with ethyl acetate. The organic layer was washed with water and brine, dried, and concentrated in vacuo. The resulting residue was purified by silica gel column chromatography (eluant: a mixture of chloroform/methanol = 40/1) to give 6-{2-[4-hydroxy-4-(thiophene-2-yl)piperidino]propionyl}benzoxazol-2(3H)-one (906 mg, 22.3%).

Step 2:

6-{2-[4-Hydroxy-4-(thiophene-2-yl)piperidino]propionyl}benzoxazol-2(3H)-one (457 mg, 1.23 mol) prepared in the step 1 was suspended in ethanol (15 mL), sodium borohydride (233 mg, 5.3 mmol) was added thereto with stirring at room temperature, and the mixture was further stirred overnight at room temperature. After the reaction solution was concentrated in vacuo, water was added to the resulting residue, and the mixture was extracted with ethyl acetate. The organic layer was washed with water and brine, dried, and concentrated in vacuo. The resulting residue was purified by silica gel column chromatography (eluant: a mixture of chloroform/methanol = 10/1) to give Compound 20 (156 mg, 34%) and Compound 21 (29 mg, 6.3%), respectively.

Compound 20

$^1$H-NMR (DMSO-$d_6$/400 MHz) δ (ppm): 0.81 (3H, d, J = 6.6 Hz), 1.97-2.19 (3H, m), 2.20-2.32 (1H, m), 2.55-2.67 (1H, m), 2.67-2.83 (3H, m), 3.01-3.14 (1H, m), 4.37 (1H, d, J = 9.5 Hz), 6.95 (1H, dd, J = 3.4, 5.1 Hz), 7.00-7.03 (1H, m), 7.04-7.05 (1H, m), 7.18 (1H, dd, J = 1.3, 7.9 Hz), 7.25 (1H, dd, J = 1.2, 5.1 Hz), 7.27 (1H, d, J = 1.3 Hz).

Compound 21

$^1$H-NMR (DMSO-$d_6$/400 MHz) δ (ppm): 1.09 (3H, d, J = 6.8 Hz), 1.98-2.09 (2H, m), 2.12-2.26 (2H, m), 3.02-3.37 (5H, m), 5.13 (1H, d, J = 3.4 Hz), 6.96 (1H, dd, J = 3.5, 5.1 Hz), 7.01 (1H, dd, J = 1.2, 3.5 Hz), 7.05 (1H, d, J = 8.1 Hz), 7.18-7.21 (1H, m), 7.26-7.28 (1H, m), 7.28 (1H, d, J = 1.2 Hz).

Example 15

**[0143]** [0095] (±)-threo-7-{1-Hydroxy-2-[4-hydroxy-4-(thiophene-2-yl)piperidino]propyl}-2H-1,4-benzothiazin-3(4H)-one (Compound 22) and (±)-erythro-7-{1-hydroxy-2-[4-hydroxy-4-(thiophene-2-yl)piperidino]propyl}-2H-1,4-benzothiazin-3(4H)-one (Compound 23)

Step 1:

7-(2-Bromopropionyl)-2H-1,4-benzothiazin-3(4H)-one (2.0 g, 6.66 mmol) prepared in Reference Example 4, 4-hydroxy-4-(thiophene-2-yl)piperidine (1.22 g, 6.66 mmol) and triethylamine (0.93 mL, 6.66 mmol) were suspended in DMF (15 mL), and the mixture was stirred overnight at room temperature. To the reaction solution was added water, and the mixture was extracted with ethyl acetate. The organic layer was washed with water and brine, dried, and concentrated in vacuo. The resulting residue was purified by silica gel column chromatography (eluant: a mixture of chloroform/methanol = 50/1) to give 7-{2-[4-hydroxy-4-(thiophene-2-yl)piperidino]propionyl}-2H-1,4-benzothiazin-3 ($4_H$)-one (2.47 g, 92.1%).

Step 2:

(Method A) 7-{2-[4-Hydroxy-4-(thiophene-2-yl)piperidino]propionyl}-2H-1,4-benzothiazin-3(4H)-one (1.22 g, 3.03 mmol) prepared in the step 1 was suspended in ethanol (22 mL), sodium borohydride (585 mg, 15.1 mmol) was added thereto with stirring at room temperature, and the mixture was further stirred overnight at room temperature. The reaction solution was concentrated in vacuo, and water was added to the resulting residue, then the mixture was extracted with ethyl acetate. The organic layer was washed with water and brine, dried, and concentrated in vacuo. The resulting residue was purified by silica gel column chromatography (eluant: a mixture of chloroform/methanol = 10/1) to give Compound 22 (365 mg, 29.8%) and Compound 23 (38 mg, 3.1%), respectively.
(Method B) Compound 22 and Compound 23 were also prepared by the following method.

7-{2-[4-Hydroxy-4-(thiophene-2-yl)-piperidino]propionyl}-2H-1,4-benzothiazin-3(4H)-one (1.23 g, 3.06 mmol) prepared in the step 1 was dissolved in methanol (200 mL). To the solution was added methanolic HCl (3.06 mmol) under ice-cooling, and the mixture was concentrated in vacuo. The resulting residue was dissolved in ethanol (200 mL), and sodium borohydride (606 mg, 16.0 mmol) was added thereto with stirring at room temperature, then the mixture was further stirred overnight at room temperature. The reaction solution was concentrated in vacuo, and water was added to the resulting residue, then the mixture was extracted with ethyl acetate. The organic layer was washed with water and brine, dried, and concentrated in vacuo. The resulting residue was purified by silica gel column chromatography (eluant: a mixture of chloroform/methanol = 20/1) to give Compound 22 (232 mg, 18.8%) and Compound 23 (113 mg, 9.2%), respectively.

<u>Compound 22</u>

$^1$H-NMR (DMSO-$d_6$/400 MHz) δ (ppm): 0.73 (3H, d, J = 6.6 Hz), 1.78-2.15 (4H, m), 2.40-2.67 (4H, m), 2.84-2.96 (1H, m), 3.44 (2H, s), 4.23 (1H, d, J = 9.3 Hz), 5.03 (1H, brs), 6.90-7.03 (4H, m), 7.25 (1H, d, J = 7.8 Hz), 7.33 (1H, dd, J = 1.2, 4.9 Hz), 10.50 (1H, brs).

<u>Compound 23</u>

$^1$H-NMR ($CD_3OD$/400 MHz) δ (ppm): 1.53 (3H, d, J = 6.8 Hz), 2.37-2.64 (4H, m), 3.33-3.50 (5H, m), 3.89 (2H, s), 4.64 (1H, d, J = 3.9 Hz), 7.43 (1H, dd, J = 3.6, 5.0 Hz), 7.47 (1H, dd, J = 1.2, 3.6 Hz), 7.49-7.55 (2H, m), 7.71-7.78 (2H, m).

Example 16

(±)-threo-7-{1-Hydroxy-2-[4-hydroxy-4-(thiophene-2-yl)piperidino]propyl}-2H-1,4-benzoxazin-3(4H)-one (Compound 24) and (±)-erythro-7-{1-hydroxy-2-[4-hydroxy-4-(thiophene-2-yl)piperidino]propyl}-2H-1,4-benzoxazin-3(4H)-one

(Compound 25)

Step 1:

7-(2-Bromopropionyl)-2H-1,4-benzoxazin-3(4H)-one (2.0 g, 7.04 mmol) prepared in Reference Example 5, 4-hydroxy-4-(thiophene-2-yl)piperidine (1.3g, 7.0 mmol) and triethylamine (1.08 mL, 7.71 mmol) were suspended in DMF (15 mL), and the mixture was stirred overnight at room temperature. To the reaction solution was added water, and the mixture was extracted with ethyl acetate. The organic layer was washed with water and brine, dried, and concentrated in vacuo. The resulting residue was purified by silica gel column chromatography (eluant: a mixture of chloroform/methanol = 50/1) to give 7-{2-[4-hydroxy-4-(thiophene-2-yl)piperidino]propionyl}-2H-1,4-benzoxazin-3(4H)-one (2.83 g).

Step 2:

(Method A) 7-{2-[4-Hydroxy-4-(thiophene-2-yl)piperidino]propionyl}-2H-1,4-benzoxazin-3(4H)-one (1.40 g) prepared in the step 1 was suspended in ethanol (25 mL), sodium borohydride (700 mg, 18.5 mmol) was added thereto with stirring at room temperature, and the mixture was further stirred overnight at room temperature. After the reaction solution was concentrated in vacuo, methanol was added to the resulting residue, and the precipitated crystals were filtered. The resulting crystals were washed with water and methanol successively to give Compound 24 (324 mg). The filtrate was concentrated in vacuo, and water was added to the residue, then the mixture was extracted with ethyl acetate. The organic layer was washed with water and brine, dried, and concentrated in vacuo. The resulting residue was purified by silica gel column chromatography (eluant: a mixture of chloroform/methanol = 10/1) to give Compound 24 (491 mg) and Compound 25 (24 mg), respectively.

(Method B) 7-{2-[4-Hydroxy-4-(thiophene-2-yl)piperidino]propionyl}-2H-1,4-benzoxazin-3(4H)-one (1.30 g) prepared in the step 1 was dissolved in methanol (200 mL). To the solution was added methanolic HCl under ice-cooling, and the mixture was concentrated in vacuo. The resulting residue was dissolved in ethanol (200 mL), sodium borohydride (660 mg, 17.4 mmol) was added thereto with stirring at room temperature, and the mixture was further stirred overnight at room temperature. The reaction solution was concentrated in vacuo, and water was added to the resulting residue, then the mixture was extracted with ethyl acetate. The organic layer was washed with water and brine, dried, and concentrated in vacuo. The resulting residue was purified by silica gel column chromatography (eluant: a mixture of chloroform/methanol = 20/1) to give Compound 24 (154 mg, 11.9%) and Compound 25 (325 mg, 25.0%), respectively.

Compound 24

$^1$H-NMR (DMSO-$d_6$/400 MHz) δ (ppm): 0.72 (3H, d, J = 6.6 Hz), 1.84 (2H, brd, J = 12.2 Hz), 1.90-2.17 (2H, m), 2.37-2.69 (4H, m), 2.91 (1H, brt, J = 10.5 Hz), 4.22 (1H, brd, J = 9.0 Hz), 4.54 (2H, s), 4.98 (1H, brs), 5.29 (1H, brs), 6.83-6.93 (3H, m), 6.94-7.03 (2H, m), 7.34 (1H, dd, J = 1.3, 5.0 Hz), 10.65 (1H, brs).

Compound 25

$^1$H-NMR (DMSO-$d_6$/400 MHz) δ (ppm): 0.92 (3H, d, J = 6.6 Hz), 1.68-1.90 (4H, m), 2.43-2.84 (5H, m), 4.52 (2H, s), 4.64 (1H, brs), 5.02 (1H, brs), 5.16 (1H, brs), 6.79-6.88 (2H, m), 6.88-6.97 (3H, m), 7.31 (1H, dd, J = 1.5, 4.9 Hz), 10.61 (1H, brs).

Example 17

7-{1-Hydroxy-2-[4-hydroxy-4-(thiophene-2-yl)piperidino]ethyl}-2,3,4,5-tetrahydro-1H-1-benzoazepin-2-one (Compound 32: an enantiomer of Compound 33)

Step 1:

7-(Chloroacetyl)-2,3,4,5-tetrahydro-1H-1-benzoazepin-2-one (2.0 g, 8.41 mmol) prepared by the similar manner as mentioned in JP-A-2-72173 was suspended in ethyl acetate(80 mL), and formic acid (1.2 mL), triethylamine (1.9 mL, 13.6 mmol) and chloro[(1S,2S)-N-(p-toluenesulfonyl)-1,2-diphenylethylenediamine](pentamethylcyclopentadienyl) rhodium (CpRhCl[(S,S)-Tsdpen]: 107 mg, 0.168 mmol) were added thereto, then the mixture was stirred overnight at room temperature under an argon atmosphere. The reaction solution was passed through silica gel column chromatography (eluant: ethyl acetate), and the resulting solution was concentrated in vacuo. To the resulting residue was added ethyl acetate, and the precipitated crystals were filtered. The resulting crystals were washed with ethyl acetate, and recrystallized from ethanol to give one of the enantiomers of 7-(2-chloro-1-hydroxyethyl)-2,3,4,5-tetrahydro-1H-1-benzoazepin-2-one (1.11 g, 55.1%) as a white solid.
$^1$H-NMR (DMSO-$d_6$/400 MHz) δ (ppm): 2.03-2.20 (4H, m), 2.67 (2H, t, J = 6.7 Hz), 3.65 (1H, dd, J = 7.1, 11.0 Hz), 3.73 (1H, dd, J = 4.6, 11.0 Hz), 4.67-4.76 (1H, m), 5.72 (2H, d, J = 4.6 Hz), 6.92 (1H, d, J = 8.1 Hz), 7.23 (1H, dd, J = 2.0, 8.1 Hz), 7.27 (1H, d, J = 2.0 Hz), 9.47 (1H, s).

Step 2:

7-(2-Chloro-1-hydroxyethyl)-2,3,4,5-tetrahydro-1H-1-benzoazepin-2-one (1.07 g, 4.46 mmol) prepared in the step 1, imidazole (912 mg, 13.4 mmol) and tert-butyldimethylsilyl chloride (1.14 g, 7.56 mmol) were dissolved in anhydrous DMF (4 mL), and the mixture was stirred overnight at room temperature under an argon atmosphere. The reaction solution was concentrated in vacuo, and the resulting residue was purified by silica gel column chromatography (eluants: hexane to a mixture of hexane/ethyl acetate = 1/1) to give one of the enantiomers of 7-[1-(tert-butyldimethylsilyloxy)-2-chloroethyl]-2,3,4,5-tetrahydro-1H-1-benzoazepin-2-one (1.54 g, 97.6%) as yellow crystals. $^1$H-NMR ($CD_3OD$/400 MHz) δ (ppm): -0.07 (3H, s), 0.10 (3H, s), 0.89 (9H, s), 2.15-2.30 (4H, m), 2.78 (2H, t, J = 7.0 Hz), 3.56-3.63 (2H, m), 4.80 (1H, s), 4.87 (1H, dd, J = 5.1, 6.8 Hz), 6.99 (1H, d, J = 8.5 Hz), 7.24-7.30 (2H, m).

Step 3:

7-[1-(tert-Butyldimethylsilyloxy)-2-chloroethyl]-2,3,4,5-tetrahydro-1H-1-benzoazepin-2-one (1.24 g, 3.50 mmol) prepared in the step 2, 4-hydroxy-4-(thiophene-2-yl)-piperidine (1.61 g, 8.78 mmol), sodium iodide (786 mg, 5.25 mmol) and triethylamine (732 mg, 5.25 mmol) were dissolved in HMPA (8 mL), and the mixture was stirred at 100°C for two days. To the reaction solution was added water, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 30/1) to give one of the enantiomers of 7-{1-(tert-butyldimethylsilyloxy)-2-[4-hydroxy-4-(thiophene-2-yl)piperidino]ethyl}-2,3,4,5-tetrahydro-1H-1-benzoazepin-2-one (1.29 g, 73.7%) as a light brown amorphous powder.
$^1$H-NMR (DMSO-$d_6$/500 MHz) δ (ppm): -0.07 (3H, s), 0.07 (3H, s), 0.85 (9H, s), 1.77 (2H, brd, J = 13.5 Hz), 1.84-1.98 (2H, m), 2.01-2.16 (4H, m), 2.35 (1H, dd, J = 3.8, 13.1 Hz), 2.42-2.59 (3H, m), 2.61-2.72 (4H, m), 4.79 (1H, dd, J = 3.7, 8.0 Hz), 5.22 (1H, s), 6.90 (1H, d, J = 8.0 Hz), 6.92 (1H, dd, J = 1.3, 3.5 Hz), 6.94 (1H, dd, J = 3.5, 5.0 Hz), 7.16-7.22 (2H, m), 7.33 (1H, dd, J = 1.3, 5.0 Hz), 9.43 (1H, s).

Step 4:

7-{1-(tert-Butyldimethylsilyloxy)-2-[4-hydroxy-4-(thiophene-2-yl)piperidino]ethyl}-2,3,4,5-tetrahydro-1H-1-benzoazepin-2-one (917 mg, 1.88 mmol) prepared in the step 3 was suspended in anhydrous THF (13 mL), and TBAF (5 mL, 1 mmol/L THF solution) was added thereto at room temperature under an argon atmosphere, then the mixture was stirred for two days. After the reaction solution was concentrated in vacuo, water was added thereto, and the mixture was extracted with ethyl acetate. The resulting residue was washed with brine, dried over anhydrous magnesium sulfate, and concentrated in vacuo. The resulting residue was purified by silica gel column chromatography (eluant: a mixture of chloroform/methanol = 15/1 to 5/1), then ethyl acetate was added thereto and the precipitated crystals were filtered. The resulting crystals were washed with ethyl acetate, and recrystallized from ethanol to give Compound 32 (335 mg, 43.3%) as white crystals.

<u>Compound 32</u>

$^1$H-NMR (DMSO-$d_6$/500 MHz) δ (ppm): 1.76 (2H, brd, J = 13.3 Hz), 1.87-2.01 (2H, m), 2.03-2.18 (4H, m), 2.36-2.60 (4H, m), 2.61-2.75 (4H, m), 4.64-4.70 (1H, m), 4.88 (1H, d, J = 3.5 Hz), 5.23 (1H, s), 6.90 (1H, d, J = 8.1 Hz), 6.92-6.98 (2H, m), 7.18 (1H, dd, J = 1.8, 8.1 Hz), 7.22 (1H, d, J = 1.8 Hz), 7.30-7.36 (1H, m), 9.43 (1H, s).

Example 18

7-{1-Hydroxy-2-[4-hydroxy-4-(thiophene-2-yl)piperidino]ethyl}-2,3,4,5-tetrahydro-1H-1-benzoazepin-2-one (Compound 33: an enantiomer of Compound 32)

Step 1:

In accordance with the similar manner as mentioned in the step 1 of Example 17, one of the enantiomers of 7-(2-chloro-1-hydroxyethyl)-2,3,4,5-tetrahydro-1H-1-benzoazepin-2-one (1.12 g, 55.6%) was obtained as a white solid from 7-(chloroacetyl)-2,3,4,5-tetrahydro-1H-1-benzoazepin-2-one (2.0 g, 8.41 mmol) using chloro[(1R,2R)-N-(p-toluenesulfonyl)-1,2-diphenylethylenediamine] (pentamethylcyclopentadienyl) rhodium (CpRhCl[R,R]-Tsdpen) instead of CpRhCl[(S,S)-Tsdpen].
$^1$H-NMR (DMSO-$d_6$/400 MHz) δ (ppm): 2.03-2.20 (4H, m), 2.67 (2H, t, J = 6.7 Hz), 3.65 (1H, dd, J = 7.1, 11.0 Hz), 3.73 (1H, dd, J = 4.6, 11.0 Hz), 4.67-4.76 (1H, m), 5.72 (2H, d, J = 4.6 Hz), 6.92 (1H, d, J = 8.1 Hz), 7.23 (1H, dd, J = 2.0,

8.1 Hz), 7.27 (1H, d, J = 2.0 Hz), 9.47 (1H, s).

Step 2:

In accordance with the similar manner as mentioned in the step 2 of Example 17, one of the enantiomers of 7-[1-(tert-butyldimethylsilyloxy)-2-chloroethyl]-2,3,4,5-tetrahydro-1H-1-benzoazepin-2-one (1.56 g, 98.8%) was obtained as yellow crystals from 7-(2-chloro-1-hydroxyethyl)-2,3,4,5-tetrahydro-1H-1-benzoazepin-2-one (1.07 g, 4.46 mmol) prepared in the step 1.
$^1$H-NMR ($CD_3OD$/400 MHz) δ (ppm): -0.07 (3H, s), 0.10 (3H, s), 0.89 (9H, s), 2.15-2.30 (4H, m), 2.78 (2H, t, J = 7.0 Hz), 3.56-3.63 (2H, m), 4.80 (1H, s), 4.87 (1H, dd, J = 5.1, 6.8 Hz), 6.99 (1H, d, J = 8.5 Hz), 7.24-7.30 (2H, m).

Step 3:

In accordance with the similar manner as mentioned in the step 3 of Example 17, one of the enantiomers of 7- { [1-(tert- butyldimethylsilyloxy)- 2-[4- hydroxy- 4-(thiophene- 2- yl) piperidino] ethyl}- 2,3,4,5- tetrahydro- 1H- 1- benzoazepin-2-one (1.33 g, 76.0%) was obtained as a light brown amorphous powder from 7-[1-(tert-butyldimethylsilyloxy)-2-chloroethyl]-2,3,4,5-tetrahydro-1H-1-benzoazepin-2-one (1.24 g, 3.50 mmol) prepared in the step 2 and 4-hydroxy-4-(thiophene-2-yl)piperidine (1.61 g, 8.78 mmol).
$^1$H-NMR (DMSO-$d_6$/500 MHz) δ (ppm): -0.07 (3H, s), 0.07 (3H, s), 0.85 (9H, s), 1.77 (2H, brd, J = 13.5 Hz), 1.84-1.98 (2H, m), 2.01-2.16 (4H, m), 2.35 (1H, dd, J = 3.8, 13.1 Hz), 2.42-2.59 (3H, m), 2.61-2.72 (4H, m), 4.79 (1H, dd, J = 3.7, 8.0 Hz), 5.22 (1H, s), 6.90 (1H, d, J = 8.0 Hz), 6.92 (1H, dd, J = 1.3, 3.5 Hz), 6.94 (1H, dd, J = 3.5, 5.0 Hz), 7.16-7.22 (2H, m), 7.33 (1H, dd, J = 1.3, 5.0 Hz), 9.43 (1H, s).

Step 4:

7-{1-(tert-Butyldimethylsilyloxy)-2-[4-hydroxy-4-(thiophene-2-yl) piperidino] ethyl}-2,3,4,5-tetrahydro-1H-1-benzoazepin-2-one (1.00 g, 2.0 mmol) prepared in the step 3 was suspended in anhydrous THF (15 mL), and TBAF (5 mL, 1 mmol/L THF solution) was added thereto at room temperature under an argon atmosphere, then the mixture was stirred for two days. After the reaction solution was concentrated in vacuo, water was added thereto, and the mixture was extracted with ethyl acetate. The resulting residue was washed with brine, dried over anhydrous magnesium sulfate, and concentrated in vacuo. The resulting residue was purified by silica gel column chromatography (eluant: a mixture of chloroform/methanol = 30/1 to 5/1), then ethyl acetate was added thereto and the precipitated crystals were filtered. The resulting crystals were washed with ethyl acetate, and recrystallized from ethanol to give Compound 33 (129 mg, 16.7%) as white crystals.

<u>Compound 33</u>

$^1$H-NMR (DMSO-$d_6$/500 MHz) δ (ppm): 1.76 (2H, brd, J = 13.3 Hz), 1.87-2.01 (2H, m), 2.03-2.18 (4H, m), 2.36-2.60 (4H, m), 2.61-2.75 (4H, m), 4.64-4.70 (1H, m), 4.88 (1H, d, J = 3.5 Hz), 5.23 (1H, s), 6.90 (1H, d, J = 8.1 Hz), 6.92-6.98 (2H, m), 7.18 (1H, dd, J = 1.8, 8.1 Hz), 7.22 (1H, d, J = 1.8 Hz), 7.30-7.36 (1H, m), 9.43 (1H, s).
In accordance with the similar manner as mentioned in Example 17, Compound 26 and Compound 27 were each obtained as a crude product by selecting the conditions such as asymmetric transition metal complex.

Example 19

(-)-5-{1-Hydroxy-2-[4-hydroxy-4-(thiophene-2-yl)piperidino]ethyl}indol-2(3H)-one (Compound 26; an enantiomer of Compound 27)

The crude product of Compound 26 was further recrystallized from ethanol (40 mL) to give Compound 26 (377 mg, 63%) as white crystals.

<u>Compound 26</u>

$^1$H-NMR (DMSO-$d_6$/500 MHz) δ (ppm): 1.72-1.81 (2H, m), 1.86-2.00 (2H, m), 2.33-2.58 (4H, m), 2.63-2.73 (2H, m), 3.43 (2H, s), 4.60-4.67 (1H, m), 4.79 (1H, brs), 5.22 (1H, s), 6.74 (1H, d, J = 8.0 Hz), 6.92-6.97 (2H, m), 7.13 (1H, d, J = 8.0 Hz), 7.19 (1H, brs), 7.30-7.34 (1H, m), 10.10 (1H, s). Specific rotation $[\alpha]_D^{25}$ = -16.6° (c 0.210, dimethyl sulfoxide)

Example 20

(+)-5-{1-Hydroxy-2-[4-hydroxy-4-(thiophene-2-yl)piperidino]ethyl}indol-2(3H)-one (Compound 27; an enantiomer of Compound 26)

The crude product of Compound 27 was further recrystallized from ethanol (40 mL) to give Compound 27 (363 mg, 65%) as white crystals.

<u>Compound 27</u>

$^1$H-NMR (DMSO-$d_6$/500 MHz) δ (ppm): 1.72-1.81 (2H, m), 1.86-2.00 (2H, m), 2.33-2.58 (4H, m), 2.63-2.73 (2H, m), 3.43 (2H, s), 4.60-4.67 (1H, m), 4.79 (1H, brs), 5.22 (1H, s), 6.74 (1H, d, J = 8.0 Hz), 6.92-6.97 (2H, m), 7.13 (1H, d, J = 8.0 Hz), 7.19 (1H, brs), 7.30-7.34 (1H, m), 10.10 (1H, s). Specific rotation $[\alpha]_D^{25}$ = +14.9° (c 0.220, dimethyl sulfoxide)

Example 21

(-)-6-{1-Hydroxy-2-[4-hydroxy-4-(thiophene-2-yl)-piperidino]ethyl}-3,4-dihydroquinolin-2(1H)-one (Compound 28: an enantiomer of Compound 29)

Step 1:

6-(Chloroacetyl)-3,4-dihydroquinolin-2(1H)-one (25.0 g, 112 mmol) prepared by the similar manner as mentioned in JP-T-6-504293 or JP-A-2-72173 was suspended in ethyl acetate (1.25 L), and formic acid (15.3 mL), triethylamine (25.6 mL, 184 mmol) and CpRhCl[(S,S)-Tsdpen] (900 mg) were added thereto, then the mixture was stirred overnight at room temperature under an argon atmosphere. The reaction solution was passed through silica gel column chromatography (eluant: ethyl acetate), and the resulting solution was concentrated in vacuo. To the resulting residue was added ethyl acetate, and the precipitated crystals were filtered. The resulting crystals were washed with ethyl acetate to give (-)-6-(2-chloro-1-hydroxyethyl)-3,4-dihydroquinolin-2(1H)-one (24.2 g, 96%) as a white solid.
$^1$H-NMR (DMSO-$d_6$/500 MHz) δ (ppm): 2.39-2.47 (2H, m), 2.86 (2H, t, J = 7.5 Hz), 3.62 (1H, dd, J = 7.7, 10.9 Hz), 3.69 (1H, dd, J = 5.1, 10.9 Hz), 4.63-4.70 (1H, m), 5.65 (1H, d, J = 4.6 Hz), 6.80 (1H, d, J = 8.1 Hz), 7.11-7.17 (1H, m), 7.19 (1H, s), 10.0 (1H, s).
Specific rotation $[\alpha]_D^{25}$ = -34.4° (c 0.408, methanol)

Step 2:

In accordance with the similar manner as mentioned in the step 2 of Example 17, (-)-6-[1-(tert-butyldimethylsilyloxy)-2-chloroethyl]-3,4-dihydroquinolin-2(1H)-one (43.0 g, 89.6%) was obtained as white crystals from (-)-6-(2-chloro-1-hydroxyethyl)-3,4-dihydroquinolin-2(1H)-one (31.8 g, 141 mmol) prepared in the step 1.
Specific rotation $[\alpha]_D^{25}$ = -59.6° (c 0.465, chloroform)

Step 3:

(-)-6-[1-(tert-Butyldimethylsilyloxy)-2-chloroethyl]-3,4-dihydroquinolin-2(1H)-one (26.6 g, 78.3 mmol) prepared in the step 2, 4-hydroxy-4-(thiophene-2-yl)piperidine (35.9 g, 196 mmol), sodium iodide (17.6 g, 0.117 mol) and triethylamine (16.4 mL, 117 mmol) were dissolved in HMPA (157 mL), and the mixture was stirred at 100°C for two days. To the reaction solution was added saturated aqueous sodium hydrogen carbonate, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 40/1), and further triturated with diethyl ether to give (-)-6-{1-(tert-butyldimethylsilyloxy)-2-[4-hydroxy-4-(thiophene-2-yl)piperidino]ethyl}-3,4-dihydroquinolin-2(1H)-one (25.0 g, 65%) as a white solid.
$^1$H-NMR (DMSO-$d_6$/400 MHz) δ (ppm): -0.07 (3H, s), 0.07 (3H, s), 0.85 (9H, s), 1.77 (2H, brd, J = 12.7 Hz), 1.86-1.98 (2H, m), 2.26-2.58 (6H, m), 2.61-2.72 (2H, m), 2.80-2.89 (2H, m), 4.75 (1H, dd, J = 3.7, 8.1 Hz), 5.22 (1H, s), 6.78 (1H, d, J = 7.8 Hz), 6.90-6.97 (2H, m), 7.06-7.14 (2H, m), 7.33 (1H, dd, J = 1.3, 5.0 Hz), 10.0 (1H, s).
Specific rotation $[\alpha]_D^{25}$ = -24.5°(c 0.492, methanol)

Step 4:

Molecular sieves (0.5 g) were added to TBAF (20 mL, 1 mol/L THF solution) previously dried using 4Å Molecular Sieves (containing moisture indicator; Nakarai), and the mixture was stirred under an argon atmosphere, then (-)-6-{1-(tert-butyldimethylsilyloxy)-2-[4-hydroxy-4-(thiophene-2-yl)piperidino]ethyl}-3,4-dihydroquinolin-2(1H)-one (917 mg, 1.88 mmol) prepared in the step 3 was added thereto. The mixture was stirred at room temperature for 2 days. To the reaction mixture was added ethyl acetate, and the mixture was filterated. To the resulting filtrate was added saturated aqueous sodium hydrogen carbonate, and the mixture was extracted with ethyl acetate. The organic layer was washed with saturated aqueous sodium hydrogen carbonate, water and brine, dried over anhydrous magnesium sulfate, and concentrated in vacuo. The resulting residue was purified by silica gel column chromatography (eluant: a mixture of chloroform/methanol = 30/1), and further crystallized from methanol to give Compound 28 (527 mg, 75%) as a white solid.

<u>Compound 28</u>

$^1$H-NMR (DMSO-$d_6$/400 MHz) δ (ppm): 1.76 (2H, brd, J = 12.9 Hz), 1.85-2.02 (2H, m), 2.33-2.59 (6H, m), 2.63-2.74 (2H, m), 2.85 (2H, t, J = 7.1 Hz), 4.58-4.66 (1H, m), 4.79 (1H, d, J = 3.4 Hz), 5.23 (1H, s), 6.78 (1H, d, J = 8.1 Hz), 6.92-6.98 (2H, m), 7.06-7.17 (2H, m), 7.32 (1H, dd, J = 2.4, 3.7 Hz), 10.0 (1H, s).
Specific rotation $[\alpha]_D^{25}$ = -24.7° (c 0.535, methanol)

Example 22

(+)-6-{1-Hydroxy-2-[4-hydroxy-4-(thiophene-2-yl)piperidino]ethyl}-3,4-dihydroquinolin-2(1H)-one (Compound 29: an enantiomer of Compound 28)

Step 1:

6-(Chloroacetyl)-3,4-dihydroquinolin-2(1H)-one (22 g, 98.4 mmol) prepared by the similar manner as mentioned in JP-T-6-504293 or JP-A-2-72173 was suspended in ethyl acetate (1.0 L), and formic acid (13.5 mL, 357 mmol), triethylamine (22.4 mL, 160 mmol) and CpRhCl[(R,R)-Tsdpen] (628 mg) were added thereto, then the mixture was stirred for 14 hours at room temperature under an argon atmosphere. The reaction solution was passed through silica gel column chromatography (eluant: ethyl acetate), and the resulting solution was concentrated in vacuo. The resulting residue was triturated with ether (500 mL) and further crystallized from isopropyl alcohol (400 mL). The resulting crystals were further recrystallized from isopropyl alcohol (360 mL) to give (+)-6-(2-chloro-1-hydroxyethyl)-3,4-dihydroquinolin-2(1H)-one (15.3 g, 76%) as white crystals.
$^1$H-NMR (DMSO-$d_6$/400 MHz) δ (ppm): 2.56 (2H, t, J = 8.0 Hz), 2.95 (2H, t, J = 8.0 Hz), 3.58-3.71 (2H, m), 4.76 (1H, dd, J = 4.0, 7.6 Hz), 6.85 (1H, d, J = 8.0 Hz), 7.20 (1H, dd, J = 2.0, 8.0 Hz), 7.22 (1H, s).
Specific rotation $[\alpha]_D^{26}$ = +33.2° (c 0.350, methanol)

Step 2:

In accordance with the similar manner as mentioned the step 2 of Example 21, (+)-6-(2-Chloro-1-hydroxyethyl)-3,4-dihydroquinolin-2(1H)-one (15.2 g, 673 mmol) prepared in step 1 was dissolved in DMF (75 mL), and imidazole (13.8 g, 202 mmol) and tert-butyldimethylsilyl chloride (17.2 g, 0.114 mol) were added thereto under ice-cooling, then the mixture was stirred at room temperature for 12 hours. To the reaction solution was added water, and the mixture was extracted with ethyl acetate. The organic layer was washed with brine, dried over anhydrous sodium sulfate, and concentrated in vacuo. The residue was crystallized, and further triturated with hexane to give (+)-6-[1-(tert-butyldimethylsilyloxy)-2-chloroethyl]-3,4-dihydroquinolin-2(1H)-one.(15.9 g, 69%) as white crystals.
$^1$H-NMR (DMSO-$d_6$/400 MHz) δ (ppm): -0.06 (3H, s), 0.09 (3H, s), 0.88 (9H, s), 2.64 (2H, t, J = 8.0 Hz), 2.96 (2H, t, J = 8.0 Hz), 3.48-3.58 (2H, m), 4.75 (1H, dd, J = 4.8, 7.6 Hz), 6.77 (1H, d, J = 8.0 Hz), 7.13 (1H, s), 7.15 (1H, d, J = 8.0 Hz), 8.62 (1H, brs).
Specific rotation $[\alpha]_D^{22}$ = +54.9° (c 0.380, methanol)

Step 3:

(+)-6-[1-(tert-Butyldimethylsilyloxy)-2-chloroethyl]-3,4-dihydroquinolin-2(1H)-one (30 g, 88.3 mmol) prepared in the step 2 was dissolved in HMPA (177 mL), and triethylamine (18.4 mL, 0.132 mmol), sodium iodide (19.8 g, 0.132 mmol) and 4-hydroxy-4-(thiophene-2-yl)piperidine (40.5 g, 0.221 mmol) were added thereto, then the mixture was stirred at 100°C for 48 hours under an argon atmosphere. To the reaction solution was added saturated aqueous sodium

hydrogen carbonate, and the mixture was extracted with ethyl acetate. The organic layer was washed with water and brine, dried over anhydrous sodium sulfate, and concentrated in vacuo. The resulting residue was purified by silica gel column chromatography (eluant: a mixture of chloroform/methanol = 30/1 to 10/1), and further triturated with ether to give (+)-6-{1-(tert-butyldimethylsilyloxy)-2-[4-hydroxy-4-(thiophene-2-yl)piperidino]ethyl}-3,4-dihydroquinolin-2 (1H)-one (27.7 g, 64%) as white crystals. $^1$H-NMR (DMSO-$d_6$/400 MHz) δ (ppm): -0.08 (3H, s), 0.07 (3H, s), 0.88 (9H, s), 1.82 (1H, brs), 1.87-1.97 (2H, m), 2.07-2.21 (2H, m), 2.43 (1H, dd, J = 4.0, 12.4 Hz), 2.55-2.71 (5H, m), 2.77-2.85 (1H, m), 2.92 (2H, t, J = 8.0 Hz), 4.74 (1H, dd, J = 4.0, 8.0 Hz), 6.65 (1H, d, J = 8.0 Hz), 6.95-7.01 (2H, m), 7.11-7.16 (2H, m), 7.23 (1H, dd, J = 0.8, 4.4 Hz), 7.52 (1H, brs).
Specific rotation $[\alpha]_D^{25}$ = +21.6° (c 0.335, methanol)

Step 4:

(+)-6-{1-(tert-Butyldimethylsilyloxy)-2-[4-hydroxy-4-(thiophene-2-yl)piperidino]ethyl}-3,4-dihydroquinolin-2 (1H)-one (56.5 g, 0.116 mol) prepared in the step 3 was dissolved in anhydrous THF (580 mL), and TBAF (290 mL, 1 mol/L THF solution, dried over molecular sieve) was added thereto, then the mixture was stirred at room temperature for 48 hours under an argon atmosphere. To the reaction solution was added saturated aqueous sodium hydrogen carbonate, and the mixture was extracted with ethyl acetate. The organic layer was washed with brine and water, dried over anhydrous sodium sulfate, and concentrated in vacuo. The resulting residue was triturated with ethyl acetate, and then purified by silica gel column chromatography (eluant: a mixture of chloroform/methanol = 5/1) and followed by being crystallized from ethanol (600 mL) to give Compound 29 (29.2 g, 68%) as white crystals.

<u>Compound 29</u>

H-NMR (DMSO-$d_6$/400 MHz) δ (ppm): 1.77 (2H, d, J = 12.0 Hz), 1.87-2.01 (2H, m), 2.33-2.59 (6H, m), 2.63-2.73 (2H, m), 2.85 (2H, t, J = 8.0 Hz), 4.62 (1H, m), 4.78 (1H, d, J = 3.6 Hz), 5.23 (1H, s), 6.77 (1H, d, J = 8.0 Hz), 6.92-6.96 (2H, m), 7.09 (1H, dd, J = 2.0, 4.0 Hz), 7.14 (1H, s), 7.32 (1H, dd, J = 2.0, 4.0 Hz), 9.99 (1H, s).
Specific rotation $[\alpha]_D^{26}$ = +21.0° (c 0.320, methanol)

Example 23

(-)-threo-6-{1-Hydroxy-2-[4-hydroxy-4-(thiophene-2-yl)piperidino]propyl}-3,4-dihydroquinolin-2(1H)-one (Compound 30) and (+)-threo-6-{1-hydroxy-2-[4-hydroxy-4-(thiophene-2-yl)piperidino]propyl}-3,4-dihydroquinolin-2(1H)-one (Compound 31)

Step 1:

Compound 6 (2.0 g, 5.18 mmol) prepared in Example 4 and copper chloride (I) (512 mg, 5.18 mmol) were suspended in DMF (30 mL), and the mixture was stirred at room temperature. To the reaction solution was added (S)-(-)-1-phenylethyl isocyanate (0.8 mL, 5.70 mmol), and the mixture was stirred at 55°C overnight. The reaction solution was concentrated in vacuo, and saturated aqueous sodium hydrogen carbonate was added thereto, and the mixture was extracted with ethyl acetate. The organic layer was washed with saturated aqueous sodium hydrogen carbonate and brine, dried over anhydrous magnesium sulfate, and concentrated in vacuo. The resulting residue was purified by silica gel column chromatography (eluant: a mixture of chloroform/methanol = 20/1) to give (±)-threo-6-{1-((1S)-1-phenylethylaminocarbonyloxy)-2-[4-hydroxy-4-(thiophene-2-yl)piperidyl]propyl}-3,4-dihydroquinolin-2(1H)-one (2.48 g, 4.38 mmol, 84.5%) as a yellow amorphous powder.
$^1$H-NMR ($CD_3$0D/400 MHz) δ (ppm): 0.52-0.95 (3H, m), 2.98 (3H, d, J = 0.5 Hz), 1.62-2.21 (4H, m), 2.46-3.10 (9H, m), 4.65-4.80 (1H, m), 5.51 (1H, d, J = 9.0 Hz), 6.75-7.06 (3H, m), 7.08-7.39 (8H, m).

Step 2:

(±)-threo-6-{1-((1S)-1-Phenylethylaminocarbonyloxy)-2-[4-hydroxy-4-(thiophene-2-yl)piperidyl]propyl}-3,4-dihydroquinolin-2(1H)-one (700 mg, 1.24 mmol) prepared in the step 1 was subjected to a preparative purification using a column [Inertsil ODS-3 (20 x 250 mm, GL-Science)] for a preparative high-performance liquid chromatography (eluant: a mixture of methanol/0.1 mol/L aqueous ammonium acetate = 53/47, flow rate: 20 mL/minute, detection: 254 nm). Methanol was evaporated in vacuo from each of the prepared solutions, and the residue was made basic by addition of aqueous sodium hydrogen carbonate, then the mixture was extracted with ethyl acetate. Each of the organic layers was washed with brine, dried over anhydrous magnesium sulfate, and concentrated in vacuo. Each of the firstly eluted component in the preparation (residue A: 248 mg, 35.3%) and lately eluted component (residue B: 273 mg, 39.0%) was

obtained as a colorless amorphous powder.

Step 3:

The residue A (208 mg, 0.367 mmol) obtained in the step 2 was dissolved in methylene chloride, and triethylamine (255 μL, 1.83 mmol) and trichlorosilane (185 μL, 1.83 mmol) were added thereto, then the mixture was stirred overnight at room temperature under an argon atmosphere. The reaction solution was concentrated in vacuo, and saturated aqueous sodium hydrogen carbonate was added thereto, then the mixture was extracted with ethyl acetate. The organic layer was washed with water and brine, dried over anhydrous magnesium sulfate, and concentrated in vacuo. The resulting residue was passed through silica gel column chromatography (eluant: a mixture of chloroform/methanol = 30/1), and the resulting solution was concentrated in vacuo. To the resulting residue was added ethyl acetate, and the precipitated crystals were filtered. The resulting crystals were washed with ethyl acetate to give Compound 31 (82.1 mg, 57.9%) as white crystals.

Compound 31

Specific rotation $[\alpha]_D^{25}$ = +46.4° (c 0.700, methanol)

Step4:

In accordance with the similar manner as mentioned in the step 3, Compound 30 (86.9 mg, 54.2%) was obtained as white crystals from the residue B obtained in the step 2 (222 mg, 0.415 mmol).

Compound 30

Specific rotation $[\alpha]_D^{25}$ = -58.3° (c 0.530, methanol

Example 24

6-{2-[4-(5-Chlorothiophene-2-yl)-4-hydroxypiperidino]-1-hydroxyethyl}-3,4-dihydroquinolin-2(1H)-one (Compound 34)

Step 1:

6-(Chloroacetyl)-3,4-dihydroquinolin-2(1H)-one (500 mg, 2.24 mmol) prepared by the similar manner as mentioned in JP-T-6-504293 or JP-A-2-72173, 4-(5-chlorothiophene-2-yl)-4-hydroxypiperidine (487 mg, 2.24 mmol) prepared in Reference Example 6 and triethylamine (343 μL, 2.5 mmol) were suspended in DMF (6 mL), and the mixture was stirred overnight at room temperature. The reaction solution was concentrated in vacuo, and water was added thereto, then the mixture was extracted with ethyl acetate. The organic layer was washed with brine, dried over anhydrous magnesium sulfate, and concentrated in vacuo. To the resulting residue was added methanol, and the precipitated crystals were filtered. The resulting crystals were washed with methanol to give 6-{2-[4-(5-chlorothiophene-2-yl)-4-hydroxypiperidino]acetyl}-3,4-dihydroquinolin-2(1H)-one (330 mg, 36.4%) as light yellow crystals.
$^1$H-NMR (DMSO-$d_6$/400 MHz) δ (ppm): 1.68-1.77 (2H, m), 1.85-1.94 (2H, m), 2.47-2.55 (4H, m), 2.64-2.71 (2H, m), 3.30 (2H, t, J = 7.5 Hz), 3.75 (2H, s), 5.51 (1H, s), 6.82 (1H, d, J = 4.0 Hz), 6.90-6.95 (2H, m), 7.81-7.87 (2H, m), 10.39 (1H, s).

Step 2:

6-{2-[4-(5-Chlorothiophene-2-yl)-4-hydroxypiperidino]acetyl}-3,4-dihydroquinolin-2(1H)-one (300 mg, 0.741 mmol) prepared in the step 1 was suspended in ethanol (20 mL), and sodium borohydride (162 mg, 4.05 mmol) was added thereto with stirring at room temperature, then the mixture was stirred overnight at room temperature. The reaction solution was concentrated in vacuo, and water was added thereto, then the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 10/1 to 3/1) to give Compound 34 (229 mg, 75.9%) as white crystals.

Compound 34

$^1$H-NMR (DMSO-$d_6$/400 MHz) δ (ppm): 1.72 (2H, brd, J = 12.9 Hz), 1.83-1.98 (2H, m), 2.32-2.57 (6H, m), 2.63-2.76 (2H, m), 2.85 (2H, t, J = 7.6 Hz), 4.57-4.65 (1H, m), 4.79 (1H, d, J = 2.9 Hz), 5.45 (1H, s), 6.78 (1H, d, J = 8.4

Hz), 6.79 (1H, d, J = 3.9 Hz), 6.92 (1H, d, J = 3.9 Hz), 7.09 (1H, dd, J = 1.8, 8.4 Hz), 7.12-7.16 (1H, m), 9.99 (1H, s).

Example 25

6-{1-Hydroxy-2-[4-hydroxy-4-(3-methylthiophene-2-yl)piperidino]ethyl}-3,4-dihydroquinolin-2(1H)-One (Compound 35)

Step 1:

6-(Chloroacetyl)-3,4-dihydroquinolin-2(1H)-one (500 mg, 2.24 mmol) prepared by the similar manner as mentioned in JP-T-6-504293 or JP-A-2-72173, 4-hydroxy-4-(3-methylthiophene-2-yl)piperidine (441 mg, 2.24 mmol) prepared in Reference Example 8 and triethylamine (343 μL, 2.5 mmol) were suspended in DMF (6 mL), and the mixture was stirred overnight at room temperature. The reaction solution was concentrated in vacuo, and water was added thereto, then the mixture was extracted with ethyl acetate. The organic layer was washed with brine, dried over anhydrous magnesium sulfate, and concentrated in vacuo. To the resulting residue was added methanol, and the precipitated crystals were filtered. The resulting crystals were washed with methanol to give 6-{2-[4-hydroxy-4-(3-methylthiophene-2-yl)piperidino]acetyl}-3,4-dihydroquinolin-2(1H)-one (336 mg, 39.0%) as a brown oil.

Step 2:

6-{2-[4-Hydroxy-4-(3-methylthiophene-2-yl)piperidino]acetyl}-3,4-dihydroquinolin-2(1H)-one (498 mg, 1.30 mmol) prepared in the step 1 was suspended in ethanol (20 mL), and sodium borohydride (212 mg, 5.60 mmol) was added thereto with stirring at room temperature, then the mixture was stirred overnight at room temperature. The reaction solution was concentrated in vacuo, and water was added thereto, then the mixture was extracted with ethyl acetate. The organic layer was washed with brine, dried over anhydrous magnesium sulfate, and concentrated in vacuo. The resulting residue was purified by silica gel column chromatography (eluant: chloroform/methanol = 15/1 to 5/1) to give Compound 35 (223 mg, 44.4%) as light yellow crystals.

Compound 35

$^1$H-NMR (DMSO-$d_6$/400 MHz) δ (ppm): 1.75 (2H, brd, J = 13.7 Hz), 1.92-2.08 (2H, m), 2.30 (3H, s), 2.32-2.55 (6H, m), 2.65-2.76 (2H, m), 2.85 (2H, t, J = 7.1 Hz), 4.59-4.66 (1H, m), 4.80 (1H, d, J = 3.2 Hz), 5.14 (1H, s), 6.76-6.82 (2H, m), 7.10 (1H, dd, J = 1.7, 8.1 Hz), 7.13-7.17 (2H, m), 9.99 (1H, s).

Example 26

6-{1-Hydroxy-2-[4-hydroxy-4-(5-methylthiophene-2-yl)piperidino]ethyl}-3,4-dihydroquinolin-2(1H)-one (compound 36)

Step 1:

6-(Chloroacetyl)-3,4-dihydroquinolin-2(1H)-one (0.59 g, 2.64 mmol) prepared by the similar manner as mentioned in JP-T-6-504293 or JP-A-2-72173, 4-hydroxy-4-(5-methylthiophene-2-yl)piperidine (0.64 mg, 3.24 mmol) prepared in Reference Example 7 and triethylamine (452 μL, 3.22 mmol) were suspended in DMF (10 mL), and the mixture was stirred overnight at room temperature. The reaction solution was concentrated in vacuo, and water was added thereto, then the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 25/1) to give 6-{2-[4-hydroxy-4-(5-methylthiophene-2-yl)piperidino]acetyl}-3,4-dihydroquinolin-2(1H)-one (453 mg, 44.6%) as light yellow crystals.
$^1$H-NMR (DMSO-$d_6$/400 MHz) δ (ppm): 1.70-1.78 (2H, m), 1.84-1.93 (2H, m), 2.38 (3H, s), 2.47-2.57 (4H, m), 2.60-2.69 (2H, m), 2.94 (2H, t, J = 7.0 Hz), 3.74 (2H, s), 5.15 (1H, s), 6.60 (1H, d, J = 3.0 Hz), 6.71 (1H, d, J = 3.0 Hz), 6.93 (1H, d, J = 8.0 Hz), 7.81-7.87 (2H, m), 10.39 (1H, s).

Step 2:

6-{2-[4-Hydroxy-4-(5-methylthiophene-2-yl)piperidino]acetyl}-3,4-dihydroquinolin-2(1H)-one (190 mg, 0.494 mmol) prepared in the step 1 was suspended in ethanol (15 mL), and sodium borohydride (100 mg, 2.64 mmol) was added thereto with stirring at room temperature, then the mixture was stirred overnight at room temperature. The reaction solution was concentrated in vacuo, and water was added thereto, then the precipitated crystals were filtrated. The resulting crystals were washed with ethyl acetate to give Compound 36 (159 mg, 83%).

Compound 36

$^1$H-NMR (DMSO-$d_6$/400 MHz) δ (ppm): 1.72 (2H, brd, J = 12.7 Hz), 1.80-1.95 (2H, m), 2.28-2.52 (6H, m), 2.37 (3H, s), 2.58-2.70 (2H, m), 2.84 (2H, t, J = 7.6 Hz), 4.56-4.63 (1H, m), 4.76 (1H, d, J = 3.4 Hz), 5.09 (1H, s), 6.59 (1H, dd, J = 1.0, 3.4 Hz), 6.69 (1H, d, J = 3.4 Hz), 6.76 (1H, d, J = 8.1 Hz), 7.07 (1H, dd, J = 1.7, 8.1 Hz), 7.10-7.15 (1H, m), 9.97 (1H, s).

Example 27

6-{2-[4-(5-Chlorothiophene-2-yl)-4-hydroxypiperidino]ethyl}-3,4-dihydroquinolin-2(1H)-one (Compound 37)

6-(2-Chloroethyl)-3,4-dihydroquinolin-2(1H)-one (400 mg, 1.91 mmol) prepared in Reference Example 9, 4-(5-chlorothiophene-2-yl)-4-hydroxypiperidine (416 mg, 1.91 mmol) prepared in Reference Example 6 and potassium carbonate (290 mg, 2.1 mmol) were suspended in DMF (5 mL), and the mixture was stirred overnight at 120˚C. To the reaction solution was added saturated aqueous sodium hydrogen carbonate, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 30/1), and further crystallized from ethyl acetate to give Compound 37 (145 mg, 19.4%) as white crystals.

Compound 37

$^1$H-NMR (DMSO-$d_6$/400 MHz) δ (ppm): 1.74 (2H, brd, J = 12.4 Hz), 1.88 (2H, dt, J = 3.8, 12.4 Hz,), 2.33-2.56 (6H, m), 2.59-2.74 (4H, m), 2.85 (2H, t, J = 7.6 Hz), 5.46 (1H, s), 6.75 (1H, d), 6.80 (1H, d, J = 3.8 Hz), 6.92 (1H, d, J = 3.8 Hz), 6.97 (1H, dd, J = 1.8, 8.0 Hz), 7.00-7.03 (1H, m), 9.95 (1H, s).

Example 28

6-{2-[4-Hydroxy-4-(thiophene-2-yl)piperidino]ethyl}-3,4-dihydroquinolin-2(1H)-one (Compound 38)

4-Hydroxy-4-(thiophene-2-yl)piperidine (500 mg, 2.38 mmol), 6-(2-chloroethyl)-3,4-dihydroquinolin-2(1H)-one (437 mg, 2.38 mmol) prepared in Reference Example 9 and potassium carbonate (362 mg, 2.61 mmol) were suspended in DMF (6 mL), and the mixture was stirred at 120˚C for 10 hours. The reaction solution was concentrated, and water was added to the resulting residue, then the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 20/1), and further crystallized from ethyl acetate to give Compound 38 (488 mg, 57.5%) as white crystals.

Compound 38

$^1$H-NMR (DMSO-$d_6$/400 MHz) δ (ppm): 1.78 (2H, brd, J = 12.0 Hz), 1.85-1.98 (2H, m), 2.35-2.55 (6H, m), 2.59-2.74 (4H, m), 2.83 (2H, t, J = 7.6 Hz), 5.23 (1H, s), 6.75 (1H, d, J = 8.1 Hz), 6.92-7.06 (4H, m), 7.29-7.36 (1H, m), 9.95 (1H, s).

Example 29

6-{2-[4-(Thiophene-2-yl)piperidino]ethyl}-3,4-dihydroquinolin-2(1H)-one (Compound 39)

Compound 38 (300 mg, 0.842 mmol) prepared in Example 28 was suspended in trifluoroacetic acid (10 mL), triethylsilane (537 mL, 3.36 mmol) was added thereto under ice-cooling, and the mixture was stirred under ice-cooling for a while then stirred overnight at room temperature. The reaction solution was concentrated in vacuo, and saturated aqueous sodium hydrogen carbonate was added to the resulting residue, then the mixture was extacted with ethyl acetate. The organic layer was washed with brine, dried over anhydrous sodium sulfate, and concentrated in vacuo. The resulting residue was purified by silica gel column chromatography (eluant: a mixture of chloroform/methanol = 30/1) to give Compound 39 (239 mg, 83.3%).

Compound 39

$^1$H-NMR (DMSO-$d_6$/500 MHz) δ (ppm): 1.54-1.68 (2H, m), 1.92 (2H, brd, J = 12.4 Hz), 2.07 (2H, brt, J = 11.1

Hz), 2.43 (2H, t, J = 7.6 Hz), 2.45-2.56 (2H, m), 2.59-2.72 (2H, m), 2.73-2.86 (3H, m), 2.98 (2H, brd, J = 11.6 Hz), 6.75 (1H, d, J = 8.0 Hz), 6.88 (1H, d, J = 3.4 Hz), 6.94 (1H, dd, J = 3.4, 5.1 Hz), 6.97 (1H, dd, J = 1.8, 8.0 Hz), 7.01 (1H, s), 7.31 (1H, dd, J = 1.2, 5.1 Hz), 9.96 (1H, s).

Example 30

6-{2-[trans-3-Hydroxy-4-(thiophene-2-yl)piperidino]ethyl}-3,4-dihydroquinolin-2(1H)-one (Compound 40)

6-(2-Chloroethyl)-3,4-dihydroquinolin-2(1H)-one (206 mg, 0.982 mmol) prepared in Reference Example 9, trans-3-hydroxy-4-(4-thiophene-2-yl)piperidine (180 mg, 0.982 mmol) prepared in Reference Example 13, sodium iodide (150 mmol, 1.00 mol) and triethylamine (138 μL, 0.990 mmol) were suspended in HMPA (1.5 mL), and the mixture was stirred at 120˚C for 4.5 hours. To the reaction solution was added saturated aqueous sodium hydrogen carbonateand, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 30/1) to give Compound 40 (163 mg, 60.4%) as a light yellow powder.

Compound 40

$^1$H-NMR (DMSO-$d_6$/400 MHz) δ (ppm): 1.57-1.72 (1H, m), 1.79-2.04 (3H, m), 2.45-2.71 (5H, m), 2.82 (2H, t, J = 7.5 Hz), 2.83 (2H, dd, J = 6.7, 8.4 Hz), 2.92 (1H, brd, J = 11.2 Hz), 3.02-3.11 (1H, m), 3.32-3.35 (1H, m), 4.81 (1H, d, J = 6.3 Hz), 6.75 (1H, d, J = 8.1 Hz), 6.84-6.86 (1H, m), 6.94 (1H, dd, J = 3.4, 5.0 Hz), 6.98 (1H, dd, J = 2.0, 8.1 Hz), 7.01 (1H, s), 7.30 (1H, dd, J = 1.2, 5.0 Hz), 9.95 (1H, s).

Example 31

6-{2-[cis-3,4-Dihydroxy-4-(thiophene-2-yl)piperidino]ethyl}-3,4-dihydroquinolin-2(1H)-one (Compound 41)

Step 1:

6-(2-Chloroethyl)-3,4-dihydroquinolin-2(1H)-one (160 mg, 0.763 mmol) prepared in Reference Example 9, cis-3,4-isopropylidenedioxy-4-(thiophene-2-yl)piperidine (200 mg, 0.836 mmol) prepared in Reference Example 11, sodium iodide (150 mmol, 0.763 mmol) and triethylamine (106 μL, 0.763 mmol) were suspended in HMPA (1.5 mL), and the mixture was stirred at 120˚C for 8.5 hours. To the reaction solution was added saturated aqueous sodium hydrogen carbonate, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 40/1) to give 6-{2-[cis-3,4-isopropylidenedioxy-4-(thiophene-2-yl)piperidino]ethyl}-3,4-dihydroquinolin-2(1H)-one (283 mg, 0.686 mmol).
$^1$H-NMR ($CDCl_3$/500 MHz) δ (ppm): 1.34 (3H, s), 1.47 (3H, s), 2.07-2.22 (3H, m), 2.3.1-2.70 (11H, m), 4.21-4.26 (1H, m), 6.74 (1H, d, J = 8.1 Hz), 6.96-7.06 (3H, m), 7.16 (1H, dd, J = 1.2, 3.7 Hz), 7.49 (1H, dd, J = 1.2, 5.0 Hz), 9.94 (1H, s).

Step 2:

6-{2-[cis-Isopropylidenedioxy-4-thiophene-2-yl)piperidino]ethyl}-3,4-dihydroquinolin-2(1H)-one (250 mg, 0.606 mmol) prepared in the step 1 was suspended in THF (1 mL), and 1 mol/L aqueous hydrochloric acid (1 mL) was added thereto, then the mixture was stirred at 50˚C for three days. To the reaction solution was added saturated aqueous sodium hydrogen carbonate, and the mixture was extracted with ethyl acetate. The organic layer was washed with brine, dried over anhydrous magnesium sulfate, and concentrated in vacuo. To the resulting residues was added ethyl acetate, and the precipitated crystals were filtered to give Compound 41 (112 mg, 49.8%) as white crystals.

Compound 41

$^1$H-NMR (DMSO-$d_6$/400 MHz) δ (ppm): 1.77-1.92 (2H,m), 2.23 (1H, t, J = 9.9 Hz), 2.27-2.36 (1H, m), 2.39-2.46 (2H, m), 2.46-2.56 (2H, m), 2.59-2.70 (3H, m), 2.65 (1H, dd, J = 6.2, 9.9 Hz), 2.83 (2H, t, J = 7.5 Hz), 3.63-3.72 (1H, m), 4.60 (1H, d, J = 7.3 Hz), 4.91 (1H, s), 6.75 (1H, d, J = 8.1 Hz), 6.91-7.06 (4H, m), 7.31 (1H, dd, J = 1.2, 4.9 Hz), 9.95 (1H, s).

Example 32

6-{1-Hydroxy-2-[4-(thiophene-2-yl)piperidino]ethyl}-3,4-dihydroquinolin-2(1H)-one (Compound 42)

Step 1:

6-(Chloroacetyl)-3,4-dihydroquinolin-2(1H)-one (80.5 mg, 0.360 mmol) prepared by the similar manner as mentioned in JP-T-6-504293 or JP-A-2-72173, 4-(thiophene-2-yl)piperidine (60.3 mg, 0.360 mmol) prepared in Reference Example 12 and triethylamine (51 μL) were suspended in DMF (2 mL), and the mixture was stirred overnight at room temperature. To the reaction solution was added water, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 50/1) to give 6-{2-[4-(thiophene-2-yl)piperidino]acetyl}-3,4-dihydroquinolin-2(1H)-one (60.3 mg, 47.3%).

Step 2:

6-{2-[4-(Thiophene-2-yl)piperidino]acetyl}-3,4-dihydroquinolin-2(1H)-one (57.3 mg, 0.162 mmol) prepared in the step 1 was suspended in ethanol (10 mL), and sodium borohydride (20 mg, 0.529 mmol) was added thereto, then the mixture was stirred overnight at room temperature. To the reaction solution was added saturated aqueous sodium hydrogen carbonate, and the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 40/1) to give Compound 42(36.4 mg, 63.0%).

<u>Compound 42</u>

$^1$H-NMR (DMSO-$d_6$/500 MHz) δ (ppm): 1.55-1.70 (2H, m), 1.83-1.96 (2H, m), 2.06-2.24 (2H, m), 2.32-2.55 (4H, m), 2.72-2.90 (3H, m), 2.93-3.03 (2H, m), 4.59-4.63 (1H, m), 4.82 (1H, d, J = 3.0 Hz), 6.78 (1H, d, J = 8.1 Hz), 6.88 (1H, d, J = 3.4 Hz), 6.94 (1H, dd, J = 3.4, 5.1 Hz), 7.09 (1H, dd, J = 1.7, 8.1 Hz), 7.14 (1H, s), 7.31 (1H, dd, J = 1.2, 5.1 Hz), 9.99 (1H, s).

Example 33

6-{2-[4-(Thiophene-2-yl)-1,2,5,6-tetrahydro-pyridin-1-yl]ethyl}-3,4-dihydroquinolin-2(1H)-one (Compound 43)

Crude product of 4-(thiophene-2-yl)-1,2,5,6-tetrahydro-pyridine (640 g, 3.05 mmol) prepared in Reference Example 10, 6-(2-chloroethyl)-3,4-dihydroquinolin-2(1H)-one (554 mg, 3.35 mmol) prepared in Reference Example 9 and potassium carbonate (468 mg, 3.35 mmol) were suspended in DMF (6 mL), and the mixture was stirred overnight at 120˚C. To the reaction solution was added saturated aqueous sodium hydrogen carbonate, and the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 30/1), and further crystallized from ethyl acetate to give Compound 43 (192 mg, 18.6%) as white crystals.

<u>Compound 43</u>

$^1$H-NMR (DMSO-$d_6$/500 MHz) δ (ppm): 2.36-2.53 (4H, m), 2.54-2.76 (6H, m), 2.83 (2H, t, J = 7.5 Hz), 3.07-3.16 (2H, m), 6.04-6.10 (1H, m), 6.75 (1H, d, J = 8.1 Hz), 6.96 (4H, m), 7.36 (1H, dd, J = 1.1, 5.1 Hz), 9.95 (1H, s).

Example 34

6-{1-Hydroxy-2-[4-(thiophene-2-yl)-1,2,5,6-tetrahydropyridin-1-yl]ethyl}-3,4-dihydroquinolin-2(1H)-one

(Compound 44)

6-(chloroacetyl)-3,4-dihydroquinolin-2(1H)-one (541 mg, 2.42 mmol) prepared by the similar manner as mentioned in JP-T-6-504293 or JP-A-2-72173 and crude product of 4-(thiophene-2-yl)-1,2,5,6-tetrahydropyridine (400 mg, 2.42 mmol) prepared in Reference Example 10 were suspended in DMF (10 mL), and triethylamine (337 μL, 2.42 mmol) was added thereto, then the mixture was stirred overnight at room temperature. To the reaction solution was added

water, and the mixture was extracted with ethyl acetate. The organic layer was washed with water and brine, dried over anhydrous magnesium sulfate, and concentrated in vacuo to give 6-{2-[4-(thiophene-2-yl)-1,2,5,6-tetrahydropyridin-1-yl]-acetyl}-3,4-dihydroquinolin-2(1H)-one which was used for the next reaction without further-purification.

Step 2:

6-{2-[4-(Thiophene-2-yl)-1,2,5,6-tetrahydropyridin-1-yl]acetyl}-3,4-dihydroquinolin-2(1H)-one (200 mg, 0.564 mmol) prepared in the step 1 was suspended in ethanol, and sodium borohydride (112 mg, 2.96 mmol) was added thereto, then the mixture was stirred overnight at room temperature. The reaction solution was concentrated in vacuo, and ethyl acetate and water were added to the resulting residue, then the precipitated crystals were filtered. The crystals were washed with methanol to give Compound 44 (70.0 mg, 35.0%).

Compound 44

$^1$H-NMR (DMSO-$d_6$/500 MHz) δ (ppm): 2.37-2.63 (6H, m), 2.71 (2H, dt, J = 2.5, 5.6 Hz), 2.85 (2H, t, J = 7.6 Hz), 3.12-3.21 (2H, m), 4.62-4.70 (1H, m), 4.91 (1H, d, J = 4.1 Hz), 6.02-6.08 (1H, m), 6.78 (1H, d, J = 8.1 Hz), 7.00 (1H, dd, J = 3.6, 5.0 Hz), 7.04 (1H, dd, J = 1.0, 3.6 Hz), 7.11 (1H, dd, J = 1.8, 8.1 Hz), 7.13-7.17 (1H, m), 7.35 (1H, dd, J = 1.0, 5.0 Hz), 9.99 (1H, s).

Example 35

6-{(1S)-1-Hydroxy-2-[trans-3-hydroxy-4-(thiophene-2-yl)piperidino]ethyl}-3,4-dihydroquinolin-2(1H)-one (Compound 45)

Step 1:

In accordance with the similar manner as mentioned in the step 1 of Example 17, 6-[(1S)-2-chloro-1-hydroxyethyl]-3,4-dihydroquinolin-2(1H)-one was obtained as white crystals from 6-(2-chloroacetyl)-3,4-dihydroquinolin-2(1H)-one using CpRhCl[(R,R)-Tsdpen] instead of CpRhCl[(S,S)-Tsdpen] as an asymmetric transition metal complex. Then, in accordance with the similar manner as in the step 2 of Example 22, 6-[(1S)-1-(tert-butyldimethylsilyloxy)-2-chloroethyl]-3,4-dihydroquinolin-2(1H)-one was obtained as white crystals from the above-prepared 6-[(1S)-2-chloro-1-hydroxyethyl]-3,4-dihydroquinolin-2(1H)-one.

Step 2:

6-[(1S)-1-(tert-Butyldimethylsilyloxy)-2-chloroethyl]-3,4-dihydroquinolin-2(1H)-one (267 mg, 0.786 mmol) prepared in the step 1, trans-3-hydroxy-4-(thiophene-2-yl)piperidine (357 g, 1.96 mmol) prepared in Reference Example 13, sodium iodide (180 mg, 1.20 mmol) and triethylamine (164 ml, 1.18 mmol) were suspended in HMPA (2 mL), and the mixture was stirred overnight at 100˚C. To the reaction solution was added saturated aqueous sodium hydrogen carbonate, and the mixture was extracted with ethyl acetate. The organic layer was washed with water and brine, dried over anhydrous magnesium sulfate, and concentrated in vacuo. The resulting residue was purified by silica gel column chromatography (eluant: a mixture of chloroform/methanol = 50/1) to give 6-{(1S)-1-(tert-butyldimethylsilyloxy)-2-[trans-3-hydroxy-4-(thiophene-2-yl)piperidino]ethyl}-3,4-dihydroquinolin-2(1H)-one (a diastereo mixture; 337 mg, 45.3%) as a brown amorphous powder.

Step 3:

6-{(1S)-1-(tert-butyldimethylsilyloxy)-2-[trans-3-hydroxy-4-(thiophene-2-yl)piperidino]ethyl}-3,4-dihydroquinolin-2(1H)-one (311 mg, 0.639 mmol) prepared in the step 2 was suspended in anhydrous THF (4 mL), and TBAF (1.6 mL, 1 mol/L THF solution) was added thereto, then the mixture was stirred overnight at room temperature. The reaction solution was concentrated in vacuo, and water was added to the resulting residue, then the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 40/1) to give Compound 45 (a diastereo mixture; 80.7 mg, 33.9%) as a light yellow amorphous powder.

Compound 45

$^1$H-NMR (DMSO-$d_6$/400 MHz) δ (ppm): 1.59-1.77 (2H, m), 1.82-2.20 (6H, m), 2.31-2.64 (10H, m), 2.78-3.16

(8H, m), 3.34-3.49 (2H, m), 4.55-4.71 (2H, m), 4.74-4.96 (4H, m), 6.79 (2H, d, J = 8.1 Hz), 6.90 (2H, d, J = 3.5 Hz), 6.94 (2H, dd, J = 3.5, 5.1 Hz), 7.05-7.19 (4H, m), 7.30 (2H, dd, J = 1.2, 5.1 Hz), 10.0 (2H, s).

Example 36

6-{(1R)-1-Hydroxy-2-[trans-3-hydroxy-4-(thiophene-2-yl)piperidino]ethyl}-3,4-dihydroquinolin-2(1H)-one (Compound 46)

Step 1:

In accordance with the similar manner as mentioned in the steps 1 and 2 of Example 17, 6-[(1R)-1-(tertbutyldimethylsilyloxy)-2-chloroethyl]-3,4-dihydroquinolin-2(1H)-one was obtained as white crystals from 6-(chloroacetyl)-3,4-dihydroquinolin-2(1H)-one.

Step 2:

In accordance with the similar manner as mentioned in the step 1 of Example 35, 6-{(1R)-1-(tert-butyldimethylsilyloxy)-2-[trans-3-hydroxy-4-(thiophene-2-yl)piperidino]ethyl}-3,4-dihydroquinolin-2(1H)-one (a diastereo mixture; 320 mg, 83.6%) was obtained as a brown amorphous powder from 6-[(1R)-1-(tert-butyldimethylsilyloxy)-2-chloroethyl]-3,4-dihydroquinolin-2(1H)-one (519 mg, 1.53 mmol) prepared in the step 1 and trans-3-hydroxy-4-(thiophene-2-yl)piperidine (0.70 g, 3.82 mmol) prepared in Reference Example 13.
$^1$H-NMR ($CD_3OD$/400 MHz) □ (ppm): -0.13 (3H, s), 0.09 (3H, s), 0.88 (9H, s), 1.84-1.95 (2H, m), 2.02-2.16 (2H, m), 2.25 (2H, t, J = 7.7 Hz), 2.43-2.52 (1H, m), 2.59-2.72 (4H, m), 2.79-2.99 (3H, m), 4.85 (1H, dd, J = 4.2, 7.8 Hz), 6.81 (1H, d, J = 8.1 Hz), 6.94 (1H, dd, J = 3.5, 5.1 Hz), 6.97 (1H, dd, J = 1.3, 3.5 Hz), 7.12-7.18 (2H, m), 7.23 (1H, dd, J = 1.3, 5.1 Hz).

Step 3:

6-{(1R)-1-(tert-butyldimethylsilyloxy)-2-[trans-3-hydroxy-4-(thiophene-2-yl)piperidino]ethyl}-3,4-dihydroquinolin-2(1H)-one (306 mg, 0.629 mmol) prepared in the step 2 was suspended in anhydrous THF (4.2 mL), and TBAF (1.6 mL, 1 mmol/L THF solution) was added thereto, then the mixture was stirred overnight at room temperature. The reaction solution was concentrated in vacuo, and water was added to the resulting residue, then the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 30/1) to give Compound 46 (a diastereo mixture; 128 mg, 54.6%) as a while amorphous powder.

<u>Compound 46</u>

$^1$H-NMR (DMSO-$d_6$/400 MHz) δ (ppm): 1.59-1.77 (2H, m), 1.82-2.20 (6H, m), 2.31-2.64 (10H, m), 2.78-3.16 (8H, m), 3.34-3.49 (2H, m), 4.55-4.71 (2H, m), 4.74-4.96 (4H, m), 6.79 (2H, d, J = 8.1 Hz), 6.90 (2H, d, J = 3.5 Hz), 6.94 (2H, dd, J = 3.5, 5.1 Hz), 7.05-7.19 (4H, m), 7.30 (2H, dd, J = 1.2, 5.1 Hz), 10.0 (2H, s).

Example 37

6-{(1S)-1-Hydroxy-2-[cis-3,4-dihydroxy-4-(thiophene-2-yl)piperidino]ethyl}-3,4-dihydroquinolin-2(1H)-one (Compound 47) and 6-{(1S)-1-hydroxy-2-[trans-3,4-dihydroxy-4-(thiophene-2-yl)piperidino]ethyl}-3,4-dihydroquinolin-2(1H)-one (Compound 48)

Step 1:

In accordance with the similar manner as mentioned in the step 2 of Example 35, 6-{(1S)-1-(tert-butyldimethylsilyloxy)-2-[cis-3,4-isopropylidenedioxy-4-(thiophene-2-yl)piperidino]ethyl}-3,4-dihydroquinolin-2(1H)-one was obtained as a brown amorphous powder from 6-[(1S)-1-(tert-butyldimethylsilyloxy)-2-chloroethyl]-3,4-dihydroquinolin-2 (1H)-one (220 mg, 0.647 mmol) prepared in the step 1 of Example 35 and cis-3,4-isopropylidenedioxy-4-(thiophene-2-yl)piperidine (380 mg, 1.59 mmol) prepared in Reference Example 11.

Step 2:

In accordance with the similar manner as mentioned in the step 3 of Example 36, a crude product of the mixture of Compound 47 and Compound 48 was obtained from 6-[(1S)-1-(tert-butyldimethylsilyloxy)-2-[cis-3,4-isopropylidenedioxy-4-(thiophene-2-yl)piperidino]ethyl]-3,4-dihydroquinolin-2(1H)-one prepared in the step 1. To the resulting crude product was added ethyl acetate, and the precipitated crystals were filtered. The resulting crystals were recrystallized from ethanol to give each of Compound 47 (a diastereo mixture; 67.4 mg, 26.7%, recovering yield: 61.4%) as light pink crystals and Compound 48 (a diastereo mixture; 20.0 mg, 9.8%) as a brown amorphous powder.

Compound 47

$^1$H-NMR (DMSO-$d_6$/400 MHz) δ (ppm): 1.75-1.96 (4H, m), 2.22-2.56 (12H, m), 2.58-2.70 (2H, m), 2.74-2.84 (2H, m), 2.86 (4H, t, J = 7.5Hz), 3.64-3.77 (2H, m), 4.56-4.67 (2H, m), 4.59 (2H, d, J = 7.3 Hz), 4.84 (2H, t, J = 3.3 Hz), 4.89 (2H, s), 6.78 (2H, s), 6.86-7.00 (4H, m), 7.10 (2H, brd, J = 8.1 Hz), 7.14 (2H, brs), 7.31 (2H, dd, J = 1.3, 5.0 Hz), 9.99 (2H, s).

Compound 48

$^1$H-NMR (DMSO-$d_6$/400 MHz) δ (ppm): 1.60-1.71 (2H, m), 2.24-2.47 (10H, m), 2.53-2.92 (12H, m), 3.34-3.43 (2H, m), 4.48-4.67 (2H, m), 4.80-4.96 (2H, m), 5.37-5.42 (2H, m), 6.79 (2H, d, J = 8.1 Hz), 6.91 (4H, m), 7.06-7.16 (4H, m), 7.30-7.35 (2H, m), 9.99 (2H, s).

Example 38

6-{(1R)-1-Hydroxy-2-[cis-3,4-dihydroxy-4-(thiophene-2-yl)piperidino]ethyl}-3,4-dihydroquinolin-2(1H)-one (Compound 49) and 6-{(1R)-1-hydroxy-2-[trans-3,4-dihydroxy-4-(thiophene-2-yl)piperidino]ethyl}-3,4-dihydroquinolin-2(1H)-one (Compound 50)

Step 1:

In accordance with the similar manner as mentioned in the step 2 of Example 35, 6-{(1S)-1-(tert-butyldimethylsilyloxy)-2-[cis-3,4-isopropylidenedioxy-4-(thiophene-2-yl)piperidino]ethyl}-3,4-dihydroquinolin-2(1H)-one was obtained as a brown amorphous powder from 6-[(1R)-1-(tert-butyldimethylsilyloxy)-2-chloroethyl]-3,4-dihydroquinolin-2 (1H)-one (340 mg, 1.00 mmol) prepared in the step 1 of Example 36 and cis-3,4-isopropylidenedioxy-4-(thiophene-2-yl)piperidine (598 mg, 2.50 mmol) prepared in

Reference Example 11.

Step 2:

In accordance with the similar manner as mentioned in the step 2 of Example 35, a crude product of the mixture of Compound 49 and Compound 50 was obtained from 6-{(1R)-1-(tert-butyldimethylsilyloxy)-2-[cis-3,4-isopropylidenedioxy-4-(thiophene-2-yl)piperidino]ethyl}-3,4-dihydroquinolin-2(1H)-one prepared in the step 1. To the resulting crude product was added ethyl acetate, and the precipitated crystals were filtered. The resulting crystals were washed with ethyl acetate to give each of Compound 49 (a diastereo mixture; 115.1 mg, 27.9%) as reddish brown crystals and Compound 50 (a diastereo mixture; 74.2 mg, 18.0%) as brown crystals.

Compound 49

$^1$H-NMR (DMSO-$d_6$/400 MHz) δ (ppm): 1.75-1.96 (4H, m), 2.22-2.56 (12H, m), 2.58-2.70 (2H, m), 2.74-2.84 (2H, m), 2.86 (4H, t, J = 7.5 Hz), 3.64-3.77 (2H, m), 4.56-4.67 (2H, m), 4.59 (2H, d, J = 7.3 Hz), 4.84 (2H, t, J = 3.3 Hz), 4.89 (2H, s), 6.78 (2H, s), 6.86-7.00 (4H, m), 7.10 (2H, brd, J = 8.1 Hz), 7.14 (2H, brs), 7.31 (2H, dd, J = 1.3, 5.0 Hz), 9.99 (2H, s).

Compound 50

$^1$H-NMR (DMSO-$d_6$/400 MHz) δ (ppm): 1.60-1.71 (2H, m), 2.24-2.47 (10H, m), 2.53-2.92 (12H, m), 3.34-3.43 (2H, m), 4.48-4.67 (2H, m), 4.80-4.96 (2H, m), 5.37-5.42 (2H, m), 6.79 (2H, d, J = 8.1 Hz), 6.91 (4H, m), 7.06-7.16 (4H,

m), 7.30-7.35 (2H, m), 9.99 (2H, s).

[Reference Example 1]

6-(2-Bromopropionyl)benzothiazol-2(3H)-one

Aluminum chloride (7.20 g, 54.0 mmol) and 2-benzothiazolone (3.0 g, 19.8 mmol) were suspended in methylene chloride (50 mL), and α-bromopropionyl bromide (4.20 mL, 40.0 mmol) was dropped thereto with stirring under ice-cooling. The reaction mixture was stirred for 7 hours under heating to reflux, and then the reaction solution was poured into ice-water. The precipitated white crystals were filtered to give 6-(2-bromopropionyl)benzothiazol-2(3H)-one (1.15 g, 20.3%).
$^1$H-NMR (DMSO-$d_6$/400 MHz) δ (ppm): 1.78 (3H, d, J = 6.6 Hz), 5.78 (1H, q, J = 6.6 Hz), 7.24 (1H, d, J = 8.5 Hz), 7.97 (1H, dd, J = 1.8, 8.5 Hz), 8.33 (1H, d, J = 1.8 Hz), 12.32 (1H, brs).

[Reference Example 2]

6-(2-Bromobutyryl)benzothiazol-2(3H)-one

Aluminum chloride (7.00 g, 49.5 mmol) and 2-benzothiazolone (2.5 g, 16.5 mmol) were suspended in methylene chloride (65 mL), and α-bromobutyryl bromide (4.00 mL, 33.1 mmol) was dropped thereto with stirring under ice-cooling. The reaction mixture was stirred for 37 hours under heating to reflux, and then the reaction solution was poured into ice-water. The precipitated white crystals were filtered to give 6-(2-bromobutyryl)benzothiazol-2(3H)-one (3.13 g, 63.0%).
$^1$H-NMR (DMSO-$d_6$/500 MHz) δ (ppm): 1.01 (3H, t, J = 7.3 Hz),1.93-2.19 (2H, m), 5.61 (1H, d, J = 7.9 Hz), 7.24 (1H, d, J = 8.5 Hz), 7.99 (1H, dd, J = 1.8, 8.5 Hz), 8.34 (1H, d, J = 1.8Hz), 12.34 (1H, brs).

[Reference Example 3]

6-(2-Chloropropionyl)benzoxazol-2(3H)-one

2-Benzoxazolone (15.0 g, 111 mmol), α-chloropropionic acid (17.0 mL), 185 mmol) and polyphosphoric acid (50 g) were stirred at 135°C for 7.5 hours. The reaction solution was poured into ice-water, and the mixture was neutralized with saturated aqueous sodium hydrogen carbonate, then the mixture was extracted with ethyl acetate. The organic layer was washed with water and brine, dried, and concentrated in vacuo. The resulting residue was purified by silica gel column chromatography (eluant: a mixture of ethyl acetate/hexane = 2/5) to give 6-(2-chloropropionyl)benzoxazol-2(3H)-one (10.6 g, 42.8%).
$^1$H-NMR (DMSO-$d_6$/400 MHz) δ (ppm): 1.61 (3H, d, J = 6.6 Hz), 5.77 (1H, q, J = 6.6 Hz), 7.24 (1H, d, J = 8.8 Hz), 7.90-7.93 (2H, m), 12.12 (1H, brs).

[Reference Example 4]

7-(2-Bromopropionyl)-2H-1,4-benzothiazin-3(4H)-one

Aluminum chloride (7.30 g, 54.7 mmol) and 2H-1,4-benzothiazin-3(4H)-one (3.0 g, 18.2 mmol) were suspended in methylene chloride (50 mL). α-Bromopropionyl bromide (3.80 ml, 36.3 mmol) was dropped thereto at room temperature. The reaction mixture was stirred for 4 hours under heating to reflux, and the reaction solution was poured into ice-water. The precipitated yellow crystals were filtered to give 7-(2-bromopropionyl)-2H-1,4-benzo-thiazin-3(4H)-one (4.10 g, 75.3%).
$^1$H-NMR (DMSO-$d_6$/400 MHz) δ (ppm): 1.78 (3H, d, J = 6.5 Hz), 3.56 (2H, s), 5.68 (1H, q, J = 6.5 Hz), 7.50 (1H, d, J = 8.1 Hz), 7.59 (1H, d, J = 1.9 Hz), 7.66 (1H, dd, J = 1.9, 8.1 Hz), 10.84 (1H, brs).

[Reference Example 5]

7-(2-Bromopropionyl)-2H-1,4-benzoxazin-3(4H)-one

Aluminum chloride (7.00 g, 52.5 mmol) and 2H-1,4-benzoxazin-3(4H)-one (3.0 g, 20.1 mmol) were suspended in methylene chloride (50 mL), and α-bromopropionyl bromide (4.21 ml, 40.2 mmol) was dropped thereto at room temperature. The reaction mixture was stirred for 4 hours under heating to reflux, and the reaction solution was poured into ice-water, then the mixture was concentrated in vacuo. To the resulting residue was added water, and the precipitated

white crystals were filtered to give 7-(2-bromopropionyl)-2H-1,4-benzoxazin-3(4H)-one (5.04 g, 88.1%).
$^1$H-NMR (DMSO-$d_6$/400 MHz) δ (ppm): 1.76 (3H, d, J = 6.5 Hz), 4.70 (2H, s), 5.68 (1H, q, J = 6.5 Hz), 7.07 (1H, d, J = 8.5 Hz), 7.54 (1H, d, J = 2.2 Hz), 7.69 (1H, dd, J = 2.2, 8.5 Hz), 10.89 (1H, brs).

[Reference Example 6]

4-(5-Chlorothiophene-2-yl)-4-hydroxypiperidine

Step 1:

2-Bromo-5-chlorothiophene (12.0 g, 60.8 mmol) and magnesium granule (1.48 g, 60.9 mmol) were suspended in anhydrous THF (30 mL), and the mixture was stirred at room temperature followed by being stirred under ice-cooling. To the reaction solution was dropped 1-ethoxycarbonyl-4-piperidone (6.95 g, 40.6 mmol), and anhydrous THF (20 mL) was added thereto, then the mixture was further stirred. To the reaction solution was added saturated aqueous ammonium chloride (186 mL), and the mixture was filtrated through Celite. The filtrate was concentrated, and water was added thereto, then the mixture was extracted with ethyl acetate. The organic layer was washed with brine, dried over anhydrous magnesium sulfate, and concentrated in vacuo. To the resulting residue was added ethyl acetate, and the precipitated crystals were filtered. The resulting crystals were washed with ethyl acetate to give 4-(5-chlorothiophene-2-yl)-1-ethoxycarbonyl-4-hydroxypiperidine (6.54 g, 37.1%) as light yellow crystals.

Step 2:

4-(5-Chlorothiophene-2-yl)-1-ethoxycarbonyl-4-hydroxypiperidine (3.0 g, 10.4 mmol) prepared in the step 1 and potassium hydroxide (2.4 g, 42.8 mmol) were dissolved in isopropyl alcohol (25 mL), and the mixture was stirred overnight under heating to reflux. The reaction solution was concentrated in vacuo, and water was added to the resulting residue, then the mixture was extracted with ethyl acetate. The organic layer was washed with brine, dried over anhydrous magnesium sulfate, and concentrated in vacuo. To the resulting residue was added ethyl acetate, and the precipitated crystals were filtered. The resulting crystals were washed with ethyl acetate to give 4-(5-chlorothiophene-2-yl)-4-hydroxypiperidine (1.50 g, 66.6%) as light yellow crystals.

[Reference Example 7]

4-Hydroxy-4-(5-methylthiophene-2-yl)piperidine

Step 1:

2-Bromo-5-methylthiophenee (4.5 g, 25.4 mmol) and magnesium granule (617 mg, 2.53 mmol) were suspended in anhydrous THF (10 mL), and the mixture was stirred at room temperature, followed by being stirred under ice-cooling. To the reaction solution was dropped 1-ethoxycarbonyl-4-piperidone (3.5 mL, 22.9 mmol), and anhydrous THF (10 mL) was added thereto, then the mixture was further stirred. To the reaction solution was added water slowly, and the mixture was filtrated through Celite. The filtrate was concentrated, and water was added thereto, then the mixture was extracted with ethyl acetate. The organic layer was washed with brine, dried over anhydrous magnesium sulfate, and concentrated in vacuo. To the resulting residue was added water and methanol, and the precipitated substance was filtered to give 1-ethoxycarbonyl-4-hydroxy-4-(5-methylthiophene-2-yl)piperidine (4.49 g, 65.6%) as a brown oil.

Step 2:

1-Ethoxycarbonyl-4-hydroxy-4-(5-methylthiophene-2-yl)piperidine (4.91 g, 18.2 mmol) prepared in the step 1 and potassium hydroxide (4.10 g, 73.1 mmol) were dissolved in isopropyl alcohol (60 mL), and the mixture was stirred overnight under heating to reflux. The reaction solution was concentrated in vacuo, and to the resulting residue was added water, and the mixture was extracted with ethyl acetate. The organic layer was washed with brine, dried over anhydrous magnesium sulfate, and concentrated in vacuo to give 4-hydroxy-4-(5-methylthiophene-2-yl)piperidine (3.96 g, 100%) as a brown oil.
$^1$H-NMR (DMSO-$d_6$/400 MHz) δ (ppm) : 1. 18 (3H, t, J = 7.1 Hz), 1.71-1.80 (4H, m), 2.38 (3H, d, J = 1.2 Hz), 3.07-3.25 (2H, m), 3.79 (2H, d), 4.03 (2H, brq, J = 12.7 Hz), 5.42 (1H, s), 6.59-6.62 (1H, m), 6.72 (1H, d, J = 3.4 Hz).

[Reference Example 8]

4-Hydroxy-4-(3-methylthiophene-2-yl)piperidine

Step 1:

In accordance with the similar manner as mentioned in the step 1 of Reference Example 7, 1-ethoxycarbonyl-4-hydroxy-4-(3-methylthiophene-2-yl)piperidine (2.69 g, 39.3%) was obtained as light yellow crystals from 2-bromo-3-methylthiophenee (4.5 g, 25.4 mmol) and 1-ethoxycarbonyl-4-piperidone (3.5 mL, 22.9 mmol).
$^1$H-NMR (DMSO-$d_6$/400 MHz) δ (ppm): 1.18 (3H, t, J = 7.0 Hz), 1.65-1.90 (4H, m), 2.67 (3H, s), 3.00-3.25 (2H, m), 3.75-3.95 (2H, m), 4.04 (2H, q, J = 9.1 Hz), 5.51 (1H, s), 6.79 (1H, d, J = 5.1 Hz), 7.18 (1H, d, J = 5.1 Hz).

Step 2:

1-Ethoxycarbonyl-4-hydroxy-4-(3-methylthiophene-2-yl)piperidine (2.69 g, 9.98 mmol) prepared in the step 1 and potassium hydroxide (2.24 g, 39.9 mmol) were dissolved in isopropyl alcohol (30 mL), and the mixture was stirred for 21 hours under reflux. The reaction solution was concentrated in vacuo, and saturated aqueous sodium hydrogen carbonate was added thereto, then the mixture was extracted with ethyl acetate. The organic layer was washed with brine, dried over anhydrous magnesium sulfate, and concentrated in vacuo to give 4-hydroxy-4-(3-methylthiophene-2-yl)piperidine (1.59 g, 80.7%) as light yellow crystals.
$^1$H-NMR (DMSO-$d_6$/400 MHz) δ (ppm): 1.64-1.73 (2H, m), 1.84 (2H, dt, J = 4.6, 12.5 Hz), 2.29 (3H, s), 2.67-2.76 (2H, m), 2.82-2.93 (2H, m), 5.14 (1H, brs), 6.77 (1H, d, J = 5.1 Hz), 7.13 (1H, d, J = 5.1 Hz).

[Reference Example 9]

6-(2-Chloroethyl)-3,4-dihydroquinolin-2(1H)-one

6-(Chloroacetyl)-3,4-dihydroquinolin-2(1H)-one (6.0 g, 26.8 mmol) prepared by the similar manner as mentioned in JP-T-6-504293 or JP-A-2-72173 was suspended in trifluoroacetic acid (50 mL), and triethylsilane (17.4 mL, 0.107 mmol) was added thereto under ice-cooling, then the mixture was stirred overnight at room temperature. The reaction solution was concentrated in vacuo, and saturated aqueous sodium hydrogen carbonate was added to the resulting residue, then the mixture was extracted with ethyl acetate. The organic layer was washed with brine, dried over anhydrous magnesium sulfate, and concentrated in vacuo. To the resulting residue was added ethyl acetate, and the precipitated crystals were filtered. The resulting crystals were washed with ethyl acetate to give 6-(2-chloroethyl)-3,4-dihydroquinolin-2(1H)-one (4.98 g, 88.8%) as yellow crystals.
$^1$H-NMR ($CD_3OD$/400 MHz) δ (ppm): 2.60-2.68 (2H, m), 2.92-3.05 (4H, m), 3.69 (2H, t; J = 7.2 Hz), 6.70 (1H, d, J = 8.3 Hz), 6.99-7.07 (2H, m), 7.97 (1H, brs).

[Reference Example 10]

4-(Thiophene-2-yl)-1,2,5,6-tetrahydropyridine

4-Hydroxy-4-(thiophene-2-yl)piperidine (3.0 mg, 16.4 mmol) prepared by the similar manner as mentioned in JP-A-48-56687 was suspended in methanol (2 mL), and a methanolic hydrochloric acid (30 mL) was added thereto, then the mixture was stirred at 50 to 60°C for 3 hours. The reaction solution was concentrated in vacuo, and saturated aqueous sodium hydrogen carbonate was added to the resulting residue, then the mixture was extracted with ethyl acetate. The organic layer was washed with brine, dried over anhydrous magnesium sulfate, and concentrated in vacuo to give 4-(thiophene-2-yl)-1,2,5,6-tetrahydropyridine as a brown oil (in which 4-methoxy-4-(thiophene-2-yl)piperidine was contained as a by-product). Said product was used for the next reaction without further purification.

[Reference Example 11]

cis-3,4-Isopropylidenedioxy-4-(thiophene-2-yl)-piperidine

Step 1:

1-Ethoxycarbonyl-4-hydroxy-4-(thiophene-2-yl)piperidine (7.0 mg, 27.4 mmol) prepared by the similar manner as mentioned in JP-A-48-56687 was suspended in a methanolic hydrochloric acid, and the mixture was stirred at 60°C

for 4.5 hours. To the reaction solution was added saturated aqueous sodium hydrogen carbonate slowly under ice-cooling to make basic condition, and the mixture was extracted with ethyl acetate. The organic layer was washed with brine, dried over anhydrous magnesium sulfate, and concentrated in vacuo to give 1-ethoxycarbonyl-4-(thiophene-2-yl)-1,2,5,6-tetrahydro-pyridine (6.49 g, 99.5%) as a pink oil.

Step 2:

1-Ethoxycarbonyl-4-(thiophene-2-yl)-1,2,5,6-tetrahydropyridine (2.0 g, 8.43 mmol) prepared in the step 1 and N-methylmorpholine (1.97 g, 16.8 mmol) were suspended in acetone (40 mL), and 1% aqueous osmium tetraoxide (1 mL) was added thereto under ice-cooling, then the mixture was stirred overnight at room temperature. The reaction solution was concentrated in vacuo, and aqueous sodium thiosulfate was added to the resulting residue, then the mixture was extacted with ethyl acetate. The organic layer was washed with brine, dried over anhydrous magnesium sulfate, and concentrated in vacuo. To the resulting residue was added ethyl acetate, and the precipitated crystals were filtered. The resulting crystals were washed with ethyl acetate to give 1-ethoxycarbonyl-cis-3,4-dihydroxy-4-(thiophene-2-yl) piperidine (1.41 g, 61.6%).
$^1$H-NMR (DMSO-$d_6$/500 MHz) δ (ppm): 1.19 (3H, t, J = 7.1 Hz), 1.74-1.87 (2H, m), 2.83-3.15 (2H, m), 3.56-3.64 (1H, m), 3.70-3.94 (2H, m), 4.04 (2H, q, J = 7.1 Hz), 4.97 (1H, d, J = 6.5 Hz), 5.26 (1H, s), 6.95 (1H, dd, J = 3.5, 5.0 Hz), 6.99 (1H, dd, J = 1.2, 3.5. Hz), 7.33 (1H, dd, J = 1.2, 5.0 Hz).

Step 3:

1-Ethoxycarbonyl-cis-3,4-dihydroxy-4-(thiophene-2-yl)piperidine (1.1 g, 4.05 mmol) prepared in the step 2 was suspended in 2,2-dimethoxypropane, and p-toluenesulfonic acid monohydrate (250 mg, 1.45 mmol) was added thereto at room temperature, then the mixture was stirred overnight. To the reaction solution was added saturated aqueous sodium hydrogen carbonate, and the mixture was extracted with ethyl acetate. The organic layer was washed with saturated aqueous sodium hydrogen carbonate and brine, dried over anhydrous magnesium sulfate, and concentrated in vacuo. The resulting residue was purified by silica gel column chromatography (eluant: a mixture of hexane/ethyl acetate = 3/1) to give 1-ethoxycarbonyl-cis-3,4-isopropylidenedioxy-4-(thiophene-2-yl)piperidine (1.0 g, 79.3%) as a colorless oil.
$^1$H-NMR (DMSO-$d_6$/400 MHz) δ (ppm): 1.27 (3H, t, J = 7.1 Hz), 1.33 (3H, s), 1.47 (3H, d, J = 0.5 Hz), 2.02-2.08 (2H, m), 3.30-3.46 (2H, m), 3.57-3.69 (1H, m), 4.09-4.19 (1H, m), 4.22 (1H, dd, J = 2.7, 14.9 Hz), 4.44-4.56 (1H, m), 4.80 (1H, s), 6.99 (1H, dd, J = 3.5, 5.1 Hz), 7.05 (1H, brd, J = 3.5 Hz), 7.32 (1H, dd, J = 1.2, 5.1 Hz).

Step 4:

1-Ethoxycarbonyl-cis-3,4-isopropylidenedioxy-4-(thiophene-2-yl)piperidine (1.0 g, 3.21 mmol) prepared in the step 3 was suspended in isopropyl alcohol (20 mL), and potassium hydroxide (920 mg, 16.4 mmol) was added thereto, then the mixture was stirred overnight at 90˚C. The reaction solution was concentrated in vacuo, and water was added to the resulting residue, then the mixture was extracted with ethyl acetate. The organic layer was washed with brine, dried over anhydrous magnesium sulfate, and concentrated in vacuo. The resulting residue was purified by silica gel column chromatography (eluant: a mixture of chloroform/methanol = 20/1) to give cis-3,4-isopropylidenedioxy-4-(thiophene-2-yl)piperidine (598 mg, 77.8%) as a colorless oil.
$^1$H-NMR ($CDCl_3$/500 MHz) δ (ppm): 1.48 (3H, d, J = 0.4 Hz), 1.59 (3H, d, J = 0.4 Hz), 2.03 (1H, ddd, J = 4.8, 12.9, 13.9 Hz), 2.25 (1H, dt, J = 2.8, 13.9 Hz), 2.73 (1H, ddd, J = 2.8, 12.9, 13.9 Hz), 2.97 (1H, dd, J = 2.5, 15.1 Hz), 3.09-3.16 (1H, m), 3.41 (1H, dt, J = 1.6, 15.1 Hz), 4.03-4.07 (1H, m), 6.98 (1H, dd, J = 3.6, 5.1 Hz), 7.08 (1H, dd, J = 1.1, 3.6 Hz), 7.27 (1H, dd, J = 1.1, 5.1 Hz).

[Reference Example 12]

4-(Thiophene-2-yl)piperidine

4-Hydroxy-4-(thiophene-2-yl)piperidine (500 mg, 2.73 mmol) prepared by the similar manner as mentioned in JP-A-48-56687 was suspended in trifluoroacetic acid (10 mL), and triethylsilane (1.75 mL, 11.0 mmol) was added thereto under ice-cooling, then the mixture was stirred overnight at room temperature. The reaction solution was concentrated in vacuo, and saturated aqueous sodium hydrogen carbonate was added to the resulting residue, then the mixture was exracted with ethyl acetate. The organic layer was washed with brine, dried over anhydrous sodium sulfate, and concentrated in vacuo. The resulting residue was purified by silica gel column chromatography (Fuji Silicia Kagaku NH; eluant: a mixture of hexane/ethyl acetate = 3/1 to a mixture of chloroform/methanol = 30/1) to give 4-(thiophene-2-yl)piperidine (60.3 mg, 13.2%).

[Reference Example 13]

trans-3-Hydroxy-4-(thiophene-2-yl)piperidine

Step 1:

1-Ethoxycarbonyl-4-(thiophene-2-yl)-1,2,5,6-tetrahydropyridine (2.34 g, 9.86 mmol) prepared in the step 1 of Reference Example 11 was suspended in anhydrous THF (80 mL) under an argon atmosphere, and the mixture was ice-cooled. To the resulting suspension was added a 1 mol/L solution of borane tetrahydrofuran complex in THF (20 mL), and the mixture was stirred overnight at room temperature. To the reaction solution were added methanol (140 mL), 3 mol/L aqueous sodium hydroxide (3.6 mL) and 30% aqueous hydrogen peroxide (1.6 mL) successively under ice-cooling, and the mixture was stirred at 65°C for 2.5 hours. The reaction solution was concentrated in vacuo, and water was added to the resulting residue, then the mixture was exracted with ethyl acetate. The organic layer was washed with brine, dried over anhydrous magnesium sulfate, and concentrated in vacuo. The resulting residue was purified by silica gel column chromatography (eluant: a mixture of hexane/ethyl acetate = 3/1) to give 1-ethoxycarbonyl-trans-3-hydroxy-4-(thiophene-2-yl)piperidine(1.50g, 59.6%) as a colorless oil.
$^1$H-NMR ($CD_3OD$/400 MHz) δ (ppm): 1.26 (3H, t, J = 7.1 Hz), 1.62-1.76 (1H, m), 1.95-2.04 (1H, m), 2.61-2.76 (1H, m), 2.82-2.95 (2H, m), 3.43-3.53 (1H, m), 4.08-4.18 (1H, m), 4.13 (2H, q, J = 7.1 Hz), 4.22-4.32 (1H, m), 6.90-6.96 (2H, m), 7.22 (1H, dd, J = 1.6, 4.5 Hz).

Step 2:

1-Ethoxycarbonyl-trans-3-hydroxy-4-(thiophene-2-yl)piperidine (1.50 g, 5.87 mmol) prepared in the step 1 was suspended in isopropyl alcohol (30 mL), and potassium hydroxide (1.65 g, 29.4 mmol) was added thereto at room temperature, then the mixture was stirred overnight at 95°C. The reaction solution was concentrated in vacuo, and water was added to the resulting residue, then the mixture was exracted with ethyl acetate. The organic layer was washed with brine, dried over anhydrous magnesium sulfate, and concentrated in vacuo to give trans-3-hydroxy-4-(thiophene-2-yl)piperidine (0.70 g, 65.1%) as a colorless oil.
$^1$H-NMR ($CD_3OD$/400 MHz) δ (ppm): 1.75-1.80 (1H, m), 1.95-2.04 (1H, m), 2.44 (1H, dd, J = 10.1, 12.2 Hz), 2.60 (1H, ddd, J = 2.9, 12.5, 12.5 Hz), 2.76-2.86 (1H, m), 2.97-3.05 (1H, m), 3.14-3.22 (1H, m), 3.53 (1H, ddd, J = 4.6, 10.0, 10.1 Hz), 6.89-6.98 (2H, m), 7.20 (1H, dd, J = 1.3, 4.7 Hz).

Industrial Applicability

In accordance with the present invention, there are provided, for example, a piperidine derivative or a pharmaceutically acceptable salt thereof having an antagonistic activity at an NR2B/NMDA receptor, etc.; and an agent for the treatment of a disease in which an NR2B subunit of an NMDA receptor is participated (for example, a disease due to degeneration and injury of nerve cells such as epilepsy, stroke, anxiety, cerebral ischemia, muscle spasm, Alzheimer's disease, dementia, Huntington's disease, Parkinson's disease, cerebral apoplexy, cerebral infarction and the like, and pain such as chronic pain, neurogenic pain, headache, migraine and the like) comprising the piperidine derivative or a pharmaceutically acceptable salt thereof as an active ingredient.

## Claims

**1.** A piperidine derivative represented by the formula (I)

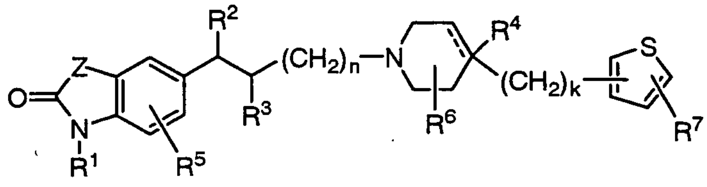

(I)

{wherein,

-C(=O)-Z- represents -C(=O)-$CH_2$-, -C(=O)-C($CH_3$)$_2$-, -C(=O)-NH-, -C(=O)-O-, -C(=O)-S-, -C(=O)-$CH_2CH_2$-, -C(=O)-CH=CH-, -C(=O)-$CH_2$O-, -C(=O)-$CH_2$S-, -C(=O)-$CH_2CH_2CH_2$-, or -C(=O)-N$R^8$$CH_2$- (wherein, $R^8$ represents a hydrogen atom, or substituted or unsubstituted lower alkyl);
$R^1$ represents a hydrogen atom, substituted or unsubstituted lower alkyl, substituted or unsubstituted lower alkenyl, or substituted or unsubstituted lower alkynyl;
$R^2$ represents a hydrogen atom or hydroxy;
$R^3$ represents a hydrogen atom, substituted or unsubstituted lower alkyl, substituted or unsubstituted lower alkenyl, or substituted or unsubstituted lower alkynyl;
$R^5$ represents a hydrogen atom, hydroxy, substituted or unsubstituted lower alkyl, substituted or unsubstituted lower alkenyl, substituted or unsubstituted lower alkynyl, or substituted or unsubstituted lower alkoxy;
$R^6$ represents a hydrogen atom, hydroxy, substituted or unsubstituted lower alkyl, substituted or unsubstituted lower alkenyl, or substituted or unsubstituted lower alkynyl;
$R^7$ represents a hydrogen atom, halogen, substituted or unsubstituted lower alkyl, substituted or unsubstituted lower alkenyl, substituted or unsubstituted lower alkynyl, substituted or unsubstituted lower alkoxy, or -($CH_2$)$_m$Y [wherein, m represents an integer of 1 to 4; Y represents - N$R^9R^{10}$ (wherein, $R^9$ and $R^{10}$ each represents independently a hydrogen atom, substituted or unsubstituted lower alkyl, or substituted or unsubstituted aralkyl, or $R^9$ and $R^{10}$ form a heterocyclic group together with adjacent nitrogen atom), or substituted or unsubstituted heterocyclic alkyl];
n and k each represents independently an integer of 0 to 2; and
$\underline{----}$ represents a single bond, or a double bond together with $R^4$, and when $\underline{----}$ is a single bond, $R^4$ represents a hydrogen atom, hydroxy, substituted or unsubstituted lower alkoxy, or halogen}, or a pharmaceutically acceptable salt thereof.

**2.** A piperidine derivative represented by the formula (IA)

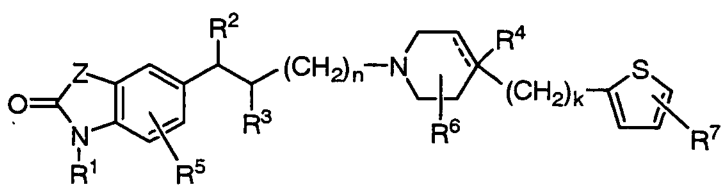

( IA )

(wherein, $\underline{----}$, -C(=O)-Z-, $R^1$, $R^2$, $R^3$, $R^4$, $R^5$, $R^6$, $R^7$, n and k have the same meaning as defined above, respectively), or a pharmaceutically acceptable salt thereof.

**3.** The piperidine derivative or a pharmaceutically acceptable salt thereof according to claim 1 or 2, wherein - C(=O)-Z- is -C(=O)-S-.

**4.** The piperidine derivative or a pharmaceutically acceptable salt thereof according to claim 1 or 2, wherein - C(=O)-Z- is -C(=O)-$CH_2$-.

**5.** The piperidine derivative or a pharmaceutically acceptable salt thereof according to claim 1 or 2, wherein - C(=O)-Z- is -C(=O)-$CH_2CH_2$-.

**6.** The piperidine derivative or a pharmaceutically acceptable salt thereof according to claim 1 or 2, wherein - C(=O)-Z- is -C(=O)-$CH_2CH_2CH_2$-.

**7.** The piperidine derivative or a pharmaceutically acceptable salt thereof according to any one of claims 1 to 6, wherein $\underline{----}$ is a single bond.

**8.** The piperidine derivative or a pharmaceutically acceptable salt thereof according to any one of claims 1 to 6, wherein $R^4$ is hydroxy.

**9.** The piperidine derivative or a pharmaceutically acceptable salt thereof according to any one of claims 1 to 6, wherein $R^4$ is a hydrogen atom or hydroxy.

**10.** The piperidine derivative or a pharmaceutically acceptable salt thereof according to any one of claims 1 to 9, wherein $R^6$ is a hydrogen atom.

**11.** The piperidine derivative or a pharmaceutically acceptable salt thereof according to any one of claims 1 to 9, wherein a binding position of $R^6$ is 3-position on the morpholine ring, and $R^6$ is hydroxy.

**12.** The piperidine derivative or a pharmaceutically acceptable salt thereof according to any one of claims 1 to 11, wherein $R^2$ is a hydrogen atom or hydroxy.

**13.** The piperidine derivative or a pharmaceutically acceptable salt thereof according to any one of claims 1 to 11, wherein $R^2$ is hydroxy.

**14.** The piperidine derivative or a pharmaceutically acceptable salt thereof according to any one of claims 1 to 13, wherein $R^3$ is a hydrogen atom or lower alkyl.

**15.** The piperidine derivative or a pharmaceutically acceptable salt thereof according to any one of claims 1 to 13, wherein $R^3$ is a hydrogen atom.

**16.** The piperidine derivative or a pharmaceutically acceptable salt thereof according to any one of claims 1 to 15, wherein n is 0.

**17.** The piperidine derivative or a pharmaceutically acceptable salt thereof according to any one of claims 1 to 16, wherein k is 0.

**18.** The piperidine derivative according or a pharmaceutically acceptable salt thereof according to any one of claims 1 to 17, wherein $R^5$ is a hydrogen atom.

**19.** The piperidine derivative or a pharmaceutically acceptable salt thereof according to any one of claims 1 to 18, wherein $R^7$ is a hydrogen atom.

**20.** The piperidine derivative or a pharmaceutically acceptable salt thereof according to any one of claims 1 to 19, wherein the piperidine derivative is a racemate.

**21.** The piperidine derivative or a pharmaceutically acceptable salt thereof according to any one of claims 1 to 19, wherein the piperidine derivative is an optically active compound.

**22.** A pharmaceutical composition comprising the piperidine derivative or a pharmaceutically acceptable salt thereof according to any one of claims 1 to 21 as an active ingredient.

**23.** An N-methyl-D-aspartic acid (NMDA) type glutamic acid receptor containing NR2B subunit (NR2B/NMDA receptor) antagonist, which comprises the piperidine derivative or a pharmaceutically acceptable salt thereof according to any one of claims 1 to 21 as an active ingredient.

**24.** A selective antagonist of NR2B/NMDA receptor (a selective NR2B/NMDA receptor antagonist) which comprises the piperidine derivative or a pharmaceutically acceptable salt thereof according to any one of claims 1 to 21 as an active ingredient.

**25.** A therapeutic agent for a disease in which an NR2B subunit of an NMDA receptor is participated, which comprises the piperidine derivative or a pharmaceutically acceptable salt thereof according to any one of claims 1 to 21 as an active ingredient.

**26.** A therapeutic agent for pain which comprises the piperidine derivative or a pharmaceutically acceptable salt thereof according to any one of claims 1 to 21 as an active ingredient.

**27.** A therapeutic agent for neurogenic pain which comprises the piperidine derivative or a pharmaceutically acceptable

salt thereof according to any one of claims 1 to 21 as an active ingredient.

**28.** A method for antagonizing at an NR2B/NMDA receptor, which comprises administering an effective amount of the piperidine derivative or a pharmaceutically acceptable salt thereof according to any one of claims 1 to 21.

**29.** A method for selective antagonizing at an NR2B/NMDA receptor, which comprises administering an effective amount of the piperidine derivative or a pharmaceutically acceptable salt thereof according to any one of claims 1 to 21.

**30.** A method for treating a disease in which an NR2B subunit of an NMDA receptor is participated, which comprises administering an effective amount of the piperidine derivative or a pharmaceutically acceptable salt thereof according to any one of claims 1 to 21.

**31.** A method for treating pain, which comprises administering an effective amount of the piperidine derivative or a pharmaceutically acceptable salt thereof according to any one of claims 1 to 21.

**32.** A method for treating neurogenic pain, which comprises administering an effective amount of the piperidine derivative or a pharmaceutically acceptable salt thereof according to any one of claims 1 to 21.

**33.** Use of the piperidine derivative or a pharmaceutically acceptable salt thereof according to any one of claims 1 to 21 for the manufacture of an NR2B/NMDA receptor antagonist.

**34.** Use of the piperidine derivative or a pharmaceutically acceptable salt thereof according to any one of claims 1 to 21 for the manufacture of a selective NR2B/NMDA receptor antagonist.

**35.** Use of the piperidine derivative or a pharmaceutically acceptable salt thereof according to any one of claims 1 to 21 for the manufacture of a therapeutic agent for a diseases in which an NR2B subunit of an NMDA receptor is participated.

**36.** Use of the piperidine derivative or a pharmaceutically acceptable salt thereof according to any one of the claims 1 to 21 for the manufacture of a therapeutic agent for pain.

**37.** Use of the piperidine derivative or a pharmaceutically acceptable salt thereof according to any one of claims 1 to 21 for the manufacture of a therapeutic agent for neurogenic pain.

[Fig. 1]

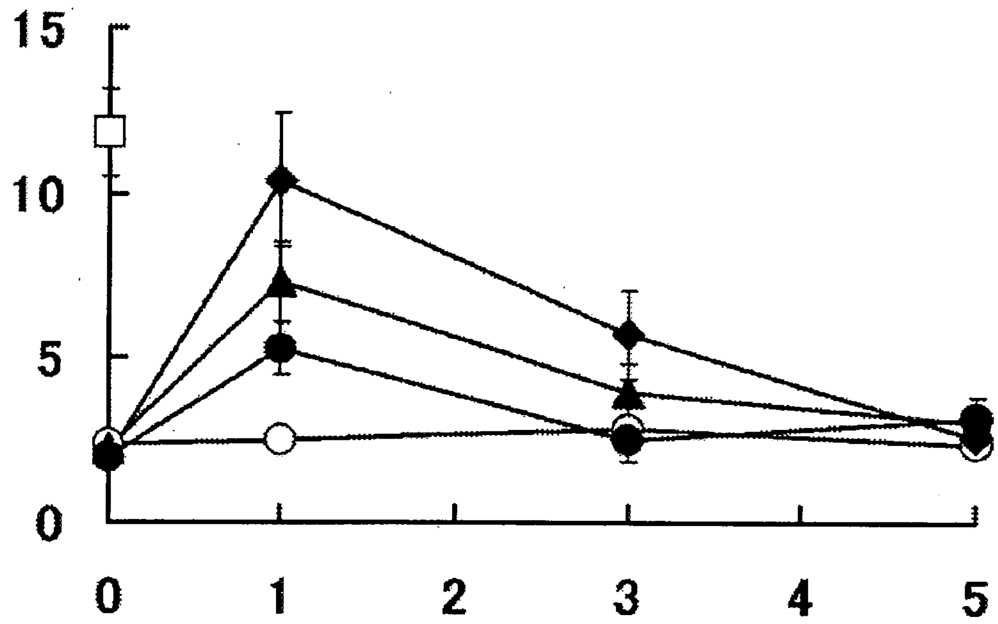


[Fig. 2]

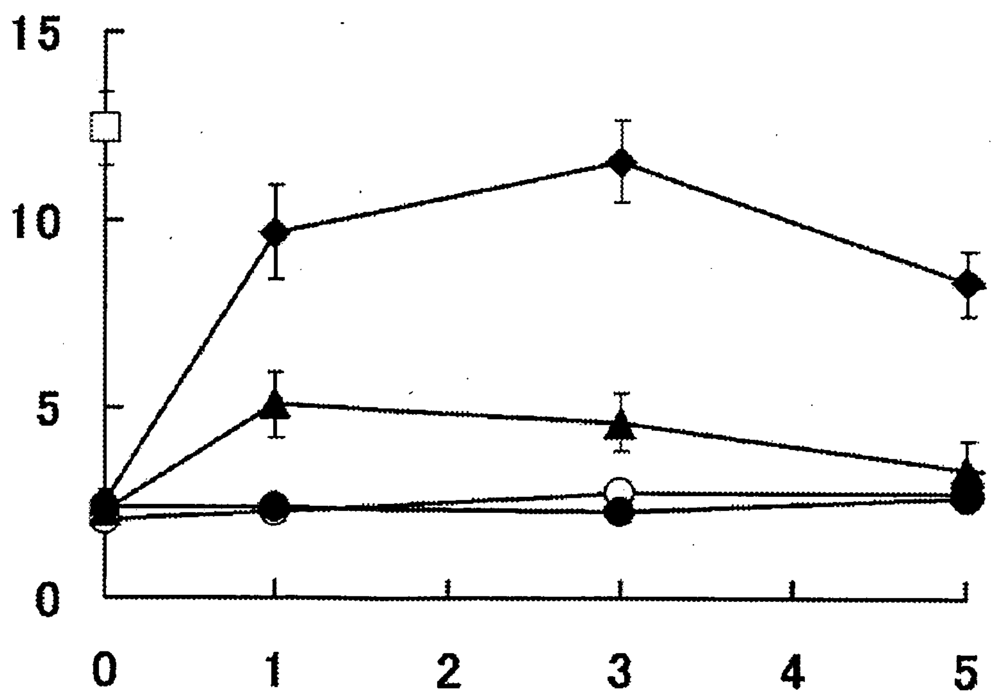

## INTERNATIONAL SEARCH REPORT

International application No.
PCT/JP2005/006859

A. CLASSIFICATION OF SUBJECT MATTER
Int.Cl[7] C07D409/14, A61K31/454, 31/4709, 31/538, 31/5415, A61P9/10, 21/02, 25/04, 25/06, 25/08, 25/14, 25/16, 25/22, 25/28, 43/00, C07D413/14, 417/14

According to International Patent Classification (IPC) or to both national classification and IPC

B. FIELDS SEARCHED

Minimum documentation searched (classification system followed by classification symbols)
Int.Cl[7] C07D409/14, A61K31/454, 31/4709, 31/538, 31/5415, A61P9/10, 21/02, 25/04, 25/06, 25/08, 25/14, 25/16, 25/22, 25/28, 43/00, C07D413/14, 417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| E,X | WO 2005/35523 A1 (PFIZER JAPAN INC.), 21 April, 2005 (21.04.05), Claims 1 to 16; page 9, lines 13 to 20 (Family: none) | 1-2,4-10, 12-27,33-37 |
| A | WO 03/91241 A1 (PFIZER PHARMACEUTICALS INC.), 06 November, 2003 (06.11.03), Full text & US 2003/216430 A1 | 1-27,33-37 |
| A | JP 5-186460 A (SYNTHELABO), 27 July, 1993 (27.07.93), Full text & EP 524846 A1 & FR 2678265 A1 | 1-27,33-37 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May, 2005 (17.05.05) | 31 May, 2005 (31.05.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/006859

C (Continuation). DOCUMENTS CONSIDERED TO BE RELEVANT

| Category* | Citation of document, with indication, where appropriate, of the relevant passages | Relevant to claim No. |
|---|---|---|
| A | JP 5-507281 A (Pfizer Inc.),<br>21 October, 1993 (21.10.93),<br>Full text<br>& EP 557289 A1 & WO 92/8718 A1 | 1-27,33-37 |

Form PCT/ISA/210 (continuation of second sheet) (January 2004)

INTERNATIONAL SEARCH REPORT

International application No.
PCT/JP2005/006859

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 28-32
because they relate to subject matter not required to be searched by this Authority, namely:
Claims 28 to 32 are relevant to methods for treatment of the human body by surgery or therapy and diagnostic methods.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 308135 A
- WO 9117156 A
- WO 9410166 A
- WO 0391241 A
- US 5710168 A
- JP 2002322092 A
- WO 9707098 A
- WO 9014087 A
- WO 9014088 A
- JP 52118465 A
- JP 6504293 T
- JP 51118770 A
- JP 56156263 A
- JP 2072173 A
- JP 48056687 A
- JP 51115480 A
- WO 9720789 A
- JP 11335385 A


### Non-patent literature cited in the description


- *Drug Dev. Res.,* 1989, vol. 17, 299
- *Clinical Sci.,* 1985, vol. 68, 113
- *Pain,* 1992, vol. 51, 249-253
- *Pain,* 1995, vol. 61, 221-228
- *Intensive Care,* 1995, vol. 23, 620-622
- *Clinical Neuropharmacology,* 1995, vol. 18, 360-368
- *Journal of Pharmaceutical and Experimental Therapeutics,* 1988, vol. 247, 1211-1221
- *Molecular Pharmacology,* 1993, vol. 44, 851-859
- *British Journal of Pharmacology,* 1997, vol. 122, 809-812
- *European Journal of Pharmacology,* 1996, vol. 298, 51-55
- **T. W. GREENE.** Protective Groups in Organic Synthesis. John Wiley & Sons Inc, 1999
- Separation of Optical Isomer. Kikan Kagaku Sosetsu 6. Chemical Society of Japan, 1989
- **G. M. PITCHER et al.** *Pain,* 1999, vol. 83, 37-46
- **T. MOSCONI ; K. KRUGER.** *Pain,* 1996, vol. 64, 37-57
- **W. J. DIXON.** *Annual Review of Pharmacology and Toxicology,* 1980, vol. 20, 441-462